(19) Europäisches Patentamt
European Patent Office
Office européen des brevets

(11) EP 3 438 266 A1

(12) EUROPEAN PATENT APPLICATION
published in accordance with Art. 153(4) EPC

(43) Date of publication:
**06.02.2019 Bulletin 2019/06**

(21) Application number: **17775570.9**

(22) Date of filing: **31.03.2017**

(51) Int Cl.:
***C12N 15/113*** *(2010.01)* ***C12M 1/00*** *(2006.01)*
***C12N 15/09*** *(2006.01)* ***C12Q 1/68*** *(2018.01)*
***G01N 33/50*** *(2006.01)*

(86) International application number:
**PCT/JP2017/013708**

(87) International publication number:
**WO 2017/171039 (05.10.2017 Gazette 2017/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **01.04.2016 JP 2016074717**

(71) Applicants:
- **Toray Industries, Inc.**
  **Tokyo 103-8666 (JP)**
- **National Cancer Center**
  **Tokyo 104-0045 (JP)**

(72) Inventors:
- **YOSHIMOTO Makiko**
  **Kamakura-shi**
  **Kanagawa 248-8555 (JP)**
- **KOZONO Satoko**
  **Kamakura-shi**
  **Kanagawa 248-8555 (JP)**
- **KAWAUCHI Junpei**
  **Kamakura-shi**
  **Kanagawa 248-8555 (JP)**
- **KONDOU Satoshi**
  **Kamakura-shi**
  **Kanagawa 248-8555 (JP)**
- **NOBUMASA Hitoshi**
  **Kamakura-shi**
  **Kanagawa 248-8555 (JP)**
- **OCHIYA Takahiro**
  **Tokyo 104-0045 (JP)**
- **NARITA Yoshitaka**
  **Tokyo 104-0045 (JP)**
- **OHNO Makoto**
  **Tokyo 104-0045 (JP)**

(74) Representative: **Mewburn Ellis LLP**
**City Tower**
**40 Basinghall Street**
**London EC2V 5DE (GB)**

# (54) KIT OR DEVICE FOR DETECTING MALIGNANT BRAIN TUMOR AND METHOD FOR DETECTING SAME

(57) The present invention provides a kit or device for the detection of malignant brain tumor, and a method for detecting malignant brain tumor. The present invention relates to a kit or device for the detection of malignant brain tumor, comprising nucleic acid(s) capable of specifically binding to predetermined miRNA(s) in a sample of a subject, and a method for detecting malignant brain tumor, comprising measuring the miRNA(s) *in vitro.*

EP 3 438 266 A1

## Description

### Technical Field

The present invention relates to a kit or a device for the detection of malignant brain tumor, comprising a nucleic acid capable of specifically binding to a particular miRNA, which is used for examining the presence or absence of malignant brain tumor in a subject, and a method for detecting malignant brain tumor, comprising measuring an expression level of the miRNA using the nucleic acid.

### Background Art

Brain tumors are divided into primary brain tumors that develop from brain tissues themselves and metastatic brain tumors that are caused by metastasis to the brain from a different organ. The primary brain tumors are divided into benign and malignant tumors and subdivided depending on cell types of origin.

The primary brain tumors are mainly classified into glioma (malignant), primary central nervous system lymphoma (malignant), meningioma (typically, benign), pituitary adenoma (benign), neurilemmoma (benign), congenital tumor, and the like. Glioma occupies 28% of the whole. Glioma is further classified into astrocytoma, oligodendroglioma, oligoastrocytoma, pilocytic astrocytoma, ependymoma, ganglioglioma, and the like depending on the forms of cells constituting tumors.

According to the 2014 statistics of cancer type-specific mortality in Japan reported by the Center for Cancer Control and Information Services, National Cancer Center (Japan), the number of brain and central nervous system cancer deaths was 2,302 people, and cancer type-specific mortality in 2013 was 2.0% for males and 1.5% for females. Thus, the incidence of brain tumors is as low as 5 or less people per 100 cancer patients, relative to overall cancer cases. However, in childhood cancer patients alone, brain tumors are the second most common cancers following leukemia, and young people are more often affected with one out of the five childhood cancer patients.

The stage classification (TNM) of brain tumors is not defined in UICC (Unio Internationalis Contra Cancrum) "TNM Classification of Malignant Tumours", the 6th edition. The degrees of malignancy of brain tumors are classified into grades I to IV according to the 2007 WHO classification.

There is no useful blood marker for brain tumors. Brain tumors are usually detected by, for example, imaging tests of patients who complain of subjective symptoms such as headache, vomiting, paralysis, aphasia or dysarthria, or disturbed consciousness, or minimal head trauma, or imaging tests in medical checkup such as brain checkup. CT, MRI, cerebral angiography, or the like is utilized in diagnostic imaging. When a brain tumor is detected by diagnostic imaging, the tumor is excised with a craniotomy procedure and pathological diagnosis is conducted using the excised tissues. At present, any tumor marker for detecting malignant brain tumor with a marker in blood is not commonly used in clinical settings.

Meanwhile, as mentioned below, there are reports, albeit at a research stage, on methods for treating or diagnosing brain tumors on the basis of the expression levels of miRNA in biological samples including blood, which have not yet put into practical use.

Patent Literature 1 describes a method for treating or diagnosing cancers including brain tumors on the basis of miRNA that differs in its expression level between human glioblastoma stem cells and healthy neural stem cells. However, Patent Literature 1 merely shows data on change in miRNA expression level in cells. Since obtainment of brain cells as a sample places a great physical burden on patients, an examination method using brain cells as a sample is not favorable. In addition, Patent Literature 1 does not specifically describe discriminant performance, such as accuracy, sensitivity, or specificity, and an approach for discriminating brain tumors, as to the diagnosis method of Patent Literature 1, and thus the diagnosis method has little industrial utility.

### Citation List

#### Patent Literature

Patent Literature 1: US Patent Application Publication No. US 2014/0088170

### Summary of Invention

#### Technical Problem

A problem underlying the present invention is to provide a novel marker for malignant brain tumor, and a method capable of effectively detecting malignant brain tumor.

Solution to Problem

As described above, there is no existing tumor marker for the detection of malignant brain tumor, and neither performance nor detection methods are specifically shown as to the markers at a research stage. Use of these markers might impose an implementation of needless extra examination due to the false detection of healthy subjects as being malignant brain tumor patients, or might waste therapeutic opportunity because of overlooking malignant brain tumor patients. Furthermore, the collection of brain tissues for measuring the tumor markers is not favorable because of its risks associated with craniotomy procedures and high invasiveness to patients. Hence, it is clinically important to develop convenient, highly accurate, and noninvasive diagnostic markers in blood, which can be collected in a less invasive manner. Particularly, early detection or early treatment of malignant brain tumor, which exhibits poor prognosis, is expected to achieve a breakthrough in improvement in treatment outcomes.

The present inventors have conducted diligent studies to solve the problem and consequently completed the present invention by finding a plurality of genes (miRNAs) usable as markers for the detection of malignant brain tumor from blood, which can be collected low invasively, and finding that malignant brain tumor can be significantly detected by using a nucleic acid capable of specifically binding to any of these markers.

Specifically, the present invention includes the followings:

(1) A kit for the detection of malignant brain tumor, comprising nucleic acid(s) capable of specifically binding to at least one polynucleotide selected from the group consisting of malignant brain tumor markers: miR-1909-3p, miR-6869-5p, miR-3178, miR-4787-5p, miR-6510-5p, miR-4695-5p, miR-4634, miR-4449, miR-3195, and miR-6836-3p.

(2) The kit according to (1), wherein miR-1909-3p is hsa-miR-1909-3p, miR-6869-5p is hsa-miR-6869-5p, miR-3178 is hsa-miR-3178, miR-4787-5p is hsa-miR-4787-5p, miR-6510-5p is hsa-miR-6510-5p, miR-4695-5p is hsa-miR-4695-5p, miR-4634 is hsa-miR-4634, miR-4449 is hsa-miR-4449, miR-3195 is hsa-miR-3195, and miR-6836-3p is hsa-miR-6836-3p.

(3) The kit according to (1) or (2), wherein the nucleic acid is a polynucleotide selected from the group consisting of the following polynucleotides (a) to (e):

(a) a polynucleotide consisting of a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,

(b) a polynucleotide comprising a nucleotide sequence represented by any of SEQ ID NOs: 1 to 10,

(c) a polynucleotide consisting of a nucleotide sequence complementary to a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 1 to 10 or a nucleotide sequence derived from the nucleotide sequence by the replacement of u with t, and

(e) a polynucleotide hybridizing under stringent conditions to any of the polynucleotides (a) to (d).

(4) The kit according to any one of (1) to (3), wherein the kit further comprises a nucleic acid capable of specifically binding to a polynucleotide of another malignant brain tumor marker miR-187-5p.

(5) The kit according to (4), wherein miR-187-5p is hsa-miR-187-5p.

(6) The kit according to (4) or (5), wherein the nucleic acid capable of specifically binding to the polynucleotide of miR-187-5p is a polynucleotide selected from the group consisting of the following polynucleotides (f) to (j):

(f) a polynucleotide consisting of a nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,

(g) a polynucleotide comprising a nucleotide sequence represented by SEQ ID NO: 11,

(h) a polynucleotide consisting of a nucleotide sequence complementary to a nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,

(i) a polynucleotide comprising a nucleotide sequence complementary to a nucleotide sequence represented by SEQ ID NO: 11 or a nucleotide sequence derived from the nucleotide sequence by the replacement of u with t, and

(j) a polynucleotide hybridizing under stringent conditions to any of the polynucleotides (f) to (i).

(7) A device for the detection of malignant brain tumor, comprising nucleic acid(s) capable of specifically binding to at least one polynucleotide selected from the group consisting of malignant brain tumor markers: miR-1909-3p, miR-6869-5p, miR-3178, miR-4787-5p, miR-6510-5p, miR-4695-5p, miR-4634, miR-4449, miR-3195, and miR-6836-3p.
(8) The device according to (7), wherein miR-1909-3p is hsa-miR-1909-3p, miR-6869-5p is hsa-miR-6869-5p, miR-3178 is hsa-miR-3178, miR-4787-5p is hsa-miR-4787-5p, miR-6510-5p is hsa-miR-6510-5p, miR-4695-5p is hsa-miR-4695-5p, miR-4634 is hsa-miR-4634, miR-4449 is hsa-miR-4449, miR-3195 is hsa-miR-3195, and miR-6836-3p is hsa-miR-6836-3p.
(9) The device according to (7) or (8), wherein the nucleic acid is a polynucleotide selected from the group consisting of the following polynucleotides (a) to (e):

(a) a polynucleotide consisting of a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,
(b) a polynucleotide comprising a nucleotide sequence represented by any of SEQ ID NOs: 1 to 10,
(c) a polynucleotide consisting of a nucleotide sequence complementary to a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,
(d) a polynucleotide comprising a nucleotide sequence complementary to a nucleotide sequence represented by any of SEQ ID NOs: 1 to 10 or a nucleotide sequence derived from the nucleotide sequence by the replacement of u with t, and
(e) a polynucleotide hybridizing under stringent conditions to any of the polynucleotides (a) to (d).

(10) The device according to any one of (7) to (9), wherein the device further comprises a nucleic acid capable of specifically binding to a polynucleotide of another malignant brain tumor marker miR-187-5p.
(11) The device according to (10), wherein miR-187-5p is hsa-miR-187-5p.
(12) The device according to (10) or (11), wherein the nucleic acid capable of specifically binding to the polynucleotide of miR-187-5p is a polynucleotide selected from the group consisting of the following polynucleotides (f) to (j):

(f) a polynucleotide consisting of a nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(g) a polynucleotide comprising a nucleotide sequence represented by SEQ ID NO: 11,
(h) a polynucleotide consisting of a nucleotide sequence complementary to a nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(i) a polynucleotide comprising a nucleotide sequence complementary to a nucleotide sequence represented by SEQ ID NO: 11 or a nucleotide sequence derived from the nucleotide sequence by the replacement of u with t, and
(j) a polynucleotide hybridizing under stringent conditions to any of the polynucleotides (f) to (i).

(13) The device according to any one of (7) to (12), wherein the device is a device for measurement by a hybridization technique.
(14) The device according to (13), wherein the hybridization technique is a nucleic acid array technique.
(15) A method for detecting malignant brain tumor, comprising measuring an expression level of a target nucleic acid in a sample of a subject using the kit according to any one of (1) to (6) or the device according to any one of (7) to (14); and evaluating whether or not the subject has malignant brain tumor using the measured expression level and a control expression level for a healthy subject or a benign brain tumor patient measured in the same way, to detect the presence or absence of malignant brain tumor in the subject.
(16) A method for detecting malignant brain tumor in a subject, comprising measuring an expression level of a target gene in a sample of the subject using the kit according to any one of (1) to (6) or the device according to any one of (7) to (14); and substituting the expression level of the target gene in the sample derived from the subject into a discriminant that is prepared with a gene expression level in a sample derived from a subject known to have malignant brain tumor and a gene expression level in a sample derived from a healthy subject or a benign brain tumor patient as supervising samples and is capable of differentially discriminating a malignant brain tumor patient from a healthy subject or a benign brain tumor patient, thereby evaluating the presence or absence of malignant brain tumor.
(17) The method according to (15) or (16), wherein the subject is a human.

(18) The method according to any one of (15) to (17), wherein the sample is blood, serum, or plasma.

<Definition of Term>

The terms used herein are defined as follows.

The term "malignant brain tumor" used herein refers to any malignant tumor formed in the brain. Specifically, the malignant brain tumor includes glioma and primary central nervous system lymphoma, and the like.

The term "benign brain tumor" used herein refers to any benign tumor formed in the brain. The benign brain tumor includes, but are not particularly limited to, benign meningioma, as a typical example.

Abbreviations or terms such as nucleotide, polynucleotide, DNA, and RNA abide by "Guidelines for the preparation of specification which contain nucleotide and/or amino acid sequences" (edited by Japan Patent Office) and common method in the art.

In the present specification, the term "polynucleotide" is used for a nucleic acid including all of RNA, DNA, and RNA/DNA (chimera). The DNA includes all of cDNA, genomic DNA, and synthetic DNA. The RNA includes all of total RNA, mRNA, rRNA, miRNA, siRNA, snoRNA, snRNA, non-coding RNA and synthetic RNA. In the present specification, the "synthetic DNA" and the "synthetic RNA" refer to a DNA and an RNA artificially prepared using, for example, an automatic nucleic acid synthesizer, on the basis of predetermined nucleotide sequences (which may be any of natural and non-natural sequences). In the present specification, the "non-natural sequence" is intended to be used in a broad sense and includes, for example, a sequence containing substitution, deletion, insertion, and/or addition of one or more nucleotide(s) (i.e., a mutated sequence) and a sequence containing one or more modified nucleotide(s) (i.e., a modified sequence), which are different from the natural sequence. In the present specification, the term "polynucleotide" is used interchangeably with "nucleic acid."

In the present specification, the term "fragment" is a polynucleotide having a nucleotide sequence having a consecutive portion of a polynucleotide and desirably has a length of 15 or more nucleotides, preferably 17 or more nucleotides, more preferably 19 or more nucleotides.

In the present specification, the term "gene" is intended to include not only RNA and double-stranded DNA but each single-stranded DNA such as a plus strand (or a sense strand) or a complementary strand (or an antisense strand) constituting the duplex. The gene is not particularly limited by its length.

Thus, the "gene" used herein includes all of double-stranded DNA including human genomic DNA, single-stranded DNA (plus strand), single-stranded DNA that has a sequence complementary to the plus strand (complementary strand) (e.g., cDNA), microRNA (miRNA), and their fragments, and transcripts, unless otherwise specified. The "gene" includes not only a "gene" represented by a particular nucleotide sequence (or SEQ ID NO) but "nucleic acids" encoding RNAs having biological functions equivalent to an RNA encoded by the gene, for example, a congener (i.e., a homolog or an ortholog), a variant (e.g., a genetic polymorph), and a derivative. Specific examples of such a "nucleic acid" encoding a congener, a variant, or a derivative can include a "nucleic acid" having a nucleotide sequence hybridizing under stringent conditions described later to a complementary sequence of a nucleotide sequence represented by any of SEQ ID NOs: 1 to 39 or a nucleotide sequence derived from the nucleotide sequence by the replacement of u with t. The "gene" is not particularly limited by its functional region and can contain, for example, an expression control region, a coding region, an exon, or an intron. The "gene" may be contained in a cell or may exist alone after being released into the outside of a cell. Alternatively, the "gene" may be in a state enclosed in a vesicle called exosome.

In the present specification, the term "exosome" is a vesicle that is surrounded by a lipid bilayer and secreted from a cell. The exosome is derived from a multivesicular endosome and may incorporate a biomaterial such as a "gene" (e.g., RNA or DNA) or a protein when released into an extracellular environment. The exosome is known to be contained in a body fluid such as blood, serum, plasma, serum, or lymph.

In the present specification, the term "transcript" refers to an RNA synthesized with the DNA sequence of a gene as a template. RNA polymerase binds to a site called promoter located upstream of the gene and adds ribonucleotides complementary to the nucleotide sequence of the DNA to the 3' end to synthesize an RNA. This RNA contains not only the gene itself but the whole sequence from a transcription initiation site to the end of a polyA sequence, including an expression control region, a coding region, an exon, or an intron. The term "transcript" used herein also includes RNA (e.g., miRNA) produced from RNA (e.g., a miRNA precursor) synthesized from the DNA sequence of a gene as a template, unless the context requires otherwise.

The term "microRNA (miRNA)" used herein is intended to typically mean a 15- to 25-nucleotide non-coding RNA (mature miRNA) that is transcribed as an RNA precursor having a hairpin-like structure, cleaved by a dsRNA-cleaving enzyme which has RNase III cleavage activity, integrated into a protein complex called RISC, and involved in the suppression of translation of mRNA, unless otherwise specified. The term "miRNA" used herein includes not only a "miRNA" represented by a particular nucleotide sequence (or SEQ ID NO) but a precursor of the "miRNA" (pre-miRNA or pri-miRNA), and miRNAs that have biological functions equivalent thereto, for example, a congener (i.e., a homolog or an ortholog), a variant (e.g., a genetic polymorph), and a derivative, unless the context refers to only a mature miRNA.

Such a precursor, a congener, a variant, or a derivative can be specifically identified using miRBase Release 21 (http://www.mirbase.org/), and examples thereof can include a "miRNA" having a nucleotide sequence hybridizing under stringent conditions described later to a complementary sequence of a particular nucleotide sequence represented by any of SEQ ID NOs: 1 to 39. The term "miRNA" used in the present specification may be a gene product of a miR gene (gene encoding a miRNA precursor). Such a gene product includes a mature miRNA (e.g., a 15- to 25-nucleotide or 19- to 25-nucleotide non-coding RNA involved in the suppression of translation of mRNA as described above) or a miRNA precursor (e.g., pre-miRNA or pri-miRNA as described above).

In the present specification, the term "probe" includes a polynucleotide that is used for specifically detecting an RNA resulting from the expression of a gene or a polynucleotide derived from the RNA, and/or a polynucleotide complementary thereto.

In the present specification, the term "primer" includes a polynucleotide that specifically recognizes and amplifies an RNA resulting from the expression of a gene or a polynucleotide derived from the RNA, and/or a polynucleotide complementary thereto.

In this context, the complementary polynucleotide (complementary strand or reverse strand) means a polynucleotide in a complementary base relationship based on A:T (U) and G:C base-pairing with the full-length sequence of a polynucleotide consisting of a nucleotide sequence defined by any of SEQ ID NOs: 1 to 39 or a nucleotide sequence derived from the nucleotide sequence by the replacement of u with t, or a partial sequence thereof. The phrase "polynucleotide consisting of a nucleotide sequence complementary" to a nucleotide sequence represented by any of SEQ ID NOs: 1 to 39 or a nucleotide sequence derived from the nucleotide sequence by the replacement of u with t is also basically understood in the same way.

In the present specification, the term "stringent conditions" refers to conditions under which a polynucleotide such as a nucleic acid probe or a primer hybridizes to its target sequence to a larger extent (e.g., a measurement value equal to or larger than a mean of background measurement values + a standard error of the background measurement values x 2) than that for other sequences. The stringent conditions are dependent on a sequence and differ depending on an environment where hybridization is performed. A target sequence complementary 100% to the polynucleotide such as a nucleic acid probe can be identified by controlling the stringency of hybridization and/or washing conditions. Specific examples of the "stringent conditions" will be mentioned later.

In the present specification, the term "Tm value" means a temperature at which the double-stranded moiety of a polynucleotide is denatured into single strands so that the double strands and the single strands exist at a ratio of 1:1.

The term "variant" used herein means, in the case of a nucleic acid, a natural variant attributed to polymorphism, mutation, or the like; a variant containing the deletion, substitution, addition, or insertion of 1 or 2 or more (e.g., one to several) nucleotides in a nucleotide sequence represented by any of SEQ ID NOs: 1 to 39, or a nucleotide sequence derived from the nucleotide sequence by the replacement of u with t, or a partial sequence thereof; a polynucleotide variant consisting of a nucleotide sequence that exhibits percent (%) identity of approximately 90% or higher, approximately 95% or higher, approximately 97% or higher, approximately 98% or higher, approximately 99% or higher to each of the full-length sequences of these nucleotide sequences or the partial sequences thereof; or a nucleic acid that hybridizes under the stringent conditions defined above to a polynucleotide or an oligonucleotide comprising each of the full-length sequences of these nucleotide sequences or the partial sequence thereof.

In the present specification, the term "several" means an integer of approximately 10, 9, 8, 7, 6, 5, 4, 3, or 2.

In the present specification, the variant of polynucleotide can be prepared by use of a well-known technique such as site-directed mutagenesis or PCR-based mutagenesis.

In the present specification, the term "% identity" can be determined with or without an introduced gap using a protein or gene search system based on BLAST or FASTA described above (Zheng Zhang et al., 2000, J. Comput. Biol., Vol. 7, p. 203-214; Altschul, S.F. et al., 1990, Journal of Molecular Biology, Vol. 215, p. 403-410; and Pearson, W.R. et al., 1988, Proc. Natl. Acad. Sci. U. S. A., Vol. 85, p. 2444-2448).

In the present specification, the term "derivative" means a nucleic acid including a modified nucleic acid, for example, a derivative labeled with a fluorophore or the like, a derivative containing a modified nucleotide (e.g., a nucleotide containing a group such as halogen, alkyl such as methyl, alkoxy such as methoxy, thio, or carboxymethyl, and a nucleotide that has undergone base rearrangement, double bond saturation, deamination, replacement of an oxygen molecule with a sulfur atom, etc.), PNA (peptide nucleic acid; Nielsen, P.E. et al., 1991, Science, Vol. 254, p. 1497-500), and LNA (locked nucleic acid; Obika, S. et al., 1998, Tetrahedron Lett., Vol. 39, p. 5401-5404) without any limitation.

In the present specification, the "nucleic acid" capable of specifically binding to a polynucleotide selected from the malignant brain tumor marker miRNA group described above is a synthesized or prepared nucleic acid and specifically includes a "nucleic acid probe" or a "primer". The "nucleic acid" is utilized directly or indirectly for detecting the presence or absence of malignant brain tumor in a subject, for diagnosing the presence or absence or the severity of malignant brain tumor, the presence or absence or the degree of amelioration of malignant brain tumor, or the sensitivity of malignant brain tumor for treatment, or for screening for a candidate substance useful in the prevention, amelioration, or treatment of malignant brain tumor. The "nucleic acid" includes a nucleotide, an oligonucleotide, and a polynucleotide capable of specifically recognizing and binding to a transcript (polynucleotide) represented by any of SEQ ID NOs: 1 to 39 or a synthetic cDNA nucleic acid thereof *in vivo,* particularly, in a sample such as a body fluid (e.g., blood or urine), in relation to the development of malignant brain tumor. The nucleotide, the oligonucleotide, and the polynucleotide can be effectively used as probes for detecting the aforementioned gene expressed *in vivo,* in tissues, in cells, or the like on the basis of the properties described above, or as primers for amplifying the aforementioned gene expressed *in vivo.*

The term "detection" used in the present specification is interchangeable with the term "examination", "measurement", "detection", "discrimination" or "decision support". In the present specification, the term "evaluation" is meant to include diagnosis or evaluation support on the basis of examination results or measurement results.

The term "subject" used in the present specification means a mammal such as a primate including a human and a chimpanzee, a pet animal including a dog and a cat, a livestock animal including cattle, a horse, sheep, and a goat, a rodent including a mouse and a rat, and an animal that is kept in a zoo. The subject is preferably a human. The "subject" having a disease such as malignant brain tumor or benign brain tumor is also referred to as a "patient". The term "healthy subject" also means such a mammal without the cancer to be detected. The healthy subject is preferably a human.

The term "P" or "P value" used in the present specification refers to a probability at which a more extreme statistic than that actually calculated from data under null hypothesis is observed in a statistical test. Thus, smaller "P" or "P value" is regarded as being more significant difference between subjects to be compared.

In the present specification, the term "sensitivity" refers to a ratio of (the number of true positives) / (the number of true positives + the number of false negatives). High sensitivity allows malignant brain tumor to be detected early, leading to the complete resection of cancer sites and reduction in the rate of recurrence.

The term "specificity" used in the present specification refers to a ratio of (the number of true negatives) / (the number of true negatives + the number of false positives). High specificity prevents needless extra examination for healthy subjects erroneously identified as being malignant brain tumor patients, leading to reduction in burden on patients and reduction in medical expense.

The term "accuracy" used in the present specification refers to a ratio of (the number of true positives + the number of true negatives) / (the total number of cases). The accuracy indicates the ratio of samples that are correctly identified in the discriminant results relative to all samples, and serves as a primary index for evaluating discriminant performance (detection performance).

In the present specification, the "sample" that is subject to determination, detection, or diagnosis, etc. refers to a tissue and a biological material in which the expression of the gene of the present invention changes as malignant brain tumor develops, as malignant brain tumor progresses, or as therapeutic effects on malignant brain tumor are exerted. Specifically, the "sample" refers to a brain tissue, a peribiliary vascular vessel, meninges, an organ suspected of having metastasis, skin, a body fluid such as blood, urine, spinal fluid, saliva, sweat, or tissue exudates, serum or plasma prepared from blood, feces, hair, and the like. The determination, detection, or diagnosis, etc. using the above sample also includes the case of using a biological sample extracted therefrom, specifically, a gene such as RNA or miRNA.

The term "hsa-miR-1909-3p gene" or "hsa-miR-1909-3p" used in the present specification includes the hsa-miR-1909-3p gene (miRBase Accession No. MIMAT0007883) set forth in SEQ ID NO: 1, a homolog or an ortholog of a different organism species, and the like. The hsa-miR-1909-3p gene can be obtained by a method described in Bar M et al., 2008, Stem Cells, Vol. 26, p. 2496-2505. Also, "hsa-mir-1909" (miRBase Accession No. MI0008330, SEQ ID NO: 12) having a hairpin-like structure is known as a precursor of "hsa-miR-1909-3p".

The term "hsa-miR-6869-5p gene" or "hsa-miR-6869-5p" used in the present specification includes the hsa-miR-6869-5p gene (miRBase Accession No. MIMAT0027638) set forth in SEQ ID NO: 2, a homolog or an ortholog of a different organism species, and the like. The hsa-miR-6869-5p gene can be obtained by a method described in Ladewig E et al., 2012, Genome Res, Vol. 22, p. 1634-1645. Also, "hsa-mir-6869" (miRBase Accession No. MI0022716, SEQ ID NO: 13) having a hairpin-like structure is known as a precursor of "hsa-miR-6869-5p".

The term "hsa-miR-3178 gene" or "hsa-miR-3178" used in the present specification includes the hsa-miR-3178 gene (miRBase Accession No. MIMAT0015055) set forth in SEQ ID NO: 3, a homolog or an ortholog of a different organism species, and the like. The hsa-miR-3178 gene can be obtained by a method described in Stark MS et al., 2010, PLoS One, Vol. 5, e9685. Also, "hsa-mir-3178" (miRBase Accession No. MI0014212, SEQ ID NO: 14) having a hairpin-like structure is known as a precursor of "hsa-miR-3178".

The term "hsa-miR-4787-5p gene" or "hsa-miR-4787-5p" used in the present specification includes the hsa-miR-4787-5p gene (miRBase Accession No. MIMAT0019956) set forth in SEQ ID NO: 4, a homolog or an ortholog of a different organism species, and the like. The hsa-miR-4787-5p gene can be obtained by a method described in Persson H et al., 2011, Cancer Res, Vol. 71, p. 78-86. Also, "hsa-mir-4787" (miRBase Accession No. MI0017434, SEQ ID NO: 15) having a hairpin-like structure is known as a precursor of "hsa-miR-4787-5p".

The term "hsa-miR-6510-5p gene" or "hsa-miR-6510-5p" used in the present specification includes the hsa-miR-6510-5p gene (miRBase Accession No. MIMAT0025476) set forth in SEQ ID NO: 5, a homolog or an ortholog of a different organism species, and the like. The hsa-miR-6510-5p gene can be obtained by a method described in Joyce CE et al., 2011, Hum Mol Genet, Vol. 20, p. 4025-4040. Also, "hsa-mir-6510" (miRBase Accession No. MI0022222, SEQ ID NO: 16) having a hairpin-like structure is known as a precursor of "hsa-miR-6510-5p".

The term "hsa-miR-4695-5p gene" or "hsa-miR-4695-5p" used in the present specification includes the hsa-miR-4695-5p gene (miRBase Accession No. MIMAT0019788) set forth in SEQ ID NO: 6, a homolog or an ortholog of a different organism species, and the like. The hsa-miR-4695-5p gene can be obtained by a method described in Persson H et al., 2011, Cancer Res, Vol. 71, p. 78-86. Also, "hsa-mir-4695" (miRBase Accession No. MI0017328, SEQ ID NO: 17) having a hairpin-like structure is known as a precursor of "hsa-miR-4695-5p".

The term "hsa-miR-4634 gene" or "hsa-miR-4634" used in the present specification includes the hsa-miR-4634 gene (miRBase Accession No. MIMAT0019691) set forth in SEQ ID NO: 7, a homolog or an ortholog of a different organism species, and the like. The hsa-miR-4634 gene can be obtained by a method described in Persson H et al., 2011, Cancer Res, Vol. 71, p. 78-86. Also, "hsa-mir-4634" (miRBase Accession No. MI0017261, SEQ ID NO: 18) having a hairpin-like structure is known as a precursor of "hsa-miR-4634".

The term "hsa-miR-4449 gene" or "hsa-miR-4449" used in the present specification includes the hsa-miR-4449 gene (miRBase Accession No. MIMAT0018968) set forth in SEQ ID NO: 8, a homolog or an ortholog of a different organism species, and the like. The hsa-miR-4449 gene can be obtained by a method described in Jima DD et al., 2010, Blood, Vol. 116, e118-127. Also, "hsa-mir-4449" (miRBase Accession Nos. MI0016792, SEQ ID NOs: 19) having a hairpin-like structure are known as a precursor of "hsa-miR-4449".

The term "hsa-miR-3195 gene" or "hsa-miR-3195" used in the present specification includes the hsa-miR-3195 gene (miRBase Accession No. MIMAT0015079) set forth in SEQ ID NO: 9, a homolog or an ortholog of a different organism species, and the like. The hsa-miR-3195 gene can be obtained by a method described in Stark MS et al., 2010, PLoS One, Vol. 5, e9685. Also, "hsa-mir-3195" (miRBase Accession No. MI0014240, SEQ ID NO: 20) having a hairpin-like structure is known as a precursor of "hsa-miR-3195".

The term "hsa-miR-6836-3p gene" or "hsa-miR-6836-3p" used in the present specification includes the hsa-miR-6836-3p gene (miRBase Accession No. MIMAT0027575) set forth in SEQ ID NO: 10, a homolog or an ortholog of a different organism species, and the like. The hsa-miR-6836-3p gene can be obtained by a method described in Ladewig E et al., 2012, Genome Res, Vol. 22, p. 1634-1645. Also, "hsa-mir-6836" (miRBase Accession No. MI0022682, SEQ ID NO: 21) having a hairpin-like structure is known as a precursor of "hsa-miR-6836-3p".

The term "hsa-miR-187-5p gene" or "hsa-miR-187-5p" used in the present specification includes the hsa-miR-187-5p gene (miRBase Accession No. MIMAT0004561) set forth in SEQ ID NO: 11, a homolog or an ortholog of a different organism species, and the like. The hsa-miR-187-5p gene can be obtained by a method described in Lim LP et al., 2003, Science, Vol. 299, p. 1540. Also, "hsa-mir-187" (miRBase Accession No. MI0000274, SEQ ID NO: 22) having a hairpin-like structure is known as a precursor of "hsa-miR-187-5p".

A mature miRNA may become a variant due to the sequence cleaved shorter or longer by one to several upstream or downstream nucleotides or nucleotide substitution when cleaved as the mature miRNA from its RNA precursor having a hairpin-like structure. This variant is called isomiR (Morin RD. et al., 2008, Genome Research, Vol. 18, p. 610-621). miRBase Release 21 shows the nucleotide sequences represented by SEQ ID NOs: 1 to 9 and 11 as well as a large number of the polynucleotide variants and fragments represented by SEQ ID NOs: 23 to 39, called isomiRs. These can also be obtained as variants or fragments of miRNAs having a nucleotide sequence represented by any of SEQ ID NOs: 1 to 9 and 11.

Specifically, among the variants of polynucleotides consisting of nucleotide sequences represented by SEQ ID NOs: 1 to 9 and 11 and polynucleotides derived from the nucleotide sequences by the replacement of u with t according to the present invention, the longest variants registered in miRBase Release 21 are polynucleotides consisting of the nucleotide sequences represented by SEQ ID NOs: 23 to 32, respectively. Also, among the variants of polynucleotides consisting of nucleotide sequences represented by SEQ ID NOs: 1, 3, 4, 5, 8, 9, and 11 and polynucleotides derived from the nucleotide sequences by the replacement of u with t according to the present invention, the shortest variants registered in miRBase Release 21 are polynucleotides consisting of the nucleotide sequences represented by SEQ ID NOs: 33 to 39, respectively. In addition to these variants and fragments, examples thereof include a large number of isomiR polynucleotides of miRNA of SEQ ID NOs: 1, 3, 4, 8, 9 and 11 registered in miRBase. Examples of the polynucleotide comprising a nucleotide sequence represented by any of SEQ ID NOs: 1 to 11 include a polynucleotide represented by any of SEQ ID NOs: 12 to 22, which are their respective precursors.

The names and miRBase Accession Nos. (registration numbers) of the genes (miRNA) represented by SEQ ID NOs: 1 to 39 are indicated in Table 1.

In the present specification, the term "capable of specifically binding" means that the nucleic acid, for example, the nucleic acid probe or the primer, used in the present invention binds to a particular target nucleic acid and cannot substantially bind to other nucleic acids.

Table 1

| SEQ ID NO: | Gene name | miRBase Accession No. |
|---|---|---|
| 1 | hsa-miR-1909-3p | MIMAT0007883 |
| 2 | hsa-miR-6869-5p | MIMAT0027638 |
| 3 | hsa-miR-3178 | MIMAT0015055 |
| 4 | hsa-miR-4787-5p | MIMAT0019956 |
| 5 | hsa-miR-6510-5p | MIMAT0025476 |
| 6 | hsa-miR-4695-5p | MIMAT0019788 |
| 7 | hsa-miR-4634 | MIMAT0019691 |
| 8 | hsa-miR-4449 | MIMAT0018968 |
| 9 | hsa-miR-3195 | MIMAT0015079 |
| 10 | hsa-miR-6836-3p | MIMAT0027575 |
| 11 | hsa-miR-187-5p | MIMAT0004561 |
| 12 | hsa-mir-1909 | MI0008330 |
| 13 | hsa-mir-6869 | MI0022716 |
| 14 | hsa-mir-3178 | MI0014212 |
| 15 | hsa-mir-4787 | MI0017434 |
| 16 | hsa-mir-6510 | MI0022222 |
| 17 | hsa-mir-4695 | MI0017328 |
| 18 | hsa-mir-4634 | MI0017261 |
| 19 | hsa-mir-4449 | MI0016792 |
| 20 | hsa-mir-3195 | MI0014240 |
| 21 | hsa-mir-6836 | MI0022682 |
| 22 | hsa-mir-187 | MI0000274 |
| 23 | isomiR example 1 of 1 SEQ ID NO: 1 | - |
| 24 | isomiR example 1 of 1 SEQ ID NO: 2 | - |
| 25 | isomiR example 1 of 1 SEQ ID NO: 3 | - |
| 26 | isomiR example 1 of 1 SEQ ID NO: 4 | - |
| 27 | isomiR example 1 of 1 SEQ ID NO: 5 | - |
| 28 | isomiR example 1 of 1 SEQ ID NO: 6 | - |
| 29 | isomiR example 1 of 1 SEQ ID NO: 7 | - |
| 30 | isomiR example 1 of 1 SEQ ID NO: 8 | - |
| 31 | isomiR example 1 of 1 SEQ ID NO: 9 | - |
| 32 | isomiR example 1 of 1 SEQ ID NO: 11 | - |
| 33 | isomiR example 1 of 2 SEQ ID NO: 1 | - |
| 34 | isomiR example 1 of 2 SEQ ID NO: 3 | - |
| 35 | isomiR example 1 of 2 SEQ ID NO: 4 | - |
| 36 | isomiR example 1 of 2 SEQ ID NO: 5 | - |
| 37 | isomiR example 1 of 2 SEQ ID NO: 8 | - |

(continued)

| SEQ ID NO: | Gene name | miRBase Accession No. |
|---|---|---|
| 38 | isomiR example 1 of 2 SEQ ID NO: 9 | - |
| 39 | isomiR example 1 of 2 SEQ ID NO: 11 | - |

The present specification includes the contents described in the specification and drawings of Japanese Patent Application No. 2016-074717 on which the present application claims the priority.

Advantageous Effects of Invention

According to the present invention, malignant brain tumor can be detected easily and highly accurately.

For example, the presence or absence of malignant brain tumor in a patient can be easily detected (determined) by using, as an indicator, the measurement values of the miRNAs described above in a sample (blood, serum, etc.) of the patient, which can be collected with limited invasiveness.

Brief Description of Drawings

[Figure 1] Figure 1 shows the relationship between the nucleotide sequences of hsa-miR-1909-3p represented by SEQ ID NO: 1 and hsa-miR-1909-5p, which are produced from a precursor hsa-mir-1909 represented by SEQ ID NO: 12.

[Figure 2] Left diagram: the measurement values of hsa-miR-1909-3p (SEQ ID NO: 1) in malignant brain tumor patients (98 persons), benign brain tumor patients (14 persons), and healthy subjects (100 persons) selected as a training cohort were each plotted on the ordinate. Right diagram: the measurement values of hsa-miR-1909-3p (SEQ ID NO: 1) in malignant brain tumor patients (49 persons), benign brain tumor patients (7 persons), and healthy subjects (50 persons) selected as a validation cohort were each plotted on the ordinate.

[Figure 3] Left diagram: a discriminant (z = -1.952 x (hsa-miR-1909-3p expression level) - 1.071 x (hsa-miR-6869-5p expression level) + 30.884) was prepared by Fisher's linear discriminant analysis of the measurement values of hsa-miR-1909-3p (SEQ ID NO: 1) and hsa-miR-6869-5p (SEQ ID NO: 2) in malignant brain tumor patients (98 persons), healthy subjects (100 persons), and benign brain tumor patients (14 persons) selected in a training cohort, and discriminant scores obtained from the discriminant were plotted on the ordinate against the sample groups on the abscissa. The dotted line in the diagram depicts a discriminant boundary that offered a discriminant score of 0 and discriminated between the groups. Right diagram: discriminant scores were obtained using the discriminant prepared from the training cohort as to the measurement values of hsa-miR-1909-3p (SEQ ID NO: 1) and hsa-miR-6869-5p (SEQ ID NO: 2) in malignant brain tumor patients (49 persons), healthy subjects (50 persons), and benign brain tumor patients (7 persons) selected as a validation cohort and were plotted on the ordinate against the sample groups on the abscissa. The dotted line in the diagram depicts the discriminant boundary that offered a discriminant score of 0 and discriminated between the two groups.

[Figure 4] Left diagram (which is the same as the left diagram of Figure 3): a discriminant (z = -1.952 x (hsa-miR-1909-3p expression level) - 1.071 x (hsa-miR-6869-5p expression level) + 30.884) was prepared by Fisher's linear discriminant analysis of the measurement values of hsa-miR-1909-3p (SEQ ID NO: 1) and hsa-miR-6869-5p (SEQ ID NO: 2) in malignant brain tumor patients (98 persons), healthy subjects (100 persons), and benign brain tumor patients (14 persons) selected as a training cohort, and discriminant scores were obtained using the discriminant and were plotted on the ordinate against the sample groups on the abscissa. The dotted line in the diagram depicts a discriminant boundary that offered a discriminant score of 0 and discriminated between the groups. Right diagram: discriminant scores were obtained using the discriminant prepared from the training cohort as to the measurement values of hsa-miR-1909-3p (SEQ ID NO: 1) and hsa-miR-6869-5p (SEQ ID NO: 2) in 37 primary central nervous system lymphoma patients, 6 ependymoma patients, 5 ganglioglioma patients, and 3 pilocytic astrocytoma patients selected as a validation cohort and were plotted on the ordinate against the sample groups on the abscissa. The dotted line in the diagram depicts the discriminant boundary that offered a discriminant score of 0 and discriminated between the two groups.

Description of Embodiments

Hereinafter, the present invention will be described further specifically.

1. Target nucleic acid for malignant brain tumor

A primary target nucleic acid as a malignant brain tumor marker for detecting the presence and/or absence of malignant brain tumor or malignant brain tumor cells using the nucleic acid/polynucleotide (e.g., the nucleic acid probe or the primer) for the detection of malignant brain tumor defined above according to the present invention can be at least one miRNA selected from the group consisting of hsa-miR-1909-3p, hsa-miR-6869-5p, hsa-miR-3178, hsa-miR-4787-5p, hsa-miR-6510-5p, hsa-miR-4695-5p, hsa-miR-4634, hsa-miR-4449, hsa-miR-3195, and hsa-miR-6836-3p. Furthermore, as another malignant brain tumor marker that can be combined with these miRNAs, hsa-miR-187-5p miRNA can also be preferably used as a target nucleic acid. These target nucleic acid miRNAs include, for example, a human gene comprising a nucleotide sequence represented by any of SEQ ID NOs: 1 to 11 (e.g., hsa-miR-1909-3p, hsa-miR-6869-5p, hsa-miR-3178, hsa-miR-4787-5p, hsa-miR-6510-5p, hsa-miR-4695-5p, hsa-miR-4634, hsa-miR-4449, hsa-miR-3195, hsa-miR-6836-3p, and hsa-miR-187-5p, respectively), a congener thereof, a transcript thereof, and a variant and a derivative thereof. In this context, the gene, the congener, the transcript, the variant, and the derivative are as defined above.

The target nucleic acid in a human subject is preferably a human gene comprising a nucleotide sequence represented by any of SEQ ID NOs: 1 to 39 or a transcript thereof, more preferably the transcript, e.g., an miRNA of hsa-miR-1909-3p, hsa-miR-6869-5p, hsa-miR-3178, hsa-miR-4787-5p, hsa-miR-6510-5p, hsa-miR-4695-5p, hsa-miR-4634, hsa-miR-4449, hsa-miR-3195, hsa-miR-6836-3p, and hsa-miR-187-5p or its precursor RNA (pri-miRNA or pre-miRNA).

The first target gene is the hsa-miR-1909-3p gene, a congener thereof, a transcript thereof, or a variant or a derivative thereof. None of the previously known reports show that change in the expression of the gene, the transcript thereof or the like can serve as a marker for malignant brain tumor.

The second target gene is the hsa-miR-6869-5p gene, a congener thereof, a transcript thereof, or a variant or a derivative thereof. None of the previously known reports show that change in the expression of the gene, the transcript thereof or the like can serve as a marker for malignant brain tumor.

The third target gene is the hsa-miR-3178 gene, a congener thereof, a transcript thereof, or a variant or a derivative thereof. None of the previously known reports show that change in the expression of the gene, the transcript thereof or the like can serve as a marker for malignant brain tumor.

The fourth target gene is the hsa-miR-4787-5p gene, a congener thereof, a transcript thereof, or a variant or a derivative thereof. None of the previously known reports show that change in the expression of the gene, the transcript thereof or the like can serve as a marker for malignant brain tumor.

The fifth target gene is the hsa-miR-6510-5p gene, a congener thereof, a transcript thereof, or a variant or a derivative thereof. None of the previously known reports show that change in the expression of the gene, the transcript thereof or the like can serve as a malignant brain tumor.

The sixth target gene is the hsa-miR-4695-5p gene, a congener thereof, a transcript thereof, or a variant or a derivative thereof. None of the previously known reports show that change in the expression of the gene, the transcript thereof or the like can serve as a marker for malignant brain tumor.

The seventh target gene is the hsa-miR-4634 gene, a congener thereof, a transcript thereof, or a variant or a derivative thereof. None of the previously known reports show that change in the expression of the gene, the transcript thereof or the like can serve as a marker for malignant brain tumor.

The eighth target gene is the hsa-miR-4449 gene, a congener thereof, a transcript thereof, or a variant or a derivative thereof. None of the previously known reports show that change in the expression of the gene, the transcript thereof or the like can serve as a marker for malignant brain tumor.

The ninth target gene is the hsa-miR-3195 gene, a congener thereof, a transcript thereof, or a variant or a derivative thereof. None of the previously known reports show that change in the expression of the gene, the transcript thereof or the like can serve as a marker for malignant brain tumor.

The 10th target gene is the hsa-miR-6836-3p gene, a congener thereof, a transcript thereof, or a variant or a derivative thereof. None of the previously known reports show that change in the expression of the gene, the transcript thereof or the like can serve as a marker for malignant brain tumor.

The 11th target gene is the hsa-miR-187-5p gene, a congener thereof, a transcript thereof, or a variant or a derivative thereof. Patent Literature 1 has reported that change in the expression of hsa-miR-187-5p miRNA can serve as a marker for malignant brain tumor.

2. Nucleic acid for detection of malignant brain tumor

In the present invention, a nucleic acid capable of specifically binding to any of the target nucleic acids as the malignant brain tumor markers described above can be used as a nucleic acid, for example, a nucleic acid probe or a primer, for the detection (discrimination) or diagnosis of malignant brain tumor.

In the present invention, the nucleic acid, for example, the nucleic acid probe or the primer, that can be used for detecting malignant brain tumor or for diagnosing malignant brain tumor enables qualitative and/or quantitative measurement of the presence, expression level, or abundance of a target nucleic acid as the malignant brain tumor marker described above, i.e., miR-1909-3p, miR-6869-5p, miR-3178, miR-4787-5p, miR-6510-5p, miR-4695-5p, miR-4634, miR-4449, miR-3195, miR-6836-3p, or miR-187-5p gene, for example, human-derived hsa-miR-1909-3p, hsa-miR-6869-5p, hsa-miR-3178, hsa-miR-4787-5p, hsa-miR-6510-5p, hsa-miR-4695-5p, hsa-miR-4634, hsa-miR-4449, hsa-miR-3195, hsa-miR-6836-3p, or hsa-miR-187-5p gene, or a congener thereof, a transcript thereof, a variant or a derivative thereof, a precursor thereof, or any combination thereof.

The expression level of each target nucleic acid described above is increased or decreased (hereinafter, referred to as "increased/decreased") depending on the type of the target nucleic acid in a subject who has malignant brain tumor as compared with a healthy subject or a benign brain tumor patient. Hence, the nucleic acid of the present invention capable of specifically binding to each target nucleic acid described above can be effectively used for detecting malignant brain tumor by measuring the expression level of the target nucleic acid in a sample (e.g., a body fluid such as blood) derived from a subject (e.g., a human) suspected of having malignant brain tumor and a sample derived from a healthy subject and comparing them. The nucleic acid of the present invention can also be effectively used for specifically detecting malignant brain tumor by discriminating it from benign brain tumor by measuring the expression level of the target nucleic acid in a sample (e.g., a body fluid such as blood) derived from a subject (e.g., a human) suspected of having malignant brain tumor and a sample derived from a benign brain tumor patient and comparing them to discriminating malignant brain tumor from benign brain tumor.

The nucleic acid such as nucleic acid probe or primer for detection of malignant brain tumor that can be used in the present invention, is for example, nucleic acid probe(s) capable of specifically binding to polynucleotide(s) consisting of nucleotide sequence(s) represented by at least one of SEQ ID NOs: 1 to 10, or primer(s) for amplifying polynucleotide(s) consisting of nucleotide sequence(s) represented by at least one of SEQ ID NOs: 1 to 10.

As the nucleic acid such as nucleic acid probe or primer for detection of malignant brain tumor that can be used in the present invention, for example, a nucleic acid probe capable of specifically binding to a polynucleotide consisting of the nucleotide sequence represented by SEQ ID NO: 11, or a primer for amplifying a polynucleotide consisting of the nucleotide sequence represented by of SEQ ID NO: 11 can be further used.

Specifically, the nucleic acids such as nucleic acid probes or primers for detection of malignant brain tumor comprise a combination of one or more polynucleotides selected from a group of polynucleotides comprising nucleotide sequences represented by any of SEQ ID NOs: 1 to 39 or nucleotide sequences derived from the nucleotide sequences by the replacement of u with t, and a group of complementary polynucleotides thereof, a group of polynucleotides respectively hybridizing under stringent conditions (mentioned later) to polynucleotides (e.g., DNAs) consisting of nucleotide sequences complementary to these nucleotide sequences, and a group of complementary polynucleotides thereof, and a group of polynucleotides comprising 15 or more, preferably 17 or more consecutive nucleotides in the nucleotide sequences of these polynucleotide groups. These polynucleotides can be used as nucleic acid probes and primers for detecting the malignant brain tumor markers as target nucleic acids.

More specifically, examples of the nucleic acid/polynucleotide such as the nucleic acid probe or primer for detection of malignant brain tumor that can be used in the present invention include one or more polynucleotide(s) selected from the group consisting of the following polynucleotides (a) to (e):

(a) a polynucleotide consisting of a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,

(b) a polynucleotide comprising a nucleotide sequence represented by any of SEQ ID NOs: 1 to 10,

(c) a polynucleotide consisting of a nucleotide sequence complementary to a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 1 to 10 or a nucleotide sequence derived from the nucleotide sequence by the replacement of u with t, and

(e) a polynucleotide hybridizing under stringent conditions to any of the polynucleotides (a) to (d).

In addition to at least one more polynucleotide(s) selected from the group consisting of the above polynucleotides (a) to (e), the nucleic acid for detection of malignant brain tumor, for example, the nucleic acid probe or primer that can be further used in the present invention can comprise at least one polynucleotide selected from the group consisting of the following polynucleotides (f) to (j):

(f) a polynucleotide consisting of the nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(g) a polynucleotide comprising the nucleotide sequence represented by SEQ ID NO: 11,
(h) a polynucleotide consisting of a nucleotide sequence complementary to the nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(i) a polynucleotide comprising a nucleotide sequence complementary to the nucleotide sequence represented by SEQ ID NO: 11 or a nucleotide sequence derived from the nucleotide sequence by the replacement of u with t, and
(j) a polynucleotide hybridizing under stringent conditions to any of the polynucleotides (f) to (i).

The "fragment thereof comprising 15 or more consecutive nucleotides" of these polynucleotides can comprise the sequences having a range of nucleotides, for example, 15 consecutive nucleotides to less than the total number of nucleotides of the sequence, 17 consecutive nucleotides to less than the total number of nucleotides of the sequence, or 19 consecutive nucleotides to less than the total number of nucleotides of the sequence, though the fragment is not limited thereto.

These polynucleotides or the fragments thereof used in the present invention may each be DNA or may each be RNA. A polynucleotide consisting of a nucleotide sequence derived from a predetermined nucleotide sequence by the replacement of u with t is DNA.

The polynucleotides that can be used in the present invention can each be prepared by use of common techniques such as a DNA recombination technique, PCR, or a method using an automatic DNA/RNA synthesizer.

The DNA recombination technique and the PCR can employ a technique described in, for example, Ausubel et al., Current Protocols in Molecular Biology, John Willey & Sons, US (1993); and Sambrook et al., Molecular Cloning - A Laboratory Manual, Cold Spring Harbor Laboratory Press, US (1989).

The human-derived hsa-miR-1909-3p, hsa-miR-6869-5p, hsa-miR-3178, hsa-miR-4787-5p, hsa-miR-6510-5p, hsa-miR-4695-5p, hsa-miR-4634, hsa-miR-4449, hsa-miR-3195, hsa-miR-6836-3p, and hsa-miR-187-5p represented by SEQ ID NOs: 1 to 11 are known in the art, and their obtainment methods are also known as mentioned above. Therefore, each polynucleotide that can be used as a nucleic acid probe or primer in the present invention can be prepared by cloning the gene.

Such a nucleic acid including a nucleic acid probe or primer for detection of malignant brain tumor can be chemically synthesized using an automated DNA synthesizer. In general, a phosphoramidite method is used in this synthesis, and single-stranded DNA up to approximately 100 nucleotides can be automatically synthesized by this method. The automated DNA synthesizer is commercially available from, for example, Polygen GmbH, ABI, or Applied Biosystems, Inc.

Alternatively, the polynucleotide of the present invention can also be prepared by a cDNA cloning method. The cDNA cloning technique can employ, for example, microRNA Cloning Kit Wako.

In this context, the polynucleotides consisting of nucleotide sequences complementary to the nucleotide sequences represented by SEQ ID NOs: 1 to 11 do not exist as miRNA or a precursor thereof *in vivo.* For example, the nucleotide sequence of hsa-miR-1909-3p represented by SEQ ID NO: 1 is produced from the precursor hsa-mir-1909 represented by SEQ ID NO: 12. This precursor has a hairpin-like structure as shown in Figure 1, and the nucleotide sequences of hsa-miR-1909-3p (SEQ ID NO: 1) and hsa-miR-1909-5p have mismatch sequences with each other. Therefore, a polynucleotide consisting of a nucleotide sequence completely complementary to the nucleotide sequence of hsa-miR-1909-3p represented by SEQ ID NO: 1 is not naturally produced *in vivo.* Likewise, a polynucleotide consisting of a nucleotide sequence completely complementary to the nucleotide sequence represented by any of SEQ ID NOs: 2 to 11 has an artificial nucleotide sequence that does not exist *in vivo.* In this context, the polynucleotide consisting of a nucleotide sequence completely complementary to the nucleotide sequence of interest means a polynucleotide consisting of only a nucleotide sequence complementary to the full-length sequence of the nucleotide sequence of interest.

3. Kit or device for detection of malignant brain tumor

The present invention also provides a kit or a device for the detection of malignant brain tumor, comprising one or more polynucleotide(s) (which may include a variant, a fragment, and a derivative; hereinafter, also referred to as a polynucleotide for detection) that can be used as a nucleic acid probe or primer in the present invention for measuring a target nucleic acid as a malignant brain tumor marker.

The target nucleic acid as a malignant brain tumor marker according to the present invention is preferably one or more nucleic acid(s) selected from the following group 1: miR-1909-3p, miR-6869-5p, miR-3178, miR-4787-5p, miR-6510-5p, miR-4695-5p, miR-4634, miR-4449, miR-3195, and miR-6836-3p. In combination with these malignant brain tumor markers, other malignant brain tumor markers such as miR-187-5p may be further used as target nucleic acids.

The kit or the device of the present invention comprises nucleic acid(s) capable of specifically binding to any of the target nucleic acids as the malignant brain tumor markers described above, preferably one or more polynucleotide(s) selected from the nucleic acids, such as the nucleic acid probes or primers, for the detection of malignant brain tumor as described in Section "2. Nucleic acid for detection of malignant brain tumor" above, specifically, the polynucleotides described in Section 2 above.

Specifically, the kit or the device of the present invention may comprise at least one polynucleotide selected from the group consisting of polynucleotides comprising (or consisting of) the nucleotide sequences represented by SEQ ID NOs: 1 to 11 or a nucleotide sequence derived from the nucleotide sequence by the replacement of u with t, polynucleotides comprising (or consisting of) a nucleotide sequence complementary thereto, variants or derivatives of these polynucleotides, fragments comprising 15 or more consecutive nucleotides of these polynucleotides, and polynucleotides hybridizing under stringent conditions to these polynucleotides.

The polynucleotide fragment that may be contained in the kit or the device of the present invention is, for example, one or more, preferably two or more polynucleotides selected from the following group:

(1) a polynucleotide comprising 15 or more consecutive nucleotides in a nucleotide sequence derived from the nucleotide sequence represented by any of SEQ ID NOs: 1 to 11 by the replacement of u with t, or a complementary nucleotide sequence thereof.

In a preferred embodiment, the polynucleotide is a polynucleotide consisting of a nucleotide sequence represented by any of SEQ ID NOs: 1 to 11 or a nucleotide sequence derived from the nucleotide sequence by the replacement of u with t, a polynucleotide consisting of a complementary sequence thereof, a polynucleotide hybridizing under stringent conditions to any of these polynucleotides, or a polynucleotide fragment thereof comprising 15 or more, preferably 17 or more, more preferably 19 or more consecutive nucleotides of these polynucleotide sequences.

In the present invention, the size of the polynucleotide fragment is the number of nucleotides in the range of, for example, consecutive 15 nucleotides to less than the total number of nucleotides of the polynucleotide sequence, consecutive 17 nucleotides to less than the total number of nucleotides of the sequence, or consecutive 19 nucleotides to less than the total number of nucleotides of the sequence, in the nucleotide sequence of each polynucleotide.

The aforementioned polynucleotide combination constituting the kit or the device of the present invention may be, for example, any combination out of the polynucleotides consisting of the nucleotide sequences represented by SEQ ID NOs indicated in Table 1 mentioned later (SEQ ID NOs: 1 to 11 corresponding to the miRNA markers in Table 1) or nucleotide sequence complementary thereto (complementary sequences). Examples thereof include combinations of nucleic acids capable of specifically binding to each of polynucleotides of the combination of SEQ ID NOs indicated in in Table 6. However, these are given merely for illustrative purposes, and all of various other possible combinations are included in the present invention.

The aforementioned polynucleotide combination constituting the kit or the device for discriminating a malignant brain tumor patient from a healthy subject according to the present invention is desirably, for example, a combination of the aforementioned polynucleotides consisting of a nucleotide sequence represented by each SEQ ID NO of two or more SEQ ID NOs (target nucleic acids) shown in Table 1, or a nucleotide sequence complementary thereto, or a fragment thereof, etc. Usually, a combination of two SEQ ID NOs indicated in Table 1 can produce adequate discriminant performance. Alternatively, three or more thereof may be combined.

Specifically, the combination of two polynucleotides that are used as target nucleic acids for discriminating a malignant brain tumor patient from a benign brain tumor patient and a healthy subject is preferably a combination comprising one or more polynucleotide(s) selected from newly found malignant brain tumor markers represented by SEQ ID NOs: 1 to 10, among the combinations of two polynucleotides selected from the polynucleotides consisting of the nucleotide sequences represented by SEQ ID NOs: 1 to 11. A combination of nucleic acids capable of specifically binding to each of the target nucleic acids in the combination can be used in a kit or a device for discriminating a malignant brain tumor patient from a healthy subject.

The number of the aforementioned cancer type-specific polynucleotides (target nucleic acids) in the combination can be 1, 2, 3, 4, 5 or more for the combination and is more preferably 4 or more for the combination. Usually, the combination of 4 of these polynucleotides can produce adequate performance.

As for the combination of the target nucleic acids (malignant brain tumor markers) used in the present invention, non-limiting examples of the combination of the polynucleotide consisting of the nucleotide sequence represented by SEQ ID NO: 1 or a nucleotide sequence complementary thereto and polynucleotides consisting of nucleotide sequences represented by three SEQ ID NOs selected from the other SEQ ID NOs (SEQ ID NOs: 2 to 10) included in the cancer type-specific polynucleotide group 1 and SEQ ID NO: 11 (miR-187-5p) or nucleotide sequences complementary thereto are listed below:

(1) a combination of SEQ ID NOs: 1, 3, 4, 6 (markers: miR-1909-3p, miR-3178, miR-4787-5p, miR-4695-5p);
(2) a combination of SEQ ID NOs: 1, 3, 5, 6 (markers: miR-1909-3p, miR-3178, miR-6510-5p, miR-4695-5p);
(3) a combination of SEQ ID NOs: 1, 3, 7, 8 (markers: miR-1909-3p, miR-3178, miR-4634, miR-4449);
(4) a combination of SEQ ID NOs: 1, 3, 7, 10 (markers: miR-1909-3p, miR-3178, miR-4634, miR-6836-3p); and
(5) a combination of SEQ ID NOs: 1, 3, 7, 11 (markers: miR-1909-3p, miR-3178, miR-4634, miR-187-5p).

As for the combination of the target nucleic acids (malignant brain tumor markers) used in the present invention, non-limiting examples of the combination of the polynucleotide consisting of the nucleotide sequence represented by SEQ ID NO: 2 or a nucleotide sequence complementary thereto and polynucleotides consisting of nucleotide sequences represented by three SEQ ID NOs selected from the other SEQ ID NOs (SEQ ID NOs: 1 and 3 to 10) included in the cancer type-specific polynucleotide group 1 and SEQ ID NO: 11 (miR-187-5p) or nucleotide sequences complementary thereto are listed below:

(1) a combination of SEQ ID NOs: 2, 3, 5, 7 (markers: miR-6869-5p, miR-3178, miR-6510-5p, miR-4634);
(2) a combination of SEQ ID NOs: 2, 4, 5, 11 (markers: miR-6869-5p, miR-4787-5p, miR-6510-5p, miR-187-5p);
(3) a combination of SEQ ID NOs: 2, 5, 6, 11 (markers: miR-6869-5p, miR-6510-5p, miR-4695-5p, miR-187-5p);
(4) a combination of SEQ ID NOs: 2, 5, 7, 11 (markers: miR-6869-5p, miR-6510-5p, miR-4634, miR-187-5p); and
(5) a combination of SEQ ID NOs: 2, 5, 9, 11 (markers: miR-6869-5p, miR-6510-5p, miR-3195, miR-187-5p).

As for the combination of the target nucleic acids (malignant brain tumor markers) used in the present invention, non-limiting examples of the combination of the polynucleotide consisting of the nucleotide sequence represented by SEQ ID NO: 3 or a nucleotide sequence complementary thereto and polynucleotides consisting of nucleotide sequences represented by three SEQ ID NOs selected from the other SEQ ID NOs (SEQ ID NOs: 1, 2, and 4 to 10) included in the cancer type-specific polynucleotide group 1 and SEQ ID NO: 11 (miR-187-5p) or nucleotide sequences complementary thereto are listed below:

(1) a combination of SEQ ID NOs: 3, 4, 5, 9 (markers: miR-3178, miR-4787-5p, miR-6510-5p, miR-3195);
(2) a combination of SEQ ID NOs: 3, 4, 5, 10 (markers: miR-3178, miR-4787-5p, miR-6510-5p, miR-6836-3p);
(3) a combination of SEQ ID NOs: 3, 4, 5, 11 (markers: miR-3178, miR-4787-5p, miR-6510-5p, miR-187-5p);
(4) a combination of SEQ ID NOs: 3, 5, 9, 10 (markers: miR-3178, miR-6510-5p, miR-3195, miR-6836-3p); and
(5) a combination of SEQ ID NOs: 3, 5, 9, 11 (markers: miR-3178, miR-6510-5p, miR-3195, miR-187-5p).

Nucleic acids capable of specifically binding to each of the target nucleic acids in the combinations listed above can be suitably used as sets of polynucleotides for the detection of malignant brain tumor in the kit or the device of the present invention or the method for detecting (discriminating) malignant brain tumor according to the present invention.

The kit or the device of the present invention may also comprise a polynucleotide that is already known or that will be found in the future, to enable detection of malignant brain tumor, in addition to the polynucleotide(s) (which can include polynucleotide(s) consisting of nucleotide sequence(s) represented by at least one of SEQ ID NOs: 1 to 10 or a nucleotide sequence complementary thereto, and variant(s), fragment(s), and derivative(s) thereof) for the detection of malignant brain tumor according to the present invention as described above.

These polynucleotides contained in the kit of the present invention may be packaged in different containers either individually or in any combination.

The kit of the present invention may further contain a kit for extracting a nucleic acid (e.g., total RNA) from body fluids, cells, or tissues, a fluorescent material for labeling, an enzyme and a medium for nucleic acid amplification, an instruction manual, etc.

The device of the present invention is a device for cancer marker measurement in which nucleic acids such as the polynucleotides according to the present invention described above are bonded or attached to, for example, a solid phase. Examples of the material for the solid phase include plastics, paper, glass, and silicon. The material for the solid phase is preferably a plastic from the viewpoint of easy processability. The solid phase has any shape and is, for example, square, round, reed-shaped, or film-shaped. The device of the present invention includes, for example, a device for measurement by a hybridization technique. Specific examples thereof include blotting devices and nucleic acid arrays (e.g., microarrays, DNA chips, and RNA chips).

The nucleic acid array technique is a technique which involves bonding or attaching the nucleic acids one by one by use of a method [e.g., a method of spotting the nucleic acids using a high-density dispenser called spotter or arrayer onto the surface of the solid phase surface-treated, if necessary, by coating with L-lysine or the introduction of a functional group such as an amino group or a carboxyl group, a method of spraying the nucleic acids onto the solid phase using an inkjet which injects very small liquid droplets by a piezoelectric element or the like from a nozzle, or a method of sequentially synthesizing nucleotides on the solid phase] to prepare an array such as a chip and measuring a target nucleic acid through the use of hybridization using this array.

The kit or the device of the present invention comprises nucleic acids capable of specifically binding to each of the polynucleotides of at least one, preferably at least two, more preferably at least three, most preferably at least five to all of the malignant brain tumor marker miRNAs of group 1 described above. The kit or the device of the present invention can optionally further comprise a nucleic acid capable of specifically binding to the already known malignant brain tumor marker miR-187-5p.

The kit or the device of the present invention can be used for detecting malignant brain tumor as described in "4. Method for detecting malignant brain tumor" below.

4. Method for detecting malignant brain tumor

The present invention further provides a method for detecting malignant brain tumor, comprising using the kit or the device of the present invention (comprising the above-mentioned nucleic acid(s) that can be used in the present invention) described in Section "3. Kit or device for detection of malignant brain tumor" above or the polynucleotide(s) for the detection of malignant brain tumor to measure (preferably *in vitro*) expression level(s) of target nucleic acid(s), specifically, an expression level (typically, a miRNA or miRNA precursor level) of at least one gene selected from the following group: miR-1909-3p, miR-6869-5p, miR-3178, miR-4787-5p, miR-6510-5p, miR-4695-5p, miR-4634, miR-4449, miR-3195, and miR-6836-3p and optionally an expression level (typically, a miRNA or miRNA precursor level) of the miR-187-5p gene in a sample. This method involves measuring expression level(s) of target nucleic acid(s) in a sample (e.g., blood, serum, or plasma) collected from a subject suspected of having malignant brain tumor, and then further conducting analysis using the expression level measurement value(s) of the target nucleic acid(s) in the sample of the subject suspected of having malignant brain tumor, and expression levels (control expression levels) of the same target nucleic acid(s) obtained in the same type of samples as above (e.g., blood, serum, or plasma) collected from a nonmalignant control group (including healthy subjects or benign brain tumor patients), for example, comparing the measured expression level(s) with the control expression levels. This method may comprise evaluating the subject suspected of having malignant brain tumor as having malignant brain tumor if the expression level(s) of the target nucleic acid(s) is statistically significantly different between the groups. This method may comprise using (comparing) the measured expression level(s) of the target nucleic acid(s) in the sample derived from the subject and the expression levels thereof in the nonmalignant control group, and evaluating whether or not the subject has malignant brain tumor (the presence or absence of malignant brain tumor) to detect the presence or absence of malignant brain tumor. The evaluation on whether or not the subject has malignant brain tumor (the presence or absence of malignant brain tumor) may be obtainment of an indicator that indicates the presence or absence of malignant brain tumor. When an indicator value indicating that a subject has malignant brain tumor is obtained, it can be considered that the presence of malignant brain tumor in the subject is detected. When an indicator value indicating that a subject does not have malignant brain tumor is obtained, it can be considered that malignant brain tumor in the subject is not detected.

This method of the present invention enables low invasive early diagnosis of cancer with high sensitivity and specificity and thereby brings about early treatment and improved prognosis. In addition, the method enables monitoring of exacerbation of the disease or the effectiveness of surgical, radiotherapeutic, and chemotherapeutic treatments.

In the method of the present invention, nucleic acids (typically, RNA) that may comprise the malignant brain tumor-derived target nucleic acids may be extracted from the sample such as blood, serum, or plasma according to the present invention, and the nucleic acid extract may be subjected to target nucleic acid detection assay using the kit or the device of the present invention or the polynucleotide(s) for the detection of malignant brain tumor. The method for extracting the nucleic acids to be subjected to target nucleic acid detection assay from the sample particularly preferably involves preparation of nucleic acids with the addition of a reagent for RNA extraction in 3D-Gene(R) RNA extraction reagent from liquid sample kit (Toray Industries, Inc.). A general acidic phenol method (acid guanidinium-phenol-chloroform (AGPC)) may be used, or Trizol(R) (Life Technologies Corp.) may be used. The nucleic acids may be prepared by the addition of a reagent for RNA extraction containing acidic phenol, such as Trizol (Life Technologies Corp.) or Isogen (Nippon Gene Co., Ltd.). Alternatively, a kit such as miRNeasy(R) Mini Kit (Qiagen N.V.) can be used, though the method is not limited thereto.

The present invention also provides use of the kit or the device of the present invention or the polynucleotide(s) for the detection of malignant brain tumor (a polynucleotide capable of specifically binding to one target nucleic acid miRNA described above, or a combination of polynucleotides capable of specifically binding to each of two or more target nucleic acid miRNAs described above) that can be used therein, for detecting *in vitro* an expression product of a malignant brain tumor-derived miR gene in a sample derived from a subject. The present invention also provides use of the kit or the device of the present invention or the polynucleotide(s) for the detection of malignant brain tumor (a polynucleotide capable of specifically binding to one target nucleic acid miRNA described above, or a combination of polynucleotides capable of specifically binding to each of two or more target nucleic acid miRNAs described above), for detecting malignant brain tumor in a subject. The present invention also provides the kit or the device of the present invention or the polynucleotide(s) described above (a polynucleotide capable of specifically binding to one target nucleic acid miRNA described above, or a combination of polynucleotides capable of specifically binding to each of two or more target nucleic acid miRNAs described above), for the detection or diagnosis of malignant brain tumor in a subject. The present invention also provides a diagnostic drug for malignant brain tumor, comprising the polynucleotide(s) described above (a polynucleotide capable of specifically binding to one target nucleic acid miRNA described above, or a combination of polynucleotides capable of specifically binding to each of two or more target nucleic acid miRNAs described above). The polynucleotide(s), the kit and the device, etc. for the detection of malignant brain tumor of the present invention are useful in the diagnosis of malignant brain tumor.

In the method of the present invention, the kit or the device described above comprising a single polypeptide or any possible combination of the polynucleotide for detection of malignant brain tumor that can be used in the present invention as described above is used.

In the detection or (genetic) diagnosis of malignant brain tumor according to the present invention, each polynucleotide contained in the kit or the device of the present invention can be used as a probe or a primer. In the case of using the polynucleotide as a primer, TaqMan$^{(R)}$ MicroRNA Assays from Life Technologies Corp., miScript PCR System from Qiagen N.V., or the like can be used, though the method is not limited thereto.

The polynucleotide contained in the kit or the device of the present invention can be used as a primer or a probe according to a conventional method in a method known in the art for specifically detecting the particular gene, for example, a hybridization technique such as Northern blot, Southern blot, *in situ* hybridization, Northern hybridization, or Southern hybridization, or a quantitative amplification technique such as quantitative RT-PCR. A body fluid such as blood, serum, plasma, or urine of the subject is collected as a sample to be assayed according to the type of the detection method used. Alternatively, total RNA prepared from such a body fluid by the method described above may be used, and various polynucleotides including cDNA prepared on the basis of the RNA may be used.

The kit or the device of the present invention is useful for the diagnosis of malignant brain tumor or the detection of the presence or absence of malignant brain tumor. Specifically, the detection of malignant brain tumor using the kit or the device can be performed by detecting *in vitro* an expression level of a gene using the nucleic acid probe or the primer contained in the kit or the device in a sample such as blood, serum, plasma, or urine from a subject suspected of having malignant brain tumor. The level of a target nucleic acid (malignant brain tumor marker such as miRNA) in the sample such as blood, serum, plasma, or urine of the subject suspected of having malignant brain tumor is measured using polynucleotide(s) consisting of nucleotide sequence(s) represented by at least one of SEQ ID NOs: 1 to 11 or a nucleotide sequence derived from the nucleotide sequence by the replacement of u with t, polynucleotide(s) consisting of a nucleotide sequence complementary to any of these nucleotide sequences, or variant(s) or derivative(s) thereof, or fragment(s) thereof comprising 15 or more consecutive nucleotides, contained in the kit or the device of the present invention. The subject can be evaluated as having malignant brain tumor, for example, by use of a discriminant, if the expression level is statistically significantly different compared with the expression level thereof in the samples such as blood, serum, or plasma, or urine of a nonmalignant control group (healthy subjects or benign brain tumor patients).

The method of the present invention can be combined with a diagnostic imaging method such as CT scanning, MRI (magnetic resonance imaging), or cerebral angiography.

The method for detecting the absence of an expression product of the aforementioned malignant brain tumor-derived miR gene or the presence of the expression product of the aforementioned malignant brain tumor-derived miR gene in a sample using the kit or the device of the present invention comprises: collecting a body fluid such as blood, serum, plasma, or urine of a subject; measuring the expression level of the target gene (miR gene) contained therein using one or more polynucleotide(s) (including variant(s), fragment(s), or derivative(s)) selected from the polynucleotide group for the detection of malignant brain tumor of the present invention; and evaluating the presence or absence of malignant brain tumor or detecting malignant brain tumor. Using the method for detecting malignant brain tumor according to the present invention, for example, the presence or absence of amelioration of the disease or the degree of amelioration thereof in a malignant brain tumor patient who received a therapeutic drug for amelioration of the disease can be evaluated or diagnosed.

The method of the present invention may comprise, for example, the following steps (a), (b), and (c):

(a) contacting *in vitro* a sample from a subject with a polynucleotide in the kit or the device of the present invention, or the polynucleotide for the detection of malignant brain tumor of the present invention;
(b) measuring an expression level of the target nucleic acid in the sample using the above-mentioned polynucleotide as a nucleic acid probe or primer; and
(c) evaluating the presence or absence of malignant brain tumor (cells) in the subject on the basis of measurement results obtained in the step (b).

In the step (a), blood, serum, or plasma can be used as a preferred sample.

In the step (b), the measurement of the expression level can be performed by a technique, for example, a hybridization technique such as a nucleic acid array method, a polynucleotide sequencing technique using a sequencer or the like, or a quantitative amplification technique such as quantitative RT-PCR.

The step (c) may be a step of evaluating whether or not the subject has malignant brain tumor on the basis of the measurement results obtained in the step (b) to detect the presence or absence of malignant brain tumor (cells) in the subject. In the step (c), whether or not the subject has malignant brain tumor can be evaluated (discriminated) on the basis of a discriminant score obtained from the expression level of the target nucleic acid in the sample derived from the subject and a discriminant, if the expression level of the target nucleic acid in the sample derived from the subject is statistically significantly different compared with an expression level of the target nucleic acid in a sample derived from a healthy subject or a benign brain tumor patient (nonmalignant control group) (this expression level is also referred to as a "reference" or "control").

Specifically, the present invention provides a method for detecting malignant brain tumor, comprising measuring an expression level of a target nucleic acid in a sample of a subject using nucleic acid(s) capable of specifically binding to at least one (one or more), preferably at least two, at least three, at least four, or at least five or more target nucleic acid polynucleotide(s) selected from the group consisting of miR-1909-3p, miR-6869-5p, miR-3178, miR-4787-5p, miR-6510-5p, miR-4695-5p, miR-4634, miR-4449, miR-3195, and miR-6836-3p and evaluating *in vitro* whether the subject has malignant brain tumor or the subject does not have malignant brain tumor (whether or not the subject has malignant brain tumor) using the measured expression level and an expression level (control expression level) of a healthy subject or a benign brain tumor patient (nonmalignant control group) measured in the same way as above to detect the presence or absence of malignant brain tumor in the subject.

In the present specification, the term "evaluation" may be evaluation support based on results of *in vitro* examination, not physician’s judgment.

As described above, in a preferred embodiment of the method of the present invention, specifically, miR-1909-3p is hsa-miR-1909-3p, miR-6869-5p is hsa-miR-6869-5p, miR-3178 is hsa-miR-3178, miR-4787-5p is hsa-miR-4787-5p, miR-6510-5p is hsa-miR-6510-5p, miR-4695-5p is hsa-miR-4695-5p, miR-4634 is hsa-miR-4634, miR-4449 is hsa-miR-4449, miR-3195 is hsa-miR-3195, miR-6836-3p is hsa-miR-6836-3p.

In a preferred embodiment of the method of the present invention, specifically, the nucleic acid capable of specifically binding to the target nucleic acid (specifically and typically, probe or primer) is selected from the group consisting of the following polynucleotides (a) to (e):

(a) a polynucleotide consisting of a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,

(b) a polynucleotide comprising a nucleotide sequence represented by any of SEQ ID NOs: 1 to 10,

(c) a polynucleotide consisting of a nucleotide sequence complementary to a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,

(d) a polynucleotide comprising a nucleotide sequence complementary to a nucleotide sequence represented by any of SEQ ID NOs: 1 to 10 or a nucleotide sequence derived from the nucleotide sequence by the replacement of u with t, and

(e) a polynucleotide hybridizing under stringent conditions to any of the polynucleotides (a) to (d).

In the method of the present invention, a nucleic acid capable of specifically binding to a polynucleotide of miR-187-5p may be further used, in addition to those polynucleotides.

In a preferred embodiment, as to the nucleic acid capable of specifically binding to a polynucleotide of miR-187-5p, specifically miR-187-5p is hsa-miR-187-5p.

In a preferred embodiment, specifically, the nucleic acid capable of specifically binding to a polynucleotide of miR-187-5p is further selected from the group consisting of the following polynucleotides (f) to (j):

(f) a polynucleotide consisting of the nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,

(g) a polynucleotide comprising the nucleotide sequence represented by SEQ ID NO: 11,

(h) a polynucleotide consisting of a nucleotide sequence complementary to the nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(i) a polynucleotide comprising a nucleotide sequence complementary to the nucleotide sequence represented by SEQ ID NO: 11 or a nucleotide sequence derived from the nucleotide sequence by the replacement of u with t, and
(j) a polynucleotide hybridizing under stringent conditions to any of the polynucleotides (f) to (i).

Examples of the sample used in the method of the present invention can include body tissues and body fluids such as blood, serum, plasma, urine, and spinal fluid of the subject, preferably blood, serum, and plasma. The sample such as a body tissue or a body fluid may be used directly in expression level measurement, or an RNA-containing nucleic acid sample prepared from any of these samples, or a polynucleotide-containing sample further prepared therefrom may be used for measurement.
In the present specification, the subject refers to a mammal, for example, a human, a monkey, a mouse and a rat, without any limitation, and is preferably a human.
The steps of the method of the present invention can be modified according to the type of the sample to be assayed.
In the case of using RNA as an analyte, the detection of malignant brain tumor in the subject (cells) can comprise, for example, the following steps (a), (b), and (c):

(a) binding RNA prepared from the sample of the subject or a complementary polynucleotide (cDNA) transcribed therefrom to a polynucleotide in the kit or the device of the present invention;
(b) quantitatively or qualitatively measuring the sample-derived RNA or the cDNA synthesized from the RNA, bound with the polynucleotide by a hybridization technique using the polynucleotide as a nucleic acid probe, by quantitative RT-PCR using the polynucleotide as a primer, or by sequencing the polynucleotide (above-mentioned RNA or cDNA) using a sequencer; and
(c) detecting the presence or absence of malignant brain tumor (or change in malignant brain tumor-derived gene expression level) on the basis of the measurement results of the step (b). In the step (c), whether or not the subject has malignant brain tumor may be evaluated on the basis of the measurement results of the step (b) by comparison of the measurement results with measurement results from a healthy subject or a benign brain tumor patient (nonmalignant control group) to detect the presence or absence of malignant brain tumor (or change in malignant brain tumor-derived gene expression level) in the subject. Preferably, the step (a) further comprises washing the kit or the device of the present invention after the binding with the sample-derived RNA or cDNA, with a washing solution such as a buffer solution to remove polynucleotides unbound with the polynucleotide from the kit or the device of the present invention.

For example, various hybridization methods can be used for detecting, examining, evaluating, or diagnosing malignant brain tumor (or change in malignant brain tumor-derived gene expression level) *in vitro* according to the present invention. For example, Northern blot, Southern blot, RT-PCR, DNA chip analysis, *in situ* hybridization, Northern hybridization, Southern hybridization, or a technique of sequencing polynucleotide using a sequencer or the like can be used as the hybridization method.
In the case of using the Northern blot, the presence or absence of expression of each gene or the expression level thereof in the RNA can be detected or measured by use of the nucleic acid probe that can be used in the present invention. Specific examples thereof can include a method which involves labeling the nucleic acid probe (a complementary strand) with a radioisotope ($^{32}P$, $^{33}P$, $^{35}S$, etc.), a fluorescent material, or the like, hybridizing the labeled product with RNA from a sample such as a body tissue or a body fluid (e.g., blood, serum, or plasma) of the subject that is transferred to a nylon membrane or the like according to a conventional method, and then detecting and measuring a signal from the label (radioisotope or fluorescent material) of the formed DNA/RNA duplex using a radiation detector (examples thereof can include BAS-1800 II (Fujifilm Corp.)) or a fluorescence detector (examples thereof can include STORM 865 (GE Healthcare Japan Corp.)).
In the case of using the quantitative RT-PCR, the presence or absence of expression of each gene or the expression level thereof in the RNA can be detected or measured by use of the primer that can be used in the present invention. Specific examples thereof can include a method which involves preparing cDNA from RNA from a sample such as a body tissue or a body fluid (e.g., blood, serum or plasma) of the subject according to a conventional method, hybridizing the cDNA as a template with a pair of primers (of a plus strand and a reverse strand binding to the cDNA) of the present invention such that the region of each target gene can be amplified, performing PCR according to a conventional method and detecting the obtained double-stranded DNA. The method for detecting the double-stranded DNA can include a detection method comprising performing the PCR using the primers labeled in advance with a radioisotope or a fluorescent material, a detection method comprising electrophoresing the PCR product on an agarose gel and staining the double-stranded DNA with ethidium bromide or the like, and a detection method comprising transferring the produced double-stranded DNA to a nylon membrane or the like according to a conventional method and hybridizing the double-stranded DNA to a labeled nucleic acid probe.

In the case of using the sequencer, the presence or absence of gene expression or the expression level thereof in the RNA can be detected or measured from the number of reads by use of the primer that can be used in the present invention. Specific examples thereof can include a method which comprises preparing cDNA from RNA from a sample such as a body tissue or a body fluid (e.g., blood, serum, or plasma) of the subject according to a conventional method, hybridizing the cDNA as a template with a pair of primers (each primer comprising a plus strand or a reverse strand sequence binding to the cDNA) designed such that at least a partial region of a miR gene expression product as a target nucleic acid can be amplified, performing nucleic acid amplification such as PCR according to a conventional method, and detecting or measuring the amplified DNA using a sequencer. The sequencer to be used may be, for example, HiSeq 2500 (Illumina, Inc.) or Ion Proton(TM) System (Thermo Fisher Scientific Inc.). Another example of the method can include a method which comprises detecting or measuring a target nucleic acid by directly applying a sample such as a body tissue or a body fluid (e.g., blood, serum, or plasma) of the subject to a sequencer such as PacBio RS II (Pacific Biosciences of California, Inc.) without the nucleic acid amplification of RNA in the sample.

In the case of using the nucleic acid array technique (nucleic acid array analysis), an RNA chip or a DNA chip in which the nucleic acid probes (single-stranded or double-stranded) of the present invention are attached to a substrate (solid phase) is used. Regions having the attached nucleic acid probes are referred to as probe spots, and regions having no attached nucleic acid probe are referred to as blank spots. A product in which genes are immobilized on a substrate is generally called a nucleic acid chip, a nucleic acid array, a microarray, or the like. The DNA or RNA array includes a DNA or RNA macroarray and a DNA or RNA microarray. In the present specification, the term "chip" includes all of these arrays. 3D-Gene(R) Human miRNA Oligo chip (Toray Industries, Inc.) can be used as the DNA chip, though the DNA chip is not limited thereto.

Examples of the measurement using the DNA chip can include, but are not limited to, a method of detecting and measuring a signal derived from the label on the nucleic acid probe using an image detector (examples thereof can include Typhoon 9410 (GE Healthcare Japan Corp.) and 3D-Gene(R) scanner (Toray Industries, Inc.)).

The "stringent conditions" used in the present specification are, as mentioned above, conditions under which a nucleic acid probe hybridizes to its target sequence to a larger extent (e.g., a measurement value equal to or larger than a mean of background measurement values + a standard deviation of the background measurement values x 2) than that for other sequences.

The stringent conditions are defined by conditions for hybridization and subsequent washing. The hybridization conditions are not limited to but are, for example, conditions involving 30°C to 60°C for 1 to 24 hours in a solution containing SSC, a surfactant, formamide, dextran sulfate, a blocking agent, etc. In this context, 1 x SSC is an aqueous solution (pH 7.0) containing 150 mM sodium chloride and 15 mM sodium citrate. The surfactant includes, for example, SDS (sodium dodecyl sulfate), Triton, or Tween. The hybridization conditions more preferably involve 3 to 10 x SSC and 0.1 to 1% SDS. Examples of the conditions of washing, following the hybridization, which is another condition to define the stringent conditions, can include conditions involving continuous washing at 30°C in a solution containing 0.5 x SSC and 0.1% SDS, at 30°C in a solution containing 0.2 x SSC and 0.1% SDS, and at 30°C in a 0.05 x SSC solution. It is desirable that the complementary strand should maintain its hybridized state with a target plus strand even by washing under such conditions. Specifically, examples of such a complementary strand can include a strand consisting of a nucleotide sequence in a completely complementary relationship with the nucleotide sequence of the target plus strand, and a strand consisting of a nucleotide sequence having at least 80%, preferably at least 85%, more preferably at least 90% or at least 95%, for example, at least 98% or at least 99% identity to the strand.

Other examples of the "stringent conditions" for the hybridization are described in, for example, Sambrook, J. & Russel, D., Molecular Cloning, A LABORATORY MANUAL, Cold Spring Harbor Laboratory Press, published on January 15, 2001, Vol. 1, 7.42 to 7.45 and Vol. 2, 8.9 to 8.17, and can be used in the present invention.

Examples of the conditions for carrying out PCR using a polynucleotide fragment for detection of malignant brain tumor in the kit of the present invention as a primer include treatment for approximately 15 seconds to 1 minute at 5 to 10°C plus a Tm value calculated from the sequence of the primer, using a PCR buffer having composition such as 10 mM Tris-HCL (pH 8.3), 50 mM KCL, and 1 to 2 mM $MgCl_2$. Examples of the method for calculating such a Tm value include Tm value = 2 x (the number of adenine residues + the number of thymine residues) + 4 x (the number of guanine residues + the number of cytosine residues).

In the case of using the quantitative RT-PCR, a commercially available kit for measurement specially designed for quantitatively measuring miRNA, such as TaqMan(R) MicroRNA Assays (Life Technologies Corp.), LNA(R)-based MicroRNA PCR (Exiqon), or Ncode(R) miRNA qRT-PCT kit (Invitrogen Corp.) may be used.

For the calculation of gene expression levels (e.g., a miRNA or miRNA precursor level), statistical treatment described in, for example, Statistical analysis of gene expression microarray data (Speed T., Chapman and Hall/CRC), and A beginner's guide Microarray gene expression data analysis (Causton H.C. et al., Blackwell publishing) can be used in the present invention, though the calculation method is not limited thereto. For example, twice, preferably 3 times, more preferably 6 times the standard deviation of the measurement values of the blank spots are added to the average measurement value of the blank spots on the DNA chip, and probe spots having a signal value equal to or larger than the resulting value can be regarded as detection spots. Alternatively, the average measurement value of the blank spots is regarded as a background and can be subtracted from the measurement values of the probe spots to determine gene expression levels. A missing value for a gene expression level can be excluded from the analyte, preferably replaced with the smallest value of the gene expression level in each DNA chip, or more preferably replaced with a value obtained by subtracting 0.1 from a logarithmic value of the smallest value of the gene expression level. In order to eliminate low-signal genes, only a gene having a gene expression level of $2^6$, preferably $2^8$, more preferably $2^{10}$ or larger, in 20% or more, preferably 50% or more, more preferably 80% or more of the number of measurement samples can be selected as the analyte. Examples of the normalization of the gene expression level include, but are not limited to, global normalization and quantile normalization (Bolstad, B. M. et al., 2003, Bioinformatics, Vol. 19, p. 185-193) as well as a method of making correction by the expression level measurement value of internal control miRNA that is stably expressed among every sample (A. Shimomura et al., 2016, Cancer Sci, DOI: 10.1111).

The present invention also provides a method for detecting (or assisting in the detection of) malignant brain tumor in a subject, comprising measuring an expression level of a target gene in a sample derived from the subject using the polynucleotide, the kit, or the device (e.g., chip) for the detection of malignant brain tumor of the present invention, or a combination thereof, and substituting the expression level of the target gene in the sample derived from the subject into a discriminant (discriminant function) that is prepared with gene expression levels in a sample derived from a subject (or a patient) known to have malignant brain tumor and a sample derived from a healthy subject or a benign brain tumor patient as supervising samples and is capable of discriminating a malignant brain tumor patient from a healthy subject or a benign brain tumor patient, to evaluate the presence or absence of malignant brain tumor, wherein the discriminant.

The present invention further provides the method comprising: a first step of measuring *in vitro* an expression level of a target gene (target nucleic acid) in multiple samples from subjects known to have and/or not have malignant brain tumor, using the polynucleotide, the kit, or the device (e.g., chip) for detection of the present invention, or a combination thereof; a second step of preparing a discriminant with the measurement values of the expression level of the target gene obtained in the first step as supervising samples; a third step of measuring *in vitro* an expression level of the target gene in a sample derived from a subject in the same way as in the first step; and a fourth step of substituting the measurement value of the expression level of the target gene obtained in the third step into the discriminant obtained in the second step, and determining or evaluating the presence or absence of malignant brain tumor in the subject on the basis of the results obtained from the discriminant, wherein the target gene can be detected using the polynucleotide or using a polynucleotide for detection contained in the kit or the device (e.g., chip).

The discriminant used herein can be prepared by use of any discriminant analysis method capable of preparing a discriminant for differentially discriminating a malignant brain tumor patient from a healthy subject, for example, Fisher's linear discriminant analysis, nonlinear discriminant analysis based on Mahalanobis' distance, neural network, Support Vector Machine (SVM), or the like, though the method is not limited to these specific examples.

When a clustering boundary is a straight line or a hyperplane, the linear discriminant analysis is a method for determining the belonging to a cluster using Formula 1 as a discriminant. In this context, x represents an explanatory variable, w represents a coefficient of the explanatory variable, and $w_0$ represents a constant term.

$$f(x) = w_0 + \sum_{i=1}^{n} w_i x_i \qquad \text{Formula 1}$$

Values obtained from the discriminant are referred to as discriminant scores. The measurement values of a newly offered data set can be substituted as explanatory variables into the discriminant to determine clusters on the basis of the signs of the discriminant scores.

The Fisher's linear discriminant analysis, one type of linear discriminant analysis, is a dimension reduction method for selecting a dimension suitable for classification, and constructs a highly discriminating synthetic variable by focusing on the variance of synthetic variables and minimizing the variance of data having the same label (Venables, W.N. et al., Modern Applied Statistics with S. Fourth edition. Springer., 2002). In the Fisher's linear discriminant analysis, direction w of projection is determined so as to maximize Formula 2. In Formula 2, $\mu$ represents an average input, $n_g$ represents the number of data belonging to class g, and $\mu_g$ represents an average input of the data belonging to class g. The numerator and the denominator are between-class variance and within-class variance, respectively, when each data is projected in the direction of the vector w. Discriminant coefficient $w_i$ is determined by maximizing this ratio (Takafumi Kanamori et al., "Pattern Recognition", Kyoritsu Shuppan Co., Ltd. (2009); and Richard O. et al., Pattern Classification Second Edition., Wiley-Interscience, 2000).

$$J(w)=\frac{\sum_{g=1}^{G} n_g\left(w^T\mu_g-w^T\mu\right)\left(w^T\mu_g-w^T\mu\right)^T}{\sum_{g=1}^{G}\sum_{i:y_i=g}\left(w^T x_i-w^T\mu_g\right)\left(w^T x_i-w^T\mu_g\right)} \quad \text{Formula 2}$$

subject to

$$\mu=\sum_{i=1}^{n}\frac{x_i}{n},\quad \mu_g=\sum_{i:u_i=g}^{n}\frac{x_i}{n_g}$$

The Mahalanobis' distance is calculated according to Formula 3 in consideration of data correlation and can be used in nonlinear discriminant analysis for determining a cluster having a closer Mahalanobis' distance from each cluster, as a belonging cluster. In this context, $\mu$ represents a central vector of each cluster, and $S^{-1}$ represents an inverse matrix of the variance-covariance matrix of the cluster. The central vector is calculated from explanatory variable x, and an average vector, a median value vector, or the like can be used.

$$D(x,\mu)=\left\{(x-\mu)^t S^{-1}(x-\mu)\right\}^{\frac{1}{2}} \quad \text{Formula 3}$$

SVM is a discriminant analysis method devised by V. Vapnik (The Nature of Statistical Leaning Theory, Springer, 1995). Particular data points of a data set having known classes are defined as explanatory variables, and classes are defined as objective variables. A boundary plane called hyperplane for correctly classifying the data set into the known classes is determined, and a discriminant for data classification is determined using the boundary plane. Then, the measurement values of a newly offered data set can be substituted as explanatory variables into the discriminant to determine classes. In this respect, the discrimination results may be classes, may be a probability of being classified into correct classes, or may be the distance from the hyperplane. In SVM, a method of nonlinearly converting a feature vector to a high dimension and performing linear discrimination in the space is known as a method for tackling nonlinear problems. An formula in which an inner product of two factors in a nonlinearly mapped space is expressed only by inputs in their original spaces is called kernel. Examples of the kernel can include a linear kernel, a RBF (radial basis function) kernel, and a Gaussian kernel. While highly dimensional mapping is performed according to the kernel, the optimum discriminant, i.e., a discriminant, can be actually constructed by mere calculation according to the kernel, which avoids calculating features in the mapped space (e.g., Hideki Aso et al., Frontier of Statistical Science 6 "Statistics of pattern recognition and learning - New concepts and approaches", Iwanami Shoten, Publishers (2004); Nello Cristianini et al., Introduction to SVM, Kyoritsu Shuppan Co., Ltd. (2008)).

C-support vector classification (C-SVC), one type of SVM, involves preparing a hyperplane by learning with the explanatory variables of two groups and classifying an unknown data set into either of the groups (C. Cortes et al., 1995, Machine Learning, Vol. 20, p. 273-297).

Exemplary calculation of a C-SVC discriminant that can be used in the method of the present invention will be given below. First, all subjects are divided into two groups, i.e., a malignant brain tumor patient group and a healthy subject group. For example, malignant brain tumor examination can be used for confirming each subject as a biliary tract patient or a healthy subject.

Next, a data set consisting of comprehensive gene expression levels of serum-derived samples of the two divided groups (hereinafter, this data set is referred to as a training cohort) is prepared, and a C-SVC discriminant is determined by using genes found to differ clearly in their gene expression levels between the two groups as explanatory variables and this grouping as objective variables (e.g., -1 and +1). An optimizing objective function is represented by Formula 4 wherein e represents all input vectors, y represents an objective variable, a represents a Lagrange's undetermined multiplier vector, Q represents a positive definite matrix, and C represents a parameter for adjusting constrained conditions.

$$\min_{a} \quad \frac{1}{2}a^T Qa - e^T a \qquad \text{Formula 4}$$

*subject to* $y^T a = 0$, $0 \le a_i \le C$, $i$=1,...,$l$,

Formula 5 is a finally obtained discriminant, and a belonging group can be determined on the basis of the sign of a value obtained according to the discriminant. In this formula, x represents a support vector, y represents a label indicating the belonging to a group, a represents the corresponding coefficient, b represents a constant term, and K represents a kernel function.

$$f(x) = \operatorname{sgn}\left(\sum_{i=1}^{l} y_i a_i K(x_i, x) + b\right) \qquad \text{Formula 5}$$

For example, a RBF kernel defined by Formula 6 can be used as the kernel function. In this formula, x represents a support vector, and γ represents a kernel parameter for adjusting the complexity of the hyperplane.

$$K(x_i, x_j) = \exp\left(-r\left\|x_i - x_j\right\|^2\right), \quad r < 0 \qquad \text{Formula 6}$$

In addition, an approach such as neural network, k-nearest neighbor algorithms, decision trees, or logistic regression analysis can be selected as a method for determining or evaluating the presence or absence of malignant brain tumor in a subject or for evaluating the expression level of a malignant brain tumor-derived target gene in a sample derived from a subject by comparison with a control derived from a healthy subject.

The method of the present invention can comprise, for example, the following steps (a), (b), and (c):

(a) measuring an expression level of a target gene (target nucleic acid) in samples already known to be derived from malignant brain tumor patients and samples already known to be derived from healthy subjects or benign brain tumor patients having no malignant brain tumor, using the polynucleotide, the kit, or the device (e.g., DNA chip) for detection according to the present invention;
(b) preparing the discriminants of Formulas 1 to 3, 5, and 6 described above from the measurement values of the expression level measured in the step (a); and
(c) measuring an expression level of the target gene in a sample derived from a subject using the polynucleotide, the kit, or the device (e.g., DNA chip) for detection according to the present invention, substituting the measurement value into the discriminants prepared in the step (b), and, on the basis of the obtained results, determining or evaluating the presence or absence of malignant brain tumor in the subject, or evaluating the malignant brain tumor-derived expression level thereof by comparison with a healthy subject- or benign brain tumor patient-derived control expression level.

In this context, x in Formulas 1 to 3, 5, and 6, represents an explanatory variable and includes a value obtained by measuring a polynucleotide selected from the polynucleotides described above in Section 2, or a fragment thereof, etc. Specifically, the explanatory variable for discriminating a malignant brain tumor patient from a healthy subject or a benign brain tumor patient according to the present invention is a gene expression level selected from, for example, the following expression levels (1) to (2):

(1) gene expression levels in the sera of a malignant brain tumor patient, a healthy subject, and a benign brain tumor patient measured by any polynucleotide such as DNA comprising 15 or more consecutive nucleotides in a nucleotide sequence represented by any of SEQ ID NOs: 1 to 10 or a nucleotide sequence complementary thereto, and
(2) gene expression levels in the sera of a malignant brain tumor, a healthy subject, and a benign brain tumor patient measured by any polynucleotide such as DNA comprising 15 or more consecutive nucleotides in a nucleotide sequence represented by SEQ ID NO: 11 or a nucleotide sequence complementary thereto.

As described above, for the method for determining or evaluating the presence or absence of malignant brain tumor in a subject using a sample derived from the subject, the preparation of a discriminant requires a discriminant prepared in a training cohort. For enhancing the discrimination accuracy of the discriminant, it is necessary for the discriminant to use genes that show clear difference in their expression levels between two groups of a malignant brain tumor patient group and a healthy subject group or two groups of a malignant brain tumor patient group and a benign brain tumor patient group in the training cohort.

Each gene that is used for an explanatory variable in a discriminant is preferably determined as follows. First, comprehensive gene expression levels of a malignant brain tumor patient group and comprehensive gene expression levels of a healthy subject group in a training cohort are used as a data set, the degree of difference in the expression level of each gene between the two groups is determined through the use of, for example, the P value of t test, which is parametric analysis, or the P value of Mann-Whitney's U test or Wilcoxon test, which is nonparametric analysis.

The gene can be regarded as being statistically significant when the critical rate (significance level) of the P value obtained by the test is smaller than, for example, 5%, 1%, or 0.01%.

In order to correct an increased probability of type I error attributed to the repetition of a test, a method known in the art, for example, Bonferroni or Holm method, can be used for the correction (e.g., Yasushi Nagata et al., "Basics of statistical multiple comparison methods", Scientist Press Co., Ltd. (2007)). As an example of the Bonferroni correction, for example, the P value obtained by a test is multiplied by the number of repetitions of the test, i.e., the number of genes used in the analysis, and the obtained value can be compared with a desired significance level to suppress a probability of causing type I error in the whole test.

Instead of the test, the absolute value (fold change) of an expression ratio of a median value of each gene expression level between gene expression levels of a malignant brain tumor patient group and gene expression levels of a healthy subject group may be calculated to select a gene that is used for an explanatory variable in a discriminant. Alternatively, ROC curves may be prepared using gene expression levels of a malignant brain tumor patient group and a healthy subject group, and a gene that is used for an explanatory variable in a discriminant can be selected on the basis of an AUROC value.

Next, a discriminant that can be calculated by various methods described above is prepared using any number of genes having large difference in their gene expression levels determined here. Examples of the method for constructing a discriminant that produces the largest discrimination accuracy include a method of constructing a discriminant in every combination of genes that satisfy the significance level of a P value, and a method of repetitively evaluating the genes for use in the preparation of a discriminant while increasing the number of genes one by one in a descending order of difference in gene expression level (Furey TS. et al., 2000, Bioinformatics., Vol. 16, p. 906-14). A gene expression level of another independent malignant brain tumor patient or healthy subject is substituted as an explanatory variable into this discriminant to calculate discrimination results of the group to which this independent malignant brain tumor patient or healthy subject belongs. Specifically, the found gene set for diagnosis and the discriminant constructed using the gene set for diagnosis can be evaluated in an independent sample group to find a more universal gene set for diagnosis capable of detecting malignant brain tumor and a more universal method for discriminating malignant brain tumor.

Split-sample method is preferably used for evaluating the discrimination performance (generality) of the discriminant. Specifically, a data set is divided into a training cohort and a validation cohort, and gene selection by a statistical test and discriminant preparation are performed from the training cohort. Accuracy, sensitivity, and specificity are calculated using results of discriminating a validation cohort using the discriminant and a true group to which the validation cohort belongs, to evaluate the discrimination performance. Alternatively, instead of dividing a data set, gene selection by a statistical test and discriminant preparation may be performed using all of samples, and accuracy, sensitivity, and specificity can be calculated by the discrimination of newly prepared samples using the discriminant to evaluate the discrimination performance.

For example, the gene set for diagnosis is set to any combination comprising one or two or more of the polynucleotides based on a nucleotide sequence represented by any of SEQ ID NOs: 1 to 10 or a complementary sequence thereof as described above, and optionally further comprising one or two or more of the polynucleotides based on the nucleotide sequence represented by SEQ ID NO: 11 or a complementary sequence thereof. Further, a discriminant is constructed using expression levels of the gene set for diagnosis in samples derived from patients diagnosed with malignant brain tumor as a result of an imaging test or tissue diagnosis and samples derived from benign brain tumor patients or healthy subjects. As a result, the presence or absence of malignant brain tumor in a subject from which an unknown sample is derived can be determined with high accuracy and sensitivity by measuring expression levels of the gene set for diagnosis in the unknown sample.

## Examples

Hereinafter, the present invention will be described further specifically with reference to Examples below. However, the scope of the present invention is not intended to be limited by these Examples.

[Reference Example 1]

<Collection of samples of brain tumor patients and healthy subjects>

Serum samples were each collected using VENOJECT II vacuum blood collecting tube VP-AS109K60 (Terumo Corp.) from 100 healthy subjects, 98 glioma (astrocytoma, oligodendroglioma, or oligoastrocytoma) patients (23 cases with grade II, 25 cases with grade III, and 50 cases with grade IV) as malignant brain tumor patients confirmed to have no primary cancer other than brain tumors, and 14 meningioma patients as benign brain tumor patients after acquisition of informed consent, and used as a training cohort. Likewise, serum samples were each collected using VENOJECT II vacuum blood collecting tube VP-AS109K60 (Terumo Corp.) from 50 healthy subjects, 49 glioma (astrocytoma, oligodendroglioma, or oligoastrocytoma) patients (7 cases with grade II, 13 cases with grade III, and 29 cases with grade IV) as malignant brain tumor patients confirmed to have no primary cancer in organs other than the brain, and 7 meningioma patients as benign brain tumor patients after acquisition of informed consent, and used as a validation cohort.

<Extraction of total RNA>

Total RNA was obtained using a reagent for RNA extraction in 3D-Gene(R) RNA extraction reagent from liquid sample kit (Toray Industries, Inc.) according to the protocol provided by the manufacturer, from 300 μL of the serum sample obtained from each of 318 persons in total of 150 healthy subjects, 147 malignant brain tumor patients and 21 benign brain tumor patients included in the training cohort and the validation cohort.

<Measurement of gene expression level>

miRNAs in the total RNA obtained from the serum sample of each of 318 persons in total of 150 healthy subjects, 147 malignant brain tumor patients and 21 benign brain tumor patients included in the training cohort and the validation cohort were fluorescently labeled using 3D-Gene(R) miRNA Labeling kit (Toray Industries, Inc.) according to the protocol (ver. 2.20) provided by the manufacturer. The oligo DNA chip used was 3D-Gene(R) Human miRNA Oligo chip (Toray Industries, Inc.) with mounted probes having sequences complementary to 2,565 miRNAs among the miRNAs registered in miRBase Release 21. Hybridization between the miRNAs in the total RNA and the probes on the DNA chip under stringent conditions and washing following the hybridization were performed according to the protocol provided by the manufacturer. The DNA chip was scanned using 3D-Gene(R) scanner (Toray Industries, Inc.) to obtain images. Fluorescence intensity was digitized using 3D-Gene(R) Extraction (Toray Industries, Inc.). The digitized fluorescence intensity was converted to a logarithmic value having a base of 2 and used as a gene expression level, from which a blank value was subtracted. A missing value was replaced with a value obtained by subtracting 0.1 from a logarithmic value of the smallest value of the gene expression level in each DNA chip. As a result, the comprehensive gene expression levels of the miRNAs in the sera were obtained for 318 persons in total of the 150 healthy subjects, the 147 malignant brain tumor patients and 21 benign brain tumor patients. Calculation and statistical analysis using the digitized gene expression levels of the miRNAs were carried out using R language 3.0.2 (R Development Core Team (2013). R: A language and environment for statistical computing. R Foundation for Statistical Computing, URL http://www.R-project.org/.) and MASS package 7.3-30 (Venables, W. N. & Ripley, B. D. (2002) Modern Applied Statistics with S. Fourth Edition. Springer, New York. ISBN 0-387-95457-0).

[Reference Example 2]

<Collection of samples of other malignant brain tumors>

Sera were each collected using VENOJECT II vacuum blood collecting tube VP-AS109K60 (Terumo Corp.) from 51 persons in total of 37 primary central nervous system lymphoma patients, 6 ependymoma patients, 5 ganglioglioma patients, and 3 pilocytic astrocytoma patients confirmed to have no cancer other than brain tumor after acquisition of informed consent, and used as a validation cohort. Subsequent extraction of total RNA and measurement and analysis of gene expression levels were conducted in the same way as in Reference Example 1.

[Example 1]

<Selection of gene marker using samples in the training cohort, and method for evaluating brain tumor discriminant performance of single gene marker using samples in the validation cohort>

In this Example, a gene marker for discriminating a malignant brain tumor patient from a benign brain tumor patient and a healthy subject was selected from the training cohort, and a method for evaluating malignant brain tumor discriminant performance of each selected gene marker alone was studied in samples of the validation cohort independent from the training cohort.

Specifically, first, the miRNA expression levels of the training cohort and the validation cohort obtained in Reference Example 1 above were normalized. The normalization was carried out in a manner that the ratio of the average of expression level measurement values of three internal miRNA controls (hsa-miR-2861, hsa-miR-149-3p, and hsa-miR-4463) on a DNA chip relative to the pre-set value is determined for each sample, and this ratio is applied to all detection values of miRNAs in each sample. This approach is described in A. Shimomura et al., 2016, Cancer Sci., DOI: 10.1111.

Next, genes for diagnosis were selected using the training cohort. Here, in order to acquire diagnostic markers with higher reliability, only genes having the gene expression level of $2^6$ or higher in 50% or more of the samples in either of the malignant brain tumor patient group in the training cohort or the benign brain tumor patient plus healthy subject group in the training cohort were selected. In order to further acquire statistically significant genes for discriminating a malignant brain tumor patient group from a benign brain tumor patient group and a healthy subject group, the P value obtained by two-tailed t-test assuming equal variance as to each gene expression level was corrected by the Bonferroni method, and genes that satisfied $p < 0.01$ were acquired as gene markers for use in explanatory variables of a discriminant. The obtained genes are indicated in Table 2.

In this way, hsa-miR-1909-3p, hsa-miR-6869-5p, hsa-miR-3178, hsa-miR-4787-5p, hsa-miR-6510-5p, hsa-miR-4695-5p, hsa-miR-4634, hsa-miR-4449, hsa-miR-3195, hsa-miR-6836-3p, hsa-miR-187-5p genes represented by SEQ ID NOs: 1 to 11 were found as malignant brain tumor markers relative to the benign brain tumor patients and the healthy subjects.

A discriminant for determining the presence or absence of malignant brain tumor was further prepared by Fisher's linear discriminant analysis with the expression levels of these genes as an indicator. Specifically, the expression level measurement value of a polynucleotide consisting of the nucleotide sequence represented by each of SEQ ID NOs: 1 to 11 corresponding to the 11 genes selected in the training cohort was applied to Formula 2 to construct a discriminant "z = a x (gene expression level) + b". Calculated accuracy, sensitivity, and specificity are shown in Table 3. In this respect, a discriminant coefficient a and a constant term b are shown in Table 4. For example, the discriminant for the hsa-miR-1909-3p gene (SEQ ID NO: 1) is "z = 3.058 x (hsa-miR-1909-3p expression level) - 26.421" on the basis of the discriminant coefficient a (3.058) and the constant term b (26.421) indicated in Table 4.

Accuracy, sensitivity, and specificity in the validation cohort were calculated using the discriminant thus prepared, and the discriminant performance of the selected polynucleotides was validated using the independent samples (Table 3). For example, the expression level measurement value of the nucleotide sequence represented by SEQ ID NO: 1 was compared between the malignant brain tumor patients (98 persons), and the benign brain tumor patients (14 persons) and the healthy subjects (100 persons) in the training cohort. As a result, the gene expression level measurement values were found to be significantly lower in the malignant brain tumor patient group than in the benign brain tumor patient and healthy subject groups (see the left diagram of Figure 2). These results were also reproducible between the malignant brain tumor patients (49 persons), and the benign brain tumor patients (7 persons) and the healthy subjects (50 persons) in the validation cohort (see the right diagram of Figure 2). Likewise, the results obtained about the other polynucleotides shown in SEQ ID NOs: 2 to 11 showed that the gene expression level measurement values were significantly lower (-) or higher (+) in the malignant brain tumor patient group than in the benign brain tumor patient and healthy subject groups (Table 2). These results were able to be validated in the validation cohort. For example, as for this nucleotide sequence represented by SEQ ID NO: 1, a discriminant score was calculated using the discriminant coefficient (3.058) and the constant term (26.421) indicated in Table 4 for determining the presence or absence of malignant brain tumor in the training cohort, and subsequently, the number of correctly identified samples in the detection of malignant brain tumor in the validation cohort was calculated using the discriminant that determined a sample having a score larger than 0 as being derived from malignant brain tumor and a sample having a score smaller than 0 as being derived from a benign brain tumor patient or a healthy subject. As a result, 41 true positives, 54 true negatives, 3 false positives, and 8 false negatives were obtained. From these values, 90% accuracy, 84% sensitivity, and 95% specificity were obtained as the discriminant performance. In this way, the discriminant performance was calculated as to all of the polynucleotides shown in SEQ ID NOs: 1 to 11, and was shown in Table 3.

The polynucleotides consisting of the nucleotide sequences represented by SEQ ID NOs: 2 to 11 exhibited sensitivity of 84%, 84%, 84%, 80%, 76%, 84%, 74%, 76%, 84%, and 71%, respectively, in the validation cohort (Table 3). These results demonstrated that these polynucleotides can discriminate, each alone, malignant brain tumor with high sensitivity beyond 70%.

Table 2

| SEQ ID NO: | Gene name | P value after Bonferroni correction | Expression level in malignant brain tumor patient relative to healthy subject/benign brain tumor patient |
|---|---|---|---|
| 1 | hsa-miR-1909-3p | 2.2E-36 | + |
| 2 | hsa-miR-6869-5p | 1.8E-34 | + |
| 3 | hsa-miR-3178 | 3.0E-31 | + |
| 4 | hsa-miR-4787-5p | 9.4E-30 | + |
| 5 | hsa-miR-6510-5p | 2.0E-27 | - |
| 6 | hsa-miR-4695-5p | 5.3E-21 | - |
| 7 | hsa-miR-4634 | 8.3E-17 | + |
| 8 | hsa-miR-4449 | 2.0E-15 | + |
| 9 | hsa-miR-3195 | 1.1E-13 | + |
| 10 | hsa-miR-6836-3p | 1.7E-13 | + |
| 11 | hsa-miR-187-5p | 1.7E-10 | + |

Table 3

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 1 | 89 | 84 | 93 | 90 | 84 | 95 |
| 2 | 85 | 81 | 89 | 88 | 84 | 91 |
| 3 | 83 | 81 | 85 | 83 | 84 | 83 |
| 4 | 82 | 79 | 85 | 84 | 84 | 84 |
| 5 | 85 | 75 | 95 | 85 | 80 | 90 |
| 6 | 79 | 77 | 82 | 80 | 76 | 84 |
| 7 | 78 | 75 | 82 | 83 | 84 | 83 |
| 8 | 77 | 75 | 79 | 75 | 74 | 75 |
| 9 | 76 | 74 | 79 | 75 | 76 | 74 |
| 10 | 78 | 75 | 82 | 81 | 84 | 79 |
| 11 | 73 | 72 | 74 | 75 | 71 | 77 |

Table 4

| SEQ ID NO: | Discriminant coefficient a | Constant term b |
|---|---|---|
| 1 | 3.058 | 26.421 |
| 2 | 1.807 | 23.646 |
| 3 | 2.412 | 28.379 |
| 4 | 2.669 | 35.404 |
| 5 | 1.525 | 10.497 |
| 6 | 2.409 | 20.026 |
| 7 | 1.934 | 16.978 |
| 8 | 1.558 | 10.452 |
| 9 | 1.808 | 14.497 |
| 10 | 1.368 | 11.506 |
| 11 | 1.530 | 11.695 |

[Example 2]

<Method for evaluating brain tumor discriminant performance by combination of multiple gene markers using samples in the validation cohort>

In this Example, a method for evaluating malignant brain tumor discriminant performance by a combination of the gene markers selected in Example 1 was studied.

Specifically, the miRNA expression levels of the training cohort and the validation cohort obtained in Reference Example 1 above were normalized as described in Example 1. Next, Fisher's linear discriminant analysis was conducted using the training cohort as to 550 combinations of the expression level measurement values of two to four of the polynucleotides consisting of the nucleotide sequences represented by SEQ ID NOs: 1 to 11 corresponding to the 11 genes selected in Example 1, to construct a discriminant "z = a1 x (expression level of gene 1) + a2 x (expression level of gene 2) + a3 x (expression level of gene 3) + a4 x (expression level of gene 4) + b" for determining the presence or absence of malignant brain tumor (Table 5). Calculated accuracy, sensitivity, and specificity are shown in Table 6.

Next, accuracy, sensitivity, and specificity in the validation cohort were calculated using the discriminant thus prepared, and the discriminant performance was validated using the independent samples.

The presence or absence of brain tumor in the validation cohort of Reference Example 1 was determined using the combinations of the expression level measurement values of any two to four of the polynucleotides consisting of the nucleotide sequences represented by SEQ ID NOs: 1 to 11. For example, as for the expression level measurement values of the polynucleotides consisting of the nucleotide sequences represented by SEQ ID NO: 1 and SEQ ID NO: 2, a discriminant score was calculated using the discriminant coefficients (SEQ ID NO: 1: -1.952, SEQ ID NO: 2: -1.071) and the constant term (-30.884) indicated in Table 5 on the basis of the discriminant prepared for determining the presence or absence of malignant brain tumor in the training cohort. Discriminant results were obtained from the discriminant scores by determining a sample having a discriminant score larger than 0 as being derived from malignant brain tumor and a sample having a discriminant score smaller than 0 as being derived from a benign brain tumor patient or a healthy subject. From the discriminant results, a scatter diagram that significantly separated the expression level measurement values of the malignant brain tumor patient group from those of the healthy subject group and the benign brain tumor patient group was obtained in the training cohort (see the left diagram of Figure 3). These results were also reproducible in the validation cohort (see the right diagram of Figure 3). As for these nucleotide sequences represented by SEQ ID NO: 1 and SEQ ID NO: 2, the number of correctly identified samples in the detection of malignant brain tumor was calculated using the discriminant constructed in the training cohort. As a result, 45 true positives, 55 true negatives, 2 false positives, and 4 false negatives were obtained. From these values, 94% accuracy, 92% sensitivity, and 97% specificity were obtained as the discriminant performance.

In this way, the discriminant performance was calculated for all combinations of the expression level measurement values of two to four of the polynucleotides consisting of the nucleotide sequences represented by SEQ ID NOs: 1 to 11. Among the 550 combinations in total (Table 6), 486 combinations were found to exhibit sensitivity beyond the discriminant performance of each gene marker alone. In Tables 5 and 6, "SEQ ID NO:" represents SEQ ID NOs of each combination of the multiple polynucleotides used (the same applies to the tables mentioned later herein).

For example, use of the combinations of the expression level measurement values of the polynucleotides consisting of the nucleotide sequences represented by SEQ ID NOs: 3 and 5; SEQ ID NOs: 3, 5, and 6; SEQ ID NOs: 3, 5, and 7; SEQ ID NOs: 3, 5, and 8; SEQ ID NOs: 3, 5, and 10; SEQ ID NOs: 3, 5, and 11; SEQ ID NOs: 3, 4, 5, and 6; SEQ ID NOs: 3, 4, 5, and 7,; SEQ ID NOs: 3, 4, 5, and 8,; SEQ ID NOs: 3, 5, 6, and 7; SEQ ID NOs: 3, 5, 6, and 8; SEQ ID NOs: 3, 5, 6, and 9; SEQ ID NOs: 3, 5, 6, and 10; SEQ ID NOs: 3, 5, 6, and 11; SEQ ID NOs: 3, 5, 7, and 8; SEQ ID NOs: 3, 5, 7, and 9; SEQ ID NOs: 3, 5, 7, and 10; SEQ ID NOs: 3, 5, 7, and 11; SEQ ID NOs: 3, 5, 8, and 9; SEQ ID NOs: 3, 5, 8, and 11; SEQ ID NOs: 3, 5, 10, and 11; SEQ ID NOs: 4, 5, 6, and 11; and SEQ ID NOs: 5, 6, 7, and 8 exhibited sensitivity of 98% beyond the highest sensitivity of 84% of each gene marker alone, in the validation cohort.

All of the polynucleotides of SEQ ID NOs: 1 to 11 indicated in Table 1 obtained in Example 1 were found at least once in these combinations. These results demonstrated that the combinations of the expression level measurement values of two to four polynucleotides including one or more of the polynucleotides consisting of the nucleotide sequences represented by SEQ ID NOs: 1 to 11 can discriminate malignant brain tumor with high sensitivity beyond sensitivity of 70% in the validation cohort.

Thus, markers capable of detecting malignant brain tumor with excellent sensitivity are obtained when 2, 3, 4, 5 or even more of the expression level measurement values of the polynucleotides consisting of the nucleotide sequences represented by SEQ ID NOs: 1 to 11 are combined. For example, the polynucleotides consisting of the nucleotide sequences represented by SEQ ID NOs: 1 to 11 selected in Example 1 were ranked in the descending order of their P values which indicate statistical significance, and discriminant performance was calculated using combinations of one or more miRNAs to which the miRNAs were added one by one from the top to the bottom according to the rank. As a result, the sensitivity in the validation cohort was 84% for one miRNA, 92% for two miRNAs, 94% for three miRNAs, and 94% for five miRNAs. The sensitivity of these combinations of the multiple miRNAs was higher than the sensitivity of one miRNA, which demonstrates that combinations of multiple miRNAs can serve as excellent markers for the detection of malignant brain tumor. In this context, the combinations of the multiple miRNAs are not limited to the combinations of the miRNAs in the order of statistically significant difference as described above, and any combination of the multiple miRNAs can be used in the detection of malignant brain tumor.

From these results, it can be concluded that all of the polynucleotides consisting of the nucleotide sequences represented by SEQ ID NOs: 1 to 11 serve as excellent diagnostic markers for malignant brain tumor.

Table 5

| SEQ ID NO: | Discriminant coefficient | | | | Constant term b |
|---|---|---|---|---|---|
| | Term 1 a1 | Term 2 a2 | Term 3 a3 | Term 4 a4 | |
| 1_2 | -1.952 | -1.071 | | | -30.884 |
| 1_3 | -2.141 | -1.453 | | | -35.599 |
| 1_4 | -2.189 | -1.573 | | | -39.776 |
| 1_5 | -2.212 | 0.755 | | | -13.912 |
| 1_6 | -2.442 | 1.079 | | | -12.131 |
| 1_7 | -2.602 | -0.642 | | | -28.121 |
| 1_8 | -2.613 | -0.660 | | | -27.009 |
| 1_9 | -2.823 | -0.289 | | | -26.713 |
| 1_10 | -2.696 | -0.414 | | | -26.782 |
| 1_11 | -2.845 | -0.270 | | | -26.649 |
| 2_3 | -1.143 | -1.190 | | | -28.961 |
| 2_4 | -1.254 | -1.017 | | | -29.892 |
| 2_5 | -1.338 | 0.945 | | | -11.009 |
| 2_6 | -1.417 | 1.157 | | | -8.928 |
| 2_7 | -1.764 | -0.073 | | | -23.729 |
| 2_8 | -1.527 | -0.608 | | | -24.070 |
| 2_9 | -1.617 | -0.406 | | | -24.412 |
| 2_10 | -1.638 | -0.267 | | | -23.678 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 2_11 | -1.622 | -0.462 | | | -24.765 |
| 3_4 | -1.461 | -1.189 | | | -32.961 |
| 3_5 | -1.988 | 1.190 | | | -15.198 |
| 3_6 | -1.885 | 1.434 | | | -10.269 |
| 3_7 | -3.038 | 0.674 | | | -29.826 |
| 3_8 | -2.023 | -0.508 | | | -27.206 |
| 3_9 | -2.035 | -0.795 | | | -30.318 |
| 3_10 | -2.505 | 0.085 | | | -28.760 |
| 3_11 | -2.364 | -0.059 | | | -28.267 |
| 4_5 | -2.055 | 1.126 | | | -19.507 |
| 4_6 | -2.045 | 1.438 | | | -15.170 |
| 4_7 | -3.201 | 0.511 | | | -37.960 |
| 4_8 | -2.187 | -0.579 | | | -32.899 |
| 4_9 | -2.226 | -0.785 | | | -35.816 |
| 4_10 | -2.795 | 0.100 | | | -36.228 |
| 4_11 | -2.431 | -0.296 | | | -34.509 |
| 5_6 | 1.086 | 1.269 | | | 18.024 |
| 5_7 | 1.309 | -1.358 | | | -2.913 |
| 5_8 | 1.308 | -1.041 | | | 2.020 |
| 5_9 | 1.247 | -0.854 | | | 1.741 |
| 5_10 | 1.291 | -0.798 | | | 2.170 |
| 5_11 | 1.376 | -0.941 | | | 2.282 |
| 6_7 | 1.742 | -1.188 | | | 4.055 |
| 6_8 | 1.813 | -0.958 | | | 8.643 |
| 6_9 | 1.810 | -0.902 | | | 7.811 |
| 6_10 | 1.843 | -0.750 | | | 9.011 |
| 6_11 | 1.966 | -0.798 | | | 10.239 |
| 7_8 | -1.227 | -0.888 | | | -16.734 |
| 7_9 | -1.312 | -0.972 | | | -19.322 |
| 7_10 | -1.424 | -0.448 | | | -16.273 |
| 7_11 | -1.548 | -0.456 | | | -17.079 |
| 8_9 | -1.023 | -1.052 | | | -15.303 |
| 8_10 | -1.019 | -0.768 | | | -13.295 |
| 8_11 | -1.166 | -0.578 | | | -12.243 |
| 9_10 | -1.085 | -0.804 | | | -15.464 |
| 9_11 | -1.271 | -0.846 | | | -16.660 |
| 10_11 | -0.951 | -0.650 | | | -12.969 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 1_2_3 | -1.849 | -0.581 | -0.993 | | -35.261 |
| 1_2_4 | -1.957 | -0.535 | -1.010 | | -37.306 |
| 1_2_5 | -1.326 | -1.000 | 0.671 | | -19.926 |
| 1_2_6 | -1.682 | -0.934 | 0.770 | | -20.352 |
| 1_2_7 | -1.973 | -1.143 | 0.137 | | -30.807 |
| 1_2_8 | -1.816 | -0.935 | -0.445 | | -30.919 |
| 1_2_9 | -1.991 | -1.090 | 0.073 | | -30.884 |
| 1_2_10 | -1.920 | -1.024 | -0.095 | | -30.797 |
| 1_2_11 | -1.867 | -1.051 | -0.138 | | -30.943 |
| 1_3_4 | -2.089 | -0.895 | -0.744 | | -38.444 |
| 1_3_5 | -1.131 | -1.640 | 0.905 | | -22.836 |
| 1_3_6 | -1.735 | -1.355 | 0.901 | | -23.436 |
| 1_3_7 | -2.300 | -2.448 | 1.107 | | -38.962 |
| 1_3_8 | -2.066 | -1.271 | -0.303 | | -34.838 |
| 1_3_9 | -2.024 | -1.436 | -0.162 | | -35.678 |
| 1_3_10 | -2.202 | -1.743 | 0.284 | | -37.153 |
| 1_3_11 | -2.252 | -1.649 | 0.298 | | -36.584 |
| 1_4_5 | -1.358 | -1.627 | 0.791 | | -27.867 |
| 1_4_6 | -1.798 | -1.447 | 0.879 | | -27.414 |
| 1_4_7 | -2.349 | -2.579 | 1.011 | | -45.629 |
| 1_4_8 | -2.090 | -1.362 | -0.343 | | -38.430 |
| 1_4_9 | -2.103 | -1.553 | -0.121 | | -39.747 |
| 1_4_10 | -2.254 | -1.942 | 0.312 | | -42.603 |
| 1_4_11 | -2.243 | -1.637 | 0.115 | | -40.209 |
| 1_5_6 | -1.951 | 0.598 | 0.793 | | -6.149 |
| 1_5_7 | -1.438 | 0.912 | -0.903 | | -14.080 |
| 1_5_8 | -1.677 | 0.831 | -0.741 | | -13.741 |
| 1_5_9 | -2.021 | 0.748 | -0.250 | | -14.315 |
| 1_5_10 | -1.784 | 0.794 | -0.466 | | -13.871 |
| 1_5_11 | -1.702 | 0.874 | -0.499 | | -12.504 |
| 1_6_7 | -2.108 | 1.006 | -0.546 | | -14.645 |
| 1_6_8 | -2.130 | 0.970 | -0.587 | | -14.276 |
| 1_6_9 | -2.365 | 1.052 | -0.115 | | -12.610 |
| 1_6_10 | -2.207 | 1.000 | -0.331 | | -13.543 |
| 1_6_11 | -2.262 | 1.066 | -0.243 | | -12.539 |
| 1_7_8 | -2.440 | -0.351 | -0.554 | | -27.879 |
| 1_7_9 | -2.525 | -0.608 | -0.125 | | -28.162 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 1_7_10 | -2.576 | -0.449 | -0.188 | | -27.779 |
| 1_7_11 | -2.605 | -0.648 | 0.010 | | -28.129 |
| 1_8_9 | -2.588 | -0.651 | -0.038 | | -27.042 |
| 1_8_10 | -2.486 | -0.572 | -0.218 | | -27.152 |
| 1_8_11 | -2.675 | -0.726 | 0.136 | | -26.949 |
| 1_9_10 | -2.649 | -0.082 | -0.393 | | -26.847 |
| 1_9_11 | -2.673 | -0.243 | -0.239 | | -26.870 |
| 1_10_11 | -2.691 | -0.408 | -0.013 | | -26.788 |
| 2_3_4 | -1.186 | -1.285 | 0.178 | | -28.291 |
| 2_3_5 | -0.468 | -1.559 | 1.081 | | -17.029 |
| 2_3_6 | -0.774 | -1.188 | 1.158 | | -14.491 |
| 2_3_7 | -1.324 | -2.109 | 1.173 | | -31.837 |
| 2_3_8 | -1.101 | -0.914 | -0.437 | | -28.093 |
| 2_3_9 | -0.908 | -1.233 | -0.459 | | -30.079 |
| 2_3_10 | -1.148 | -1.316 | 0.119 | | -29.504 |
| 2_3_11 | -1.167 | -1.023 | -0.177 | | -28.656 |
| 2_4_5 | -0.573 | -1.391 | 1.021 | | -18.918 |
| 2_4_6 | -0.812 | -1.109 | 1.193 | | -15.418 |
| 2_4_7 | -1.286 | -1.631 | 0.626 | | -32.968 |
| 2_4_8 | -1.210 | -0.652 | -0.532 | | -28.051 |
| 2_4_9 | -0.971 | -1.130 | -0.478 | | -31.516 |
| 2_4_10 | -1.261 | -0.961 | -0.037 | | -29.557 |
| 2_4_11 | -1.290 | -0.692 | -0.355 | | -28.773 |
| 2_5_6 | -1.208 | 0.805 | 0.638 | | -4.970 |
| 2_5_7 | -0.984 | 1.026 | -0.549 | | -10.632 |
| 2_5_8 | -1.062 | 0.976 | -0.654 | | -11.562 |
| 2_5_9 | -1.267 | 0.924 | -0.182 | | -11.676 |
| 2_5_10 | -1.153 | 0.955 | -0.298 | | -11.018 |
| 2_5_11 | -1.124 | 0.990 | -0.550 | | -12.098 |
| 2_6_7 | -1.368 | 1.158 | -0.084 | | -9.015 |
| 2_6_8 | -1.213 | 1.074 | -0.534 | | -10.525 |
| 2_6_9 | -1.344 | 1.104 | -0.198 | | -10.001 |
| 2_6_10 | -1.305 | 1.128 | -0.196 | | -9.358 |
| 2_6_11 | -1.289 | 1.099 | -0.389 | | -10.706 |
| 2_7_8 | -1.658 | 0.265 | -0.661 | | -23.810 |
| 2_7_9 | -1.621 | 0.008 | -0.407 | | -24.404 |
| 2_7_10 | -1.767 | 0.392 | -0.424 | | -23.254 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 2_7_11 | -1.854 | 0.522 | -0.648 | | -24.633 |
| 2_8_9 | -1.451 | -0.562 | -0.210 | | -24.448 |
| 2_8_10 | -1.486 | -0.579 | -0.087 | | -24.066 |
| 2_8_11 | -1.499 | -0.504 | -0.194 | | -24.482 |
| 2_9_10 | -1.537 | -0.326 | -0.184 | | -24.288 |
| 2_9_11 | -1.510 | -0.287 | -0.410 | | -25.193 |
| 2_10_11 | -1.613 | -0.024 | -0.448 | | -24.735 |
| 3_4_5 | -1.643 | -0.452 | 1.168 | | -17.288 |
| 3_4_6 | -1.245 | -0.837 | 1.385 | | -14.232 |
| 3_4_7 | -2.066 | -1.835 | 1.201 | | -38.114 |
| 3_4_8 | -1.200 | -1.068 | -0.471 | | -31.445 |
| 3_4_9 | -1.339 | -0.908 | -0.743 | | -33.752 |
| 3_4_10 | -1.584 | -1.394 | 0.261 | | -34.928 |
| 3_4_11 | -1.389 | -1.198 | -0.080 | | -32.845 |
| 3_5_6 | -1.854 | 1.025 | 0.642 | | -9.427 |
| 3_5_7 | -2.372 | 1.177 | 0.398 | | -16.309 |
| 3_5_8 | -1.689 | 1.184 | -0.445 | | -14.708 |
| 3_5_9 | -1.879 | 1.119 | -0.337 | | -17.109 |
| 3_5_10 | -2.218 | 1.202 | 0.210 | | -16.058 |
| 3_5_11 | -1.877 | 1.195 | -0.137 | | -14.907 |
| 3_6_7 | -2.643 | 1.473 | 0.814 | | -11.706 |
| 3_6_8 | -1.639 | 1.371 | -0.371 | | -10.379 |
| 3_6_9 | -1.756 | 1.241 | -0.457 | | -14.017 |
| 3_6_10 | -2.123 | 1.467 | 0.225 | | -10.896 |
| 3_6_11 | -1.899 | 1.435 | 0.017 | | -10.284 |
| 3_7_8 | -2.750 | 0.826 | -0.564 | | -28.896 |
| 3_7_9 | -3.180 | 1.337 | -1.041 | | -34.034 |
| 3_7_10 | -3.003 | 0.766 | -0.109 | | -29.532 |
| 3_7_11 | -2.984 | 0.784 | -0.193 | | -29.702 |
| 3_8_9 | -1.859 | -0.299 | -0.693 | | -29.428 |
| 3_8_10 | -2.201 | -0.535 | 0.182 | | -27.963 |
| 3_8_11 | -2.133 | -0.592 | 0.215 | | -27.420 |
| 3_9_10 | -2.496 | -0.995 | 0.501 | | -33.130 |
| 3_9_11 | -2.124 | -0.822 | 0.126 | | -30.624 |
| 3_10_11 | -2.463 | 0.125 | -0.105 | | -28.739 |
| 4_5_6 | -1.889 | 0.940 | 0.729 | | -12.523 |
| 4_5_7 | -2.029 | 1.127 | -0.024 | | -19.360 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 4_5_8 | -1.656 | 1.127 | -0.552 | | -17.907 |
| 4_5_9 | -1.913 | 1.051 | -0.376 | | -21.144 |
| 4_5_10 | -2.192 | 1.126 | 0.108 | | -20.408 |
| 4_5_11 | -1.769 | 1.145 | -0.371 | | -18.422 |
| 4_6_7 | -2.684 | 1.461 | 0.614 | | -18.058 |
| 4_6_8 | -1.737 | 1.358 | -0.440 | | -14.700 |
| 4_6_9 | -1.891 | 1.255 | -0.447 | | -18.237 |
| 4_6_10 | -2.314 | 1.465 | 0.220 | | -16.658 |
| 4_6_11 | -1.909 | 1.414 | -0.189 | | -15.002 |
| 4_7_8 | -2.918 | 0.755 | -0.649 | | -36.434 |
| 4_7_9 | -3.239 | 1.060 | -0.965 | | -41.385 |
| 4_7_10 | -3.185 | 0.536 | -0.033 | | -37.814 |
| 4_7_11 | -3.268 | 0.979 | -0.524 | | -38.754 |
| 4_8_9 | -1.986 | -0.389 | -0.648 | | -34.148 |
| 4_8_10 | -2.448 | -0.616 | 0.231 | | -34.659 |
| 4_8_11 | -2.181 | -0.572 | -0.016 | | -32.883 |
| 4_9_10 | -2.750 | -0.970 | 0.495 | | -40.087 |
| 4_9_11 | -2.144 | -0.753 | -0.126 | | -35.436 |
| 4_10_11 | -2.723 | 0.313 | -0.423 | | -36.718 |
| 5_6_7 | 1.101 | 0.746 | -1.195 | | 3.291 |
| 5_6_8 | 1.059 | 0.874 | -0.916 | | 8.413 |
| 5_6_9 | 0.985 | 1.006 | -0.625 | | 10.130 |
| 5_6_10 | 1.032 | 0.919 | -0.665 | | 9.151 |
| 5_6_11 | 1.102 | 0.921 | -0.803 | | 9.108 |
| 5_7_8 | 1.266 | -0.964 | -0.650 | | -4.112 |
| 5_7_9 | 1.252 | -1.242 | -0.257 | | -4.341 |
| 5_7_10 | 1.304 | -1.307 | -0.048 | | -2.905 |
| 5_7_11 | 1.308 | -1.069 | -0.374 | | -3.244 |
| 5_8_9 | 1.226 | -0.919 | -0.362 | | -0.629 |
| 5_8_10 | 1.243 | -0.805 | -0.459 | | -0.710 |
| 5_8_11 | 1.305 | -0.757 | -0.476 | | 0.269 |
| 5_9_10 | 1.212 | -0.394 | -0.656 | | -0.336 |
| 5_9_11 | 1.257 | -0.475 | -0.794 | | -1.232 |
| 5_10_11 | 1.306 | -0.442 | -0.622 | | 0.517 |
| 6_7_8 | 1.589 | -0.793 | -0.650 | | 1.884 |
| 6_7_9 | 1.553 | -0.988 | -0.482 | | 0.370 |
| 6_7_10 | 1.713 | -0.970 | -0.207 | | 3.976 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 6_7_11 | 1.714 | -0.950 | -0.309 | | 3.539 |
| 6_8_9 | 1.597 | -0.802 | -0.502 | | 3.867 |
| 6_8_10 | 1.637 | -0.739 | -0.446 | | 4.901 |
| 6_8_11 | 1.760 | -0.765 | -0.333 | | 6.959 |
| 6_9_10 | 1.642 | -0.528 | -0.569 | | 4.633 |
| 6_9_11 | 1.655 | -0.642 | -0.622 | | 3.857 |
| 6_10_11 | 1.793 | -0.500 | -0.447 | | 7.272 |
| 7_8_9 | -0.930 | -0.698 | -0.730 | | -18.712 |
| 7_8_10 | -0.957 | -0.853 | -0.265 | | -16.357 |
| 7_8_11 | -1.227 | -0.888 | 0.000 | | -16.734 |
| 7_9_10 | -1.174 | -0.934 | -0.144 | | -19.013 |
| 7_9_11 | -1.069 | -0.930 | -0.326 | | -19.335 |
| 7_10_11 | -1.249 | -0.332 | -0.364 | | -16.543 |
| 8_9_10 | -0.826 | -0.767 | -0.504 | | -15.937 |
| 8_9_11 | -0.808 | -0.979 | -0.383 | | -16.201 |
| 8_10_11 | -0.987 | -0.735 | -0.082 | | -13.433 |
| 9_10_11 | -1.025 | -0.504 | -0.532 | | -16.529 |
| 1_2_3_4 | -1.860 | -0.531 | -0.890 | -0.190 | -36.018 |
| 1_2_3_5 | -1.031 | -0.309 | -1.389 | 0.859 | -23.378 |
| 1_2_3_6 | -1.567 | -0.421 | -1.033 | 0.808 | -24.499 |
| 1_2_3_7 | -1.951 | -0.776 | -2.056 | 1.348 | -39.378 |
| 1_2_3_8 | -1.777 | -0.578 | -0.815 | -0.300 | -34.530 |
| 1_2_3_9 | -1.843 | -0.578 | -0.994 | -0.011 | -35.269 |
| 1_2_3_10 | -1.912 | -0.575 | -1.280 | 0.276 | -36.788 |
| 1_2_3_11 | -1.951 | -0.523 | -1.169 | 0.197 | -35.953 |
| 1_2_4_5 | -1.262 | -0.303 | -1.307 | 0.760 | -26.976 |
| 1_2_4_6 | -1.676 | -0.352 | -1.086 | 0.814 | -26.726 |
| 1_2_4_7 | -2.118 | -0.540 | -2.016 | 1.016 | -43.201 |
| 1_2_4_8 | -1.849 | -0.553 | -0.774 | -0.354 | -35.854 |
| 1_2_4_9 | -1.955 | -0.534 | -1.011 | -0.004 | -37.312 |
| 1_2_4_10 | -2.041 | -0.460 | -1.376 | 0.243 | -39.857 |
| 1_2_4_11 | -1.982 | -0.521 | -1.047 | 0.041 | -37.525 |
| 1_2_5_6 | -1.237 | -0.922 | 0.580 | 0.506 | -14.546 |
| 1_2_5_7 | -1.225 | -0.843 | 0.735 | -0.284 | -19.055 |
| 1_2_5_8 | -1.097 | -0.831 | 0.748 | -0.552 | -18.898 |
| 1_2_5_9 | -1.369 | -1.021 | 0.672 | 0.081 | -19.919 |
| 1_2_5_10 | -1.244 | -0.909 | 0.695 | -0.181 | -19.387 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 1_2_5_11 | -1.023 | -0.940 | 0.764 | -0.357 | -18.614 |
| 1_2_6_7 | -1.698 | -0.988 | 0.765 | 0.100 | -20.361 |
| 1_2_6_8 | -1.567 | -0.813 | 0.737 | -0.421 | -20.887 |
| 1_2_6_9 | -1.761 | -0.971 | 0.796 | 0.163 | -20.002 |
| 1_2_6_10 | -1.659 | -0.899 | 0.765 | -0.073 | -20.362 |
| 1_2_6_11 | -1.597 | -0.914 | 0.770 | -0.138 | -20.417 |
| 1_2_7_8 | -1.852 | -1.119 | 0.390 | -0.521 | -30.716 |
| 1_2_7_9 | -2.004 | -1.154 | 0.128 | 0.060 | -30.811 |
| 1_2_7_10 | -1.927 | -1.164 | 0.421 | -0.263 | -30.407 |
| 1_2_7_11 | -1.837 | -1.231 | 0.379 | -0.279 | -30.791 |
| 1_2_8_9 | -1.923 | -0.981 | -0.484 | 0.221 | -30.926 |
| 1_2_8_10 | -1.824 | -0.950 | -0.456 | 0.036 | -30.952 |
| 1_2_8_11 | -1.876 | -0.935 | -0.509 | 0.130 | -30.868 |
| 1_2_9_10 | -1.977 | -1.043 | 0.120 | -0.120 | -30.774 |
| 1_2_9_11 | -1.913 | -1.074 | 0.096 | -0.148 | -30.947 |
| 1_2_10_11 | -1.868 | -1.036 | -0.036 | -0.117 | -30.901 |
| 1_3_4_5 | -1.124 | -1.327 | -0.415 | 0.887 | -24.713 |
| 1_3_4_6 | -1.703 | -0.890 | -0.625 | 0.878 | -26.174 |
| 1_3_4_7 | -2.261 | -1.680 | -1.583 | 1.567 | -46.542 |
| 1_3_4_8 | -2.022 | -0.763 | -0.691 | -0.286 | -37.533 |
| 1_3_4_9 | -1.995 | -0.902 | -0.716 | -0.133 | -38.404 |
| 1_3_4_10 | -2.156 | -1.081 | -1.061 | 0.415 | -41.946 |
| 1_3_4_11 | -2.196 | -1.116 | -0.692 | 0.277 | -39.166 |
| 1_3_5_6 | -1.023 | -1.568 | 0.801 | 0.516 | -17.484 |
| 1_3_5_7 | -1.326 | -2.302 | 0.835 | 0.744 | -26.254 |
| 1_3_5_8 | -1.021 | -1.430 | 0.930 | -0.367 | -21.713 |
| 1_3_5_9 | -1.057 | -1.628 | 0.902 | -0.108 | -22.955 |
| 1_3_5_10 | -1.195 | -1.940 | 0.908 | 0.289 | -24.467 |
| 1_3_5_11 | -1.170 | -1.682 | 0.893 | 0.067 | -23.245 |
| 1_3_6_7 | -1.898 | -2.380 | 0.920 | 1.136 | -26.783 |
| 1_3_6_8 | -1.683 | -1.201 | 0.876 | -0.263 | -23.148 |
| 1_3_6_9 | -1.719 | -1.353 | 0.896 | -0.025 | -23.523 |
| 1_3_6_10 | -1.794 | -1.699 | 0.937 | 0.338 | -24.857 |
| 1_3_6_11 | -1.845 | -1.541 | 0.892 | 0.280 | -24.521 |
| 1_3_7_8 | -2.223 | -2.308 | 1.201 | -0.378 | -38.354 |
| 1_3_7_9 | -2.030 | -2.605 | 1.329 | -0.418 | -39.877 |
| 1_3_7_10 | -2.301 | -2.453 | 1.093 | 0.016 | -39.011 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 1_3_7_11 | -2.340 | -2.475 | 1.037 | 0.134 | -39.203 |
| 1_3_8_9 | -2.022 | -1.273 | -0.288 | -0.067 | -34.910 |
| 1_3_8_10 | -2.129 | -1.592 | -0.350 | 0.341 | -36.601 |
| 1_3_8_11 | -2.214 | -1.498 | -0.497 | 0.522 | -36.111 |
| 1_3_9_10 | -1.981 | -1.834 | -0.342 | 0.408 | -38.006 |
| 1_3_9_11 | -2.111 | -1.643 | -0.207 | 0.322 | -36.767 |
| 1_3_10_11 | -2.269 | -1.811 | 0.205 | 0.224 | -37.468 |
| 1_4_5_6 | -1.231 | -1.545 | 0.683 | 0.556 | -21.803 |
| 1_4_5_7 | -1.526 | -2.131 | 0.712 | 0.510 | -32.071 |
| 1_4_5_8 | -1.196 | -1.369 | 0.836 | -0.436 | -25.661 |
| 1_4_5_9 | -1.304 | -1.614 | 0.789 | -0.081 | -27.889 |
| 1_4_5_10 | -1.427 | -1.914 | 0.776 | 0.242 | -30.332 |
| 1_4_5_11 | -1.269 | -1.561 | 0.820 | -0.124 | -26.966 |
| 1_4_6_7 | -1.966 | -2.451 | 0.875 | 1.004 | -33.407 |
| 1_4_6_8 | -1.724 | -1.264 | 0.851 | -0.307 | -26.645 |
| 1_4_6_9 | -1.804 | -1.448 | 0.881 | 0.010 | -27.387 |
| 1_4_6_10 | -1.862 | -1.861 | 0.905 | 0.352 | -30.278 |
| 1_4_6_11 | -1.848 | -1.504 | 0.876 | 0.103 | -27.844 |
| 1_4_7_8 | -2.251 | -2.462 | 1.164 | -0.442 | -44.854 |
| 1_4_7_9 | -2.153 | -2.667 | 1.150 | -0.312 | -46.377 |
| 1_4_7_10 | -2.358 | -2.618 | 0.945 | 0.089 | -46.056 |
| 1_4_7_11 | -2.301 | -2.617 | 1.128 | -0.141 | -45.775 |
| 1_4_8_9 | -2.082 | -1.362 | -0.341 | -0.012 | -38.437 |
| 1_4_8_10 | -2.156 | -1.787 | -0.402 | 0.390 | -41.751 |
| 1_4_8_11 | -2.213 | -1.464 | -0.490 | 0.347 | -39.172 |
| 1_4_9_10 | -2.071 | -2.015 | -0.290 | 0.414 | -43.459 |
| 1_4_9_11 | -2.152 | -1.622 | -0.139 | 0.129 | -40.228 |
| 1_4_10_11 | -2.249 | -1.942 | 0.319 | -0.014 | -42.610 |
| 1_5_6_7 | -1.324 | 0.777 | 0.606 | -0.810 | -8.166 |
| 1_5_6_8 | -1.520 | 0.704 | 0.625 | -0.685 | -7.689 |
| 1_5_6_9 | -1.861 | 0.601 | 0.761 | -0.133 | -6.679 |
| 1_5_6_10 | -1.625 | 0.656 | 0.672 | -0.403 | -7.320 |
| 1_5_6_11 | -1.529 | 0.720 | 0.714 | -0.442 | -5.699 |
| 1_5_7_8 | -1.284 | 0.923 | -0.614 | -0.568 | -13.948 |
| 1_5_7_9 | -1.437 | 0.912 | -0.903 | -0.001 | -14.081 |
| 1_5_7_10 | -1.437 | 0.907 | -0.858 | -0.043 | -14.069 |
| 1_5_7_11 | -1.354 | 0.935 | -0.797 | -0.173 | -13.581 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 1_5_8_9 | -1.701 | 0.833 | -0.750 | 0.040 | -13.674 |
| 1_5_8_10 | -1.520 | 0.843 | -0.642 | -0.254 | -13.768 |
| 1_5_8_11 | -1.602 | 0.853 | -0.689 | -0.111 | -13.447 |
| 1_5_9_10 | -1.783 | 0.794 | -0.003 | -0.465 | -13.876 |
| 1_5_9_11 | -1.609 | 0.865 | -0.152 | -0.477 | -12.813 |
| 1_5_10_11 | -1.616 | 0.850 | -0.338 | -0.280 | -13.098 |
| 1_6_7_8 | -2.000 | 0.948 | -0.291 | -0.501 | -15.309 |
| 1_6_7_9 | -2.121 | 1.011 | -0.553 | 0.027 | -14.564 |
| 1_6_7_10 | -2.098 | 0.994 | -0.428 | -0.116 | -14.599 |
| 1_6_7_11 | -2.103 | 1.006 | -0.537 | -0.013 | -14.627 |
| 1_6_8_9 | -2.191 | 0.992 | -0.609 | 0.108 | -13.906 |
| 1_6_8_10 | -2.052 | 0.945 | -0.526 | -0.157 | -14.730 |
| 1_6_8_11 | -2.183 | 0.966 | -0.642 | 0.112 | -14.289 |
| 1_6_9_10 | -2.237 | 1.010 | 0.060 | -0.345 | -13.354 |
| 1_6_9_11 | -2.221 | 1.050 | -0.070 | -0.235 | -12.816 |
| 1_6_10_11 | -2.186 | 1.003 | -0.306 | -0.052 | -13.525 |
| 1-7-8-9 | -2.452 | -0.355 | -0.557 | 0.021 | -27.871 |
| 1_7_8_10 | -2.429 | -0.253 | -0.543 | -0.101 | -27.704 |
| 1_7_8_11 | -2.510 | -0.500 | -0.663 | 0.316 | -28.116 |
| 1_7_9_10 | -2.529 | -0.448 | -0.080 | -0.168 | -27.842 |
| 1_7_9_11 | -2.528 | -0.616 | -0.125 | 0.012 | -28.172 |
| 1_7_10_11 | -2.593 | -0.472 | -0.206 | 0.063 | -27.801 |
| 1_8_9_10 | -2.515 | -0.580 | 0.055 | -0.230 | -27.112 |
| 1_8_9_11 | -2.649 | -0.718 | -0.039 | 0.136 | -26.982 |
| 1_8_10_11 | -2.566 | -0.684 | -0.330 | 0.319 | -27.089 |
| 1_9_10_11 | -2.642 | -0.083 | -0.385 | -0.017 | -26.856 |
| 2_3_4_5 | -0.476 | -1.574 | 0.029 | 1.080 | -16.921 |
| 2_3_4_6 | -0.770 | -1.179 | -0.017 | 1.158 | -14.549 |
| 2_3_4_7 | -1.224 | -1.934 | -0.473 | 1.273 | -33.868 |
| 2_3_4_8 | -1.164 | -1.050 | 0.260 | -0.442 | -27.102 |
| 2_3_4_9 | -0.919 | -1.254 | 0.039 | -0.457 | -29.926 |
| 2_3_4_10 | -1.163 | -1.342 | 0.063 | 0.111 | -29.231 |
| 2_3_4_11 | -1.213 | -1.122 | 0.189 | -0.178 | -27.942 |
| 2_3_5_6 | -0.376 | -1.526 | 0.957 | 0.567 | -11.578 |
| 2_3_5_7 | -0.615 | -2.079 | 1.026 | 0.677 | -19.501 |
| 2_3_5_8 | -0.437 | -1.301 | 1.083 | -0.428 | -16.446 |
| 2_3_5_9 | -0.369 | -1.575 | 1.056 | -0.232 | -17.955 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 2_3_5_10 | -0.471 | -1.791 | 1.093 | 0.213 | -17.916 |
| 2_3_5_11 | -0.494 | -1.390 | 1.082 | -0.180 | -16.748 |
| 2_3_6_7 | -0.963 | -2.104 | 1.152 | 1.158 | -17.609 |
| 2_3_6_8 | -0.761 | -0.968 | 1.105 | -0.354 | -14.536 |
| 2_3_6_9 | -0.667 | -1.214 | 1.089 | -0.256 | -16.011 |
| 2_3_6_10 | -0.774 | -1.424 | 1.191 | 0.222 | -15.111 |
| 2_3_6_11 | -0.789 | -1.114 | 1.146 | -0.078 | -14.504 |
| 2_3_7_8 | -1.299 | -1.892 | 1.310 | -0.519 | -31.237 |
| 2_3_7_9 | -1.022 | -2.447 | 1.513 | -0.702 | -34.511 |
| 2_3_7_10 | -1.345 | -2.027 | 1.369 | -0.222 | -31.301 |
| 2_3_7_11 | -1.435 | -1.918 | 1.480 | -0.457 | -31.847 |
| 2_3_8_9 | -0.942 | -1.001 | -0.349 | -0.328 | -29.077 |
| 2_3_8_10 | -1.106 | -1.109 | -0.468 | 0.202 | -28.964 |
| 2_3_8_11 | -1.094 | -0.942 | -0.453 | 0.041 | -28.130 |
| 2_3_9_10 | -0.832 | -1.650 | -0.638 | 0.377 | -32.258 |
| 2_3_9_11 | -0.925 | -1.181 | -0.441 | -0.053 | -29.945 |
| 2_3_10_11 | -1.187 | -1.175 | 0.218 | -0.259 | -29.509 |
| 2_4_5_6 | -0.439 | -1.400 | 0.881 | 0.646 | -12.879 |
| 2_4_5_7 | -0.584 | -1.476 | 1.012 | 0.089 | -19.462 |
| 2_4_5_8 | -0.541 | -1.040 | 1.030 | -0.539 | -17.410 |
| 2_4_5_9 | -0.439 | -1.446 | 0.993 | -0.264 | -20.208 |
| 2_4_5_10 | -0.563 | -1.462 | 1.023 | 0.047 | -19.323 |
| 2_4_5_11 | -0.613 | -1.042 | 1.035 | -0.396 | -17.734 |
| 2_4_6_7 | -0.844 | -1.778 | 1.210 | 0.678 | -18.632 |
| 2_4_6_8 | -0.810 | -0.808 | 1.117 | -0.438 | -14.978 |
| 2_4_6_9 | -0.680 | -1.169 | 1.122 | -0.271 | -17.243 |
| 2_4_6_10 | -0.782 | -1.286 | 1.216 | 0.116 | -16.195 |
| 2_4_6_11 | -0.856 | -0.878 | 1.149 | -0.251 | -15.217 |
| 2_4_7_8 | -1.250 | -1.441 | 0.858 | -0.613 | -32.049 |
| 2_4_7_9 | -0.914 | -2.129 | 0.973 | -0.663 | -36.973 |
| 2_4_7_10 | -1.342 | -1.449 | 0.821 | -0.249 | -31.667 |
| 2_4_7_11 | -1.385 | -1.621 | 1.214 | -0.644 | -33.891 |
| 2_4_8_9 | -1.040 | -0.778 | -0.452 | -0.298 | -29.359 |
| 2_4_8_10 | -1.192 | -0.774 | -0.547 | 0.088 | -28.798 |
| 2_4_8_11 | -1.226 | -0.581 | -0.477 | -0.119 | -27.872 |
| 2_4_9_10 | -0.841 | -1.553 | -0.616 | 0.260 | -34.358 |
| 2_4_9_11 | -1.051 | -0.873 | -0.387 | -0.257 | -30.406 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 2_4_10_11 | -1.264 | -0.894 | 0.178 | -0.426 | -30.164 |
| 2_5_6_7 | -0.901 | 0.889 | 0.590 | -0.491 | -5.088 |
| 2_5_6_8 | -0.974 | 0.862 | 0.519 | -0.616 | -6.639 |
| 2_5_6_9 | -1.174 | 0.798 | 0.615 | -0.099 | -5.544 |
| 2_5_6_10 | -1.058 | 0.825 | 0.588 | -0.259 | -5.455 |
| 2_5_6_11 | -1.035 | 0.872 | 0.528 | -0.507 | -7.030 |
| 2_5_7_8 | -0.933 | 1.008 | -0.230 | -0.609 | -11.371 |
| 2_5_7_9 | -0.963 | 1.011 | -0.523 | -0.097 | -11.006 |
| 2_5_7_10 | -1.003 | 1.006 | -0.376 | -0.149 | -10.756 |
| 2_5_7_11 | -1.101 | 0.995 | -0.045 | -0.534 | -12.037 |
| 2_5_8_9 | -1.079 | 0.983 | -0.666 | 0.056 | -11.365 |
| 2_5_8_10 | -1.008 | 0.979 | -0.617 | -0.112 | -11.538 |
| 2_5_8_11 | -1.015 | 0.993 | -0.502 | -0.288 | -12.016 |
| 2_5_9_10 | -1.140 | 0.948 | -0.055 | -0.284 | -11.221 |
| 2_5_9_11 | -1.120 | 0.988 | -0.013 | -0.547 | -12.141 |
| 2_5_10_11 | -1.122 | 0.990 | -0.006 | -0.546 | -12.091 |
| 2_6_7_8 | -1.321 | 1.066 | 0.212 | -0.577 | -10.434 |
| 2_6_7_9 | -1.320 | 1.106 | -0.045 | -0.192 | -10.015 |
| 2_6_7_10 | -1.389 | 1.111 | 0.237 | -0.292 | -9.321 |
| 2_6_7_11 | -1.481 | 1.074 | 0.410 | -0.537 | -10.956 |
| 2_6_8_9 | -1.206 | 1.069 | -0.529 | -0.022 | -10.634 |
| 2_6_8_10 | -1.195 | 1.070 | -0.521 | -0.040 | -10.574 |
| 2_6_8_11 | -1.194 | 1.064 | -0.453 | -0.152 | -10.984 |
| 2_6_9_10 | -1.275 | 1.098 | -0.128 | -0.166 | -9.986 |
| 2_6_9_11 | -1.259 | 1.076 | -0.096 | -0.373 | -11.156 |
| 2_6_10_11 | -1.296 | 1.100 | 0.018 | -0.399 | -10.713 |
| 2_7_8_9 | -1.587 | 0.288 | -0.616 | -0.227 | -24.197 |
| 2_7_8_10 | -1.669 | 0.583 | -0.623 | -0.307 | -23.487 |
| 2_7_8_11 | -1.738 | 0.541 | -0.512 | -0.383 | -24.357 |
| 2_7_9_10 | -1.659 | 0.350 | -0.308 | -0.329 | -23.876 |
| 2_7_9_11 | -1.744 | 0.537 | -0.299 | -0.599 | -25.079 |
| 2_7_10_11 | -1.848 | 0.729 | -0.244 | -0.581 | -24.278 |
| 2_8_9_10 | -1.434 | -0.550 | -0.194 | -0.047 | -24.417 |
| 2_8_9_11 | -1.432 | -0.472 | -0.190 | -0.177 | -24.789 |
| 2_8_10_11 | -1.495 | -0.504 | -0.008 | -0.190 | -24.473 |
| 2_9_10_11 | -1.522 | -0.299 | 0.043 | -0.432 | -25.263 |
| 3_4_5_6 | -1.543 | -0.411 | 1.007 | 0.634 | -11.403 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 3_4_5_7 | -1.981 | -0.840 | 1.128 | 0.656 | -20.919 |
| 3_4_5_8 | -1.423 | -0.358 | 1.166 | -0.435 | -16.377 |
| 3_4_5_9 | -1.594 | -0.380 | 1.104 | -0.323 | -18.791 |
| 3_4_5_10 | -1.790 | -0.683 | 1.174 | 0.294 | -19.564 |
| 3_4_5_11 | -1.517 | -0.466 | 1.173 | -0.144 | -17.048 |
| 3_4_6_7 | -1.891 | -1.519 | 1.409 | 1.252 | -19.693 |
| 3_4_6_8 | -1.066 | -0.767 | 1.330 | -0.350 | -14.007 |
| 3_4_6_9 | -1.210 | -0.726 | 1.213 | -0.425 | -17.194 |
| 3_4_6_10 | -1.415 | -1.115 | 1.422 | 0.362 | -16.561 |
| 3_4_6_11 | -1.244 | -0.837 | 1.385 | 0.000 | -14.232 |
| 3_4_7_8 | -1.838 | -1.770 | 1.330 | -0.539 | -37.044 |
| 3_4_7_9 | -2.253 | -1.815 | 1.864 | -1.040 | -42.556 |
| 3_4_7_10 | -2.068 | -1.842 | 1.189 | 0.016 | -38.185 |
| 3_4_7_11 | -1.885 | -2.005 | 1.445 | -0.341 | -38.693 |
| 3_4_8_9 | -1.200 | -0.872 | -0.283 | -0.648 | -32.784 |
| 3_4_8_10 | -1.340 | -1.329 | -0.514 | 0.346 | -33.935 |
| 3_4_8_11 | -1.317 | -1.032 | -0.540 | 0.173 | -31.474 |
| 3_4_9_10 | -1.622 | -1.344 | -0.986 | 0.668 | -39.202 |
| 3_4_9_11 | -1.423 | -0.889 | -0.765 | 0.097 | -33.917 |
| 3_4_10_11 | -1.434 | -1.491 | 0.355 | -0.217 | -35.324 |
| 3_5_6_7 | -2.345 | 0.995 | 0.695 | 0.519 | -10.406 |
| 3_5_6_8 | -1.599 | 1.038 | 0.574 | -0.403 | -9.609 |
| 3_5_6_9 | -1.792 | 0.993 | 0.568 | -0.243 | -11.475 |
| 3_5_6_10 | -2.131 | 1.031 | 0.678 | 0.258 | -10.166 |
| 3_5_6_11 | -1.781 | 1.032 | 0.629 | -0.094 | -9.343 |
| 3_5_7_8 | -2.187 | 1.167 | 0.544 | -0.486 | -16.189 |
| 3_5_7_9 | -2.559 | 1.060 | 0.761 | -0.510 | -20.218 |
| 3_5_7_10 | -2.409 | 1.189 | 0.279 | 0.138 | -16.543 |
| 3_5_7_11 | -2.315 | 1.182 | 0.528 | -0.227 | -16.193 |
| 3_5_8_9 | -1.662 | 1.143 | -0.389 | -0.201 | -15.912 |
| 3_5_8_10 | -1.988 | 1.202 | -0.492 | 0.300 | -15.894 |
| 3_5_8_11 | -1.735 | 1.180 | -0.479 | 0.085 | -14.851 |
| 3_5_9_10 | -2.281 | 1.108 | -0.515 | 0.417 | -19.832 |
| 3_5_9_11 | -1.835 | 1.125 | -0.322 | -0.061 | -16.892 |
| 3_5_10_11 | -2.121 | 1.218 | 0.307 | -0.252 | -15.920 |
| 3_6_7_8 | -2.465 | 1.406 | 0.930 | -0.434 | -12.053 |
| 3_6_7_9 | -2.851 | 1.201 | 1.246 | -0.705 | -18.278 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 3_6_7_10 | -2.650 | 1.475 | 0.794 | 0.025 | -11.739 |
| 3_6_7_11 | -2.610 | 1.466 | 0.890 | -0.133 | -11.728 |
| 3_6_8_9 | -1.599 | 1.233 | -0.276 | -0.367 | -13.359 |
| 3_6_8_10 | -1.923 | 1.407 | -0.413 | 0.295 | -11.216 |
| 3_6_8_11 | -1.757 | 1.374 | -0.460 | 0.227 | -10.599 |
| 3_6_9_10 | -2.207 | 1.230 | -0.654 | 0.477 | -16.972 |
| 3_6_9_11 | -1.839 | 1.240 | -0.483 | 0.115 | -14.325 |
| 3_6_10_11 | -2.094 | 1.465 | 0.253 | -0.075 | -10.911 |
| 3_7_8_9 | -3.012 | 1.359 | -0.321 | -0.937 | -33.177 |
| 3_7_8_10 | -2.743 | 0.849 | -0.561 | -0.029 | -28.823 |
| 3_7_8_11 | -2.758 | 0.787 | -0.593 | 0.082 | -28.899 |
| 3_7_9_10 | -3.271 | 1.169 | -1.113 | 0.255 | -35.014 |
| 3_7_9_11 | -3.163 | 1.366 | -1.034 | -0.060 | -33.970 |
| 3_7_10_11 | -2.970 | 0.824 | -0.057 | -0.178 | -29.558 |
| 3_8_9_10 | -2.324 | -0.317 | -0.893 | 0.516 | -32.285 |
| 3_8_9_11 | -2.002 | -0.405 | -0.720 | 0.290 | -29.822 |
| 3_8_10_11 | -2.235 | -0.593 | 0.129 | 0.168 | -27.910 |
| 3_9_10_11 | -2.486 | -0.993 | 0.510 | -0.025 | -33.118 |
| 4_5_6_7 | -2.030 | 0.926 | 0.745 | 0.133 | -13.195 |
| 4_5_6_8 | -1.554 | 0.969 | 0.630 | -0.499 | -12.055 |
| 4_5_6_9 | -1.808 | 0.909 | 0.647 | -0.266 | -14.475 |
| 4_5_6_10 | -2.099 | 0.935 | 0.755 | 0.170 | -13.703 |
| 4_5_6_11 | -1.662 | 0.973 | 0.668 | -0.314 | -12.199 |
| 4_5_7_8 | -1.871 | 1.112 | 0.210 | -0.572 | -19.153 |
| 4_5_7_9 | -2.199 | 1.018 | 0.284 | -0.439 | -23.182 |
| 4_5_7_10 | -2.086 | 1.137 | -0.136 | 0.141 | -19.847 |
| 4_5_7_11 | -2.134 | 1.121 | 0.399 | -0.464 | -20.631 |
| 4_5_8_9 | -1.622 | 1.089 | -0.497 | -0.196 | -18.923 |
| 4_5_8_10 | -1.928 | 1.130 | -0.591 | 0.236 | -19.774 |
| 4_5_8_11 | -1.598 | 1.135 | -0.490 | -0.135 | -17.697 |
| 4_5_9_10 | -2.263 | 1.028 | -0.506 | 0.315 | -24.350 |
| 4_5_9_11 | -1.712 | 1.085 | -0.288 | -0.304 | -19.875 |
| 4_5_10_11 | -2.126 | 1.157 | 0.374 | -0.525 | -21.107 |
| 4_6_7_8 | -2.526 | 1.376 | 0.807 | -0.516 | -18.436 |
| 4_6_7_9 | -2.828 | 1.219 | 0.957 | -0.623 | -23.973 |
| 4_6_7_10 | -2.720 | 1.469 | 0.550 | 0.085 | -18.329 |
| 4_6_7_11 | -2.771 | 1.422 | 0.990 | -0.421 | -19.463 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 4_6_8_9 | -1.683 | 1.239 | -0.358 | -0.328 | -17.044 |
| 4_6_8_10 | -2.093 | 1.388 | -0.489 | 0.319 | -16.810 |
| 4_6_8_11 | -1.748 | 1.359 | -0.452 | 0.026 | -14.711 |
| 4_6_9_10 | -2.389 | 1.238 | -0.625 | 0.458 | -22.562 |
| 4_6_9_11 | -1.823 | 1.252 | -0.420 | -0.108 | -17.959 |
| 4_6_10_11 | -2.275 | 1.442 | 0.394 | -0.347 | -17.523 |
| 4_7_8_9 | -3.054 | 1.150 | -0.446 | -0.825 | -40.011 |
| 4_7_8_10 | -2.946 | 0.709 | -0.655 | 0.064 | -36.704 |
| 4_7_8_11 | -2.993 | 0.936 | -0.554 | -0.243 | -37.051 |
| 4_7_9_10 | -3.389 | 0.870 | -1.050 | 0.313 | -43.101 |
| 4_7_9_11 | -3.302 | 1.408 | -0.918 | -0.418 | -41.989 |
| 4_7_10_11 | -3.351 | 0.895 | 0.166 | -0.572 | -39.567 |
| 4_8_9_10 | -2.531 | -0.421 | -0.835 | 0.531 | -38.624 |
| 4_8_9_11 | -2.007 | -0.413 | -0.654 | 0.054 | -34.212 |
| 4_8_10_11 | -2.456 | -0.562 | 0.289 | -0.137 | -34.965 |
| 4_9_10_11 | -2.699 | -0.942 | 0.661 | -0.351 | -40.474 |
| 5_6_7_8 | 1.091 | 0.649 | -0.852 | -0.607 | 1.357 |
| 5_6_7_9 | 1.077 | 0.711 | -1.133 | -0.155 | 2.133 |
| 5_6_7_10 | 1.099 | 0.745 | -1.173 | -0.021 | 3.285 |
| 5_6_7_11 | 1.109 | 0.716 | -0.941 | -0.339 | 2.739 |
| 5_6_8_9 | 1.027 | 0.812 | -0.851 | -0.222 | 6.332 |
| 5_6_8_10 | 1.041 | 0.758 | -0.738 | -0.382 | 5.302 |
| 5_6_8_11 | 1.079 | 0.804 | -0.687 | -0.403 | 6.425 |
| 5_6_9_10 | 0.999 | 0.857 | -0.255 | -0.582 | 7.057 |
| 5_6_9_11 | 1.050 | 0.823 | -0.331 | -0.717 | 5.933 |
| 5_6_10_11 | 1.071 | 0.842 | -0.359 | -0.557 | 7.094 |
| 5_7_8_9 | 1.251 | -0.940 | -0.636 | -0.071 | -4.484 |
| 5_7_8_10 | 1.271 | -1.023 | -0.657 | 0.059 | -4.135 |
| 5_7_8_11 | 1.268 | -0.923 | -0.622 | -0.076 | -4.128 |
| 5_7_9_10 | 1.253 | -1.257 | -0.261 | 0.016 | -4.365 |
| 5_7_9_11 | 1.260 | -0.990 | -0.214 | -0.351 | -4.414 |
| 5_7_10_11 | 1.316 | -1.134 | 0.076 | -0.394 | -3.275 |
| 5_8_9_10 | 1.215 | -0.776 | -0.152 | -0.417 | -1.569 |
| 5_8_9_11 | 1.242 | -0.688 | -0.284 | -0.432 | -1.643 |
| 5_8_10_11 | 1.256 | -0.708 | -0.358 | -0.250 | -1.025 |
| 5_9_10_11 | 1.239 | -0.336 | -0.337 | -0.595 | -1.547 |
| 6_7_8_9 | 1.481 | -0.701 | -0.589 | -0.316 | -0.320 |

(continued)

| SEQ ID NO: | Discriminant coefficient | | | | Constant term b |
|---|---|---|---|---|---|
| | Term 1 a1 | Term 2 a2 | Term 3 a3 | Term 4 a4 | |
| 6_7_8_10 | 1.578 | -0.694 | -0.639 | -0.101 | 1.883 |
| 6_7_8_11 | 1.589 | -0.796 | -0.651 | 0.004 | 1.886 |
| 6_7_9_10 | 1.549 | -0.907 | -0.460 | -0.086 | 0.503 |
| 6_7_9_11 | 1.539 | -0.795 | -0.454 | -0.266 | 0.141 |
| 6_7_10_11 | 1.699 | -0.847 | -0.123 | -0.276 | 3.547 |
| 6_8_9_10 | 1.531 | -0.679 | -0.331 | -0.358 | 2.498 |
| 6_8_9_11 | 1.571 | -0.655 | -0.462 | -0.275 | 2.859 |
| 6_8_10_11 | 1.637 | -0.709 | -0.415 | -0.078 | 4.767 |
| 6_9_10_11 | 1.606 | -0.501 | -0.343 | -0.421 | 3.227 |
| 7_8_9_10 | -0.865 | -0.693 | -0.713 | -0.071 | -18.566 |
| 7_8_9_11 | -0.931 | -0.699 | -0.730 | 0.001 | -18.712 |
| 7_8_10_11 | -0.978 | -0.878 | -0.283 | 0.073 | -16.302 |
| 7_9_10_11 | -1.030 | -0.918 | -0.049 | -0.314 | -19.229 |
| 8_9_10_11 | -0.779 | -0.773 | -0.454 | -0.120 | -16.158 |

Table 6

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 1_2 | 90 | 87 | 93 | 94 | 92 | 97 |
| 1_3 | 92 | 92 | 91 | 93 | 94 | 93 |
| 1_4 | 91 | 92 | 90 | 93 | 94 | 93 |
| 1_5 | 90 | 86 | 94 | 90 | 90 | 90 |
| 1_6 | 91 | 86 | 96 | 88 | 86 | 90 |
| 1_7 | 90 | 89 | 91 | 92 | 90 | 93 |
| 1_8 | 89 | 88 | 90 | 91 | 90 | 91 |
| 1_9 | 89 | 84 | 93 | 88 | 86 | 90 |
| 1_10 | 91 | 92 | 90 | 89 | 88 | 90 |
| 1_11 | 91 | 88 | 93 | 90 | 86 | 93 |
| 2_3 | 88 | 88 | 88 | 86 | 88 | 84 |
| 2_4 | 85 | 82 | 89 | 87 | 88 | 86 |
| 2_5 | 92 | 87 | 96 | 93 | 92 | 93 |
| 2_6 | 89 | 85 | 92 | 91 | 88 | 93 |
| 2_7 | 85 | 81 | 89 | 88 | 84 | 91 |
| 2_8 | 84 | 82 | 85 | 86 | 84 | 88 |
| 2_9 | 84 | 81 | 87 | 89 | 88 | 90 |
| 2_10 | 84 | 82 | 86 | 86 | 86 | 86 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 2_11 | 85 | 83 | 87 | 89 | 84 | 93 |
| 3_4 | 84 | 82 | 85 | 86 | 88 | 84 |
| 3_5 | 94 | 96 | 93 | 93 | 98 | 88 |
| 3_6 | 89 | 89 | 89 | 88 | 90 | 86 |
| 3_7 | 84 | 83 | 86 | 83 | 86 | 81 |
| 3_8 | 80 | 80 | 81 | 82 | 84 | 81 |
| 3_9 | 86 | 89 | 84 | 87 | 90 | 84 |
| 3_10 | 84 | 81 | 87 | 83 | 84 | 83 |
| 3_11 | 82 | 81 | 83 | 83 | 84 | 83 |
| 4_5 | 92 | 92 | 92 | 90 | 92 | 88 |
| 4_6 | 88 | 87 | 89 | 88 | 90 | 86 |
| 4_7 | 84 | 81 | 86 | 85 | 84 | 86 |
| 4_8 | 82 | 83 | 82 | 83 | 86 | 81 |
| 4_9 | 84 | 84 | 85 | 85 | 86 | 84 |
| 4_10 | 83 | 79 | 86 | 84 | 84 | 84 |
| 4_11 | 84 | 84 | 84 | 84 | 84 | 84 |
| 5_6 | 89 | 84 | 93 | 83 | 76 | 90 |
| 5_7 | 92 | 94 | 90 | 90 | 92 | 88 |
| 5_8 | 89 | 89 | 89 | 87 | 86 | 88 |
| 5_9 | 84 | 79 | 90 | 81 | 78 | 84 |
| 5_10 | 86 | 90 | 83 | 89 | 94 | 84 |
| 5_11 | 88 | 88 | 89 | 88 | 88 | 88 |
| 6_7 | 86 | 88 | 85 | 85 | 86 | 84 |
| 6_8 | 85 | 87 | 84 | 79 | 78 | 81 |
| 6_9 | 84 | 81 | 86 | 78 | 74 | 83 |
| 6_10 | 84 | 86 | 83 | 80 | 80 | 81 |
| 6_11 | 84 | 86 | 83 | 81 | 78 | 84 |
| 7_8 | 79 | 78 | 81 | 81 | 82 | 81 |
| 7_9 | 83 | 84 | 83 | 84 | 86 | 83 |
| 7_10 | 80 | 75 | 84 | 84 | 86 | 83 |
| 7_11 | 78 | 75 | 82 | 80 | 80 | 81 |
| 8_9 | 81 | 82 | 80 | 81 | 84 | 79 |
| 8_10 | 82 | 80 | 83 | 82 | 86 | 79 |
| 8_11 | 77 | 77 | 78 | 81 | 82 | 81 |
| 9_10 | 83 | 83 | 83 | 83 | 86 | 81 |
| 9_11 | 81 | 82 | 81 | 78 | 80 | 77 |
| 10_11 | 79 | 77 | 81 | 82 | 86 | 79 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 1_2_3 | 92 | 92 | 91 | 93 | 94 | 93 |
| 1_2_4 | 91 | 90 | 91 | 93 | 94 | 93 |
| 1_2_5 | 93 | 90 | 96 | 93 | 94 | 93 |
| 1_2_6 | 92 | 88 | 95 | 92 | 90 | 93 |
| 1_2_7 | 90 | 86 | 93 | 94 | 92 | 97 |
| 1_2_8 | 91 | 90 | 91 | 92 | 90 | 93 |
| 1_2_9 | 90 | 87 | 92 | 93 | 92 | 95 |
| 1_2_10 | 90 | 87 | 92 | 95 | 92 | 98 |
| 1_2_11 | 90 | 87 | 92 | 95 | 92 | 98 |
| 1_3_4 | 92 | 92 | 91 | 93 | 94 | 93 |
| 1_3_5 | 94 | 95 | 94 | 92 | 94 | 90 |
| 1_3_6 | 93 | 94 | 92 | 92 | 94 | 90 |
| 1_3_7 | 95 | 95 | 95 | 95 | 96 | 95 |
| 1_3_8 | 91 | 91 | 90 | 93 | 94 | 91 |
| 1_3_9 | 91 | 91 | 91 | 92 | 94 | 90 |
| 1_3_10 | 93 | 92 | 94 | 93 | 94 | 93 |
| 1_3_11 | 93 | 91 | 94 | 93 | 94 | 93 |
| 1_4_5 | 93 | 92 | 94 | 91 | 92 | 90 |
| 1_4_6 | 93 | 91 | 95 | 91 | 92 | 90 |
| 1_4_7 | 90 | 88 | 92 | 94 | 94 | 95 |
| 1_4_8 | 92 | 93 | 91 | 93 | 92 | 93 |
| 1_4_9 | 92 | 92 | 91 | 93 | 94 | 91 |
| 1_4_10 | 91 | 89 | 92 | 94 | 94 | 95 |
| 1_4_11 | 90 | 91 | 90 | 93 | 94 | 93 |
| 1_5_6 | 91 | 86 | 95 | 87 | 84 | 90 |
| 1_5_7 | 92 | 92 | 91 | 90 | 88 | 91 |
| 1_5_8 | 93 | 93 | 92 | 90 | 88 | 91 |
| 1_5_9 | 90 | 86 | 94 | 89 | 86 | 91 |
| 1_5_10 | 90 | 91 | 90 | 90 | 90 | 90 |
| 1_5_11 | 91 | 89 | 93 | 92 | 90 | 93 |
| 1_6_7 | 94 | 94 | 94 | 93 | 92 | 93 |
| 1_6_8 | 92 | 92 | 91 | 91 | 92 | 90 |
| 1_6_9 | 91 | 86 | 95 | 89 | 86 | 91 |
| 1_6_10 | 91 | 91 | 91 | 90 | 90 | 90 |
| 1_6_11 | 93 | 90 | 96 | 91 | 88 | 93 |
| 1_7_8 | 90 | 90 | 90 | 92 | 90 | 93 |
| 1_7_9 | 89 | 88 | 90 | 91 | 90 | 91 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 1_7_10 | 91 | 91 | 90 | 91 | 90 | 91 |
| 1_7_11 | 90 | 89 | 91 | 92 | 90 | 93 |
| 1_8_9 | 89 | 88 | 90 | 90 | 88 | 91 |
| 1_8_10 | 89 | 92 | 86 | 90 | 88 | 91 |
| 1_8_11 | 91 | 89 | 93 | 90 | 90 | 90 |
| 1_9_10 | 90 | 92 | 88 | 89 | 88 | 90 |
| 1_9_11 | 90 | 88 | 91 | 91 | 88 | 93 |
| 1_10_11 | 91 | 92 | 90 | 90 | 88 | 91 |
| 2_3_4 | 88 | 88 | 88 | 86 | 88 | 84 |
| 2_3_5 | 93 | 93 | 93 | 92 | 96 | 88 |
| 2_3_6 | 91 | 89 | 92 | 88 | 90 | 86 |
| 2_3_7 | 90 | 88 | 91 | 89 | 88 | 90 |
| 2_3_8 | 86 | 87 | 85 | 85 | 88 | 83 |
| 2_3_9 | 87 | 88 | 86 | 88 | 92 | 84 |
| 2_3_10 | 88 | 87 | 90 | 88 | 88 | 88 |
| 2_3_11 | 87 | 88 | 87 | 86 | 88 | 84 |
| 2_4_5 | 93 | 91 | 94 | 91 | 94 | 88 |
| 2_4_6 | 89 | 86 | 91 | 87 | 88 | 86 |
| 2_4_7 | 88 | 83 | 92 | 90 | 86 | 93 |
| 2_4_8 | 84 | 83 | 84 | 85 | 84 | 86 |
| 2_4_9 | 84 | 82 | 85 | 87 | 86 | 88 |
| 2_4_10 | 84 | 82 | 87 | 87 | 88 | 86 |
| 2_4_11 | 84 | 83 | 85 | 87 | 86 | 88 |
| 2_5_6 | 92 | 89 | 95 | 93 | 94 | 93 |
| 2_5_7 | 91 | 89 | 92 | 92 | 94 | 90 |
| 2_5_8 | 92 | 89 | 94 | 92 | 90 | 93 |
| 2_5_9 | 91 | 87 | 95 | 93 | 94 | 93 |
| 2_5_10 | 91 | 90 | 92 | 93 | 96 | 90 |
| 2_5_11 | 92 | 91 | 93 | 95 | 96 | 95 |
| 2_6_7 | 88 | 85 | 91 | 89 | 86 | 91 |
| 2_6_8 | 89 | 89 | 89 | 87 | 86 | 88 |
| 2_6_9 | 88 | 85 | 91 | 91 | 88 | 93 |
| 2_6_10 | 89 | 86 | 91 | 89 | 86 | 91 |
| 2_6_11 | 87 | 84 | 90 | 92 | 88 | 95 |
| 2_7_8 | 84 | 85 | 84 | 88 | 86 | 90 |
| 2_7_9 | 84 | 81 | 87 | 89 | 88 | 90 |
| 2_7_10 | 83 | 82 | 84 | 87 | 86 | 88 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 2_7_11 | 84 | 82 | 86 | 90 | 84 | 95 |
| 2_8_9 | 84 | 83 | 84 | 85 | 84 | 86 |
| 2_8_10 | 83 | 81 | 84 | 86 | 84 | 88 |
| 2_8_11 | 84 | 83 | 84 | 86 | 82 | 90 |
| 2_9_10 | 84 | 82 | 85 | 88 | 90 | 86 |
| 2_9_11 | 83 | 81 | 85 | 87 | 84 | 90 |
| 2_10_11 | 85 | 83 | 87 | 89 | 84 | 93 |
| 3_4_5 | 94 | 96 | 92 | 91 | 96 | 86 |
| 3_4_6 | 89 | 89 | 90 | 89 | 92 | 86 |
| 3_4_7 | 84 | 81 | 88 | 86 | 88 | 84 |
| 3_4_8 | 83 | 84 | 82 | 83 | 86 | 81 |
| 3_4_9 | 86 | 88 | 85 | 86 | 88 | 84 |
| 3_4_10 | 85 | 81 | 90 | 87 | 88 | 86 |
| 3_4_11 | 83 | 81 | 85 | 85 | 86 | 84 |
| 3_5_6 | 95 | 96 | 94 | 91 | 98 | 84 |
| 3_5_7 | 95 | 96 | 95 | 93 | 98 | 88 |
| 3_5_8 | 93 | 95 | 92 | 92 | 98 | 86 |
| 3_5_9 | 94 | 95 | 93 | 90 | 96 | 84 |
| 3_5_10 | 95 | 96 | 94 | 92 | 98 | 86 |
| 3_5_11 | 93 | 95 | 92 | 92 | 98 | 86 |
| 3_6_7 | 92 | 90 | 93 | 88 | 92 | 84 |
| 3_6_8 | 90 | 91 | 89 | 88 | 92 | 84 |
| 3_6_9 | 90 | 91 | 89 | 88 | 92 | 84 |
| 3_6_10 | 90 | 88 | 91 | 88 | 90 | 86 |
| 3_6_11 | 89 | 89 | 89 | 88 | 90 | 86 |
| 3_7_8 | 83 | 83 | 83 | 82 | 88 | 77 |
| 3_7_9 | 88 | 92 | 85 | 89 | 94 | 84 |
| 3_7_10 | 84 | 82 | 86 | 83 | 86 | 81 |
| 3_7_11 | 84 | 83 | 85 | 84 | 86 | 83 |
| 3_8_9 | 86 | 88 | 84 | 87 | 90 | 84 |
| 3_8_10 | 82 | 81 | 83 | 82 | 84 | 81 |
| 3_8_11 | 82 | 81 | 83 | 80 | 84 | 77 |
| 3_9_10 | 89 | 89 | 90 | 86 | 90 | 83 |
| 3_9_11 | 86 | 89 | 84 | 87 | 90 | 84 |
| 3_10_11 | 84 | 81 | 87 | 83 | 84 | 83 |
| 4_5_6 | 94 | 94 | 94 | 91 | 96 | 86 |
| 4_5_7 | 92 | 92 | 92 | 90 | 92 | 88 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 4_5_8 | 92 | 92 | 91 | 91 | 94 | 88 |
| 4_5_9 | 91 | 91 | 91 | 90 | 94 | 86 |
| 4_5_10 | 93 | 92 | 94 | 89 | 90 | 88 |
| 4_5_11 | 93 | 95 | 92 | 92 | 96 | 88 |
| 4_6_7 | 91 | 90 | 91 | 89 | 90 | 88 |
| 4_6_8 | 88 | 89 | 88 | 89 | 94 | 84 |
| 4_6_9 | 89 | 88 | 90 | 88 | 88 | 88 |
| 4_6_10 | 90 | 88 | 92 | 88 | 90 | 86 |
| 4_6_11 | 88 | 88 | 89 | 88 | 92 | 84 |
| 4_7_8 | 82 | 82 | 83 | 85 | 88 | 83 |
| 4_7_9 | 85 | 87 | 84 | 82 | 78 | 86 |
| 4_7_10 | 84 | 81 | 86 | 84 | 84 | 84 |
| 4_7_11 | 83 | 84 | 83 | 87 | 86 | 88 |
| 4_8_9 | 85 | 87 | 83 | 85 | 88 | 83 |
| 4_8_10 | 82 | 82 | 82 | 84 | 86 | 83 |
| 4_8_11 | 82 | 83 | 82 | 83 | 86 | 81 |
| 4_9_10 | 84 | 83 | 86 | 84 | 82 | 86 |
| 4_9_11 | 85 | 85 | 85 | 84 | 86 | 83 |
| 4_10_11 | 83 | 80 | 86 | 85 | 84 | 86 |
| 5_6_7 | 93 | 94 | 91 | 90 | 94 | 86 |
| 5_6_8 | 89 | 89 | 90 | 86 | 84 | 88 |
| 5_6_9 | 90 | 84 | 95 | 85 | 82 | 88 |
| 5_6_10 | 86 | 87 | 85 | 89 | 94 | 84 |
| 5_6_11 | 89 | 89 | 90 | 90 | 92 | 88 |
| 5_7_8 | 91 | 95 | 87 | 88 | 90 | 86 |
| 5_7_9 | 93 | 95 | 90 | 89 | 92 | 86 |
| 5_7_10 | 92 | 94 | 90 | 90 | 92 | 88 |
| 5_7_11 | 91 | 94 | 89 | 90 | 94 | 86 |
| 5_8_9 | 86 | 88 | 85 | 83 | 82 | 84 |
| 5_8_10 | 87 | 91 | 83 | 87 | 88 | 86 |
| 5_8_11 | 89 | 90 | 88 | 88 | 88 | 88 |
| 5_9_10 | 85 | 89 | 83 | 87 | 92 | 83 |
| 5_9_11 | 88 | 89 | 88 | 86 | 86 | 86 |
| 5_10_11 | 86 | 89 | 83 | 90 | 92 | 88 |
| 6_7_8 | 87 | 87 | 87 | 80 | 80 | 81 |
| 6_7_9 | 87 | 86 | 88 | 82 | 82 | 83 |
| 6_7_10 | 86 | 87 | 86 | 85 | 86 | 84 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 6_7_11 | 86 | 88 | 84 | 87 | 88 | 86 |
| 6_8_9 | 86 | 86 | 86 | 79 | 78 | 81 |
| 6_8_10 | 87 | 89 | 85 | 79 | 84 | 75 |
| 6_8_11 | 87 | 89 | 86 | 80 | 82 | 79 |
| 6_9_10 | 86 | 86 | 86 | 82 | 80 | 84 |
| 6_9_11 | 85 | 83 | 88 | 80 | 74 | 86 |
| 6_10_11 | 84 | 87 | 83 | 84 | 82 | 86 |
| 7_8_9 | 83 | 85 | 82 | 85 | 88 | 83 |
| 7_8_10 | 81 | 80 | 83 | 81 | 84 | 79 |
| 7_8_11 | 79 | 78 | 81 | 81 | 82 | 81 |
| 7_9_10 | 83 | 84 | 83 | 85 | 88 | 83 |
| 7_9_11 | 82 | 81 | 83 | 84 | 86 | 83 |
| 7_10_11 | 79 | 75 | 83 | 83 | 84 | 83 |
| 8_9_10 | 84 | 85 | 83 | 85 | 88 | 83 |
| 8_9_11 | 81 | 82 | 80 | 85 | 90 | 81 |
| 8_10_11 | 81 | 79 | 83 | 82 | 86 | 79 |
| 9_10_11 | 84 | 85 | 83 | 84 | 88 | 81 |
| 1_2_3_4 | 92 | 92 | 91 | 93 | 94 | 93 |
| 1_2_3_5 | 95 | 96 | 95 | 92 | 94 | 90 |
| 1_2_3_6 | 93 | 93 | 94 | 93 | 92 | 93 |
| 1_2_3_7 | 93 | 94 | 92 | 94 | 94 | 95 |
| 1_2_3_8 | 92 | 92 | 91 | 93 | 94 | 93 |
| 1_2_3_9 | 92 | 92 | 91 | 93 | 94 | 93 |
| 1_2_3_10 | 92 | 92 | 91 | 93 | 94 | 93 |
| 1_2_3_11 | 91 | 92 | 90 | 93 | 94 | 91 |
| 1_2_4_5 | 93 | 91 | 94 | 93 | 94 | 91 |
| 1_2_4_6 | 92 | 90 | 94 | 93 | 92 | 93 |
| 1_2_4_7 | 92 | 89 | 94 | 93 | 90 | 95 |
| 1_2_4_8 | 91 | 90 | 91 | 92 | 92 | 91 |
| 1_2_4_9 | 91 | 90 | 91 | 93 | 94 | 93 |
| 1_2_4_10 | 91 | 89 | 93 | 94 | 94 | 95 |
| 1_2_4_11 | 90 | 89 | 90 | 93 | 94 | 93 |
| 1_2_5_6 | 94 | 92 | 96 | 93 | 92 | 93 |
| 1_2_5_7 | 93 | 91 | 95 | 94 | 94 | 95 |
| 1_2_5_8 | 93 | 94 | 93 | 94 | 94 | 95 |
| 1_2_5_9 | 93 | 90 | 95 | 93 | 94 | 93 |
| 1_2_5_10 | 91 | 90 | 92 | 94 | 94 | 95 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 1_2_5_11 | 92 | 91 | 93 | 94 | 94 | 95 |
| 1_2_6_7 | 92 | 88 | 95 | 92 | 90 | 93 |
| 1_2_6_8 | 91 | 90 | 91 | 93 | 90 | 95 |
| 1_2_6_9 | 92 | 88 | 96 | 92 | 92 | 91 |
| 1_2_6_10 | 92 | 88 | 95 | 94 | 92 | 97 |
| 1_2_6_11 | 91 | 88 | 94 | 94 | 92 | 97 |
| 1_2_7_8 | 91 | 88 | 93 | 93 | 90 | 95 |
| 1_2_7_9 | 89 | 86 | 92 | 93 | 92 | 95 |
| 1_2_7_10 | 90 | 86 | 93 | 94 | 92 | 97 |
| 1_2_7_11 | 90 | 87 | 93 | 93 | 90 | 97 |
| 1_2_8_9 | 91 | 89 | 92 | 92 | 90 | 93 |
| 1_2_8_10 | 91 | 90 | 91 | 92 | 90 | 93 |
| 1_2_8_11 | 90 | 89 | 90 | 93 | 90 | 95 |
| 1_2_9_10 | 89 | 87 | 91 | 93 | 92 | 95 |
| 1_2_9_11 | 90 | 87 | 92 | 93 | 92 | 95 |
| 1_2_10_11 | 90 | 87 | 92 | 95 | 92 | 98 |
| 1_3_4_5 | 94 | 96 | 93 | 92 | 94 | 90 |
| 1_3_4_6 | 93 | 94 | 93 | 93 | 96 | 90 |
| 1_3_4_7 | 94 | 95 | 94 | 95 | 96 | 95 |
| 1_3_4_8 | 91 | 93 | 90 | 93 | 94 | 91 |
| 1_3_4_9 | 91 | 92 | 90 | 93 | 94 | 91 |
| 1_3_4_10 | 92 | 91 | 93 | 93 | 94 | 93 |
| 1_3_4_11 | 92 | 92 | 92 | 93 | 94 | 91 |
| 1_3_5_6 | 95 | 96 | 94 | 93 | 96 | 90 |
| 1_3_5_7 | 95 | 96 | 95 | 93 | 96 | 91 |
| 1_3_5_8 | 93 | 95 | 91 | 93 | 96 | 90 |
| 1_3_5_9 | 93 | 95 | 92 | 92 | 94 | 90 |
| 1_3_5_10 | 96 | 97 | 95 | 93 | 94 | 91 |
| 1_3_5_11 | 94 | 95 | 94 | 92 | 94 | 90 |
| 1_3_6_7 | 94 | 95 | 94 | 93 | 96 | 90 |
| 1_3_6_8 | 93 | 94 | 92 | 91 | 94 | 88 |
| 1_3_6_9 | 93 | 94 | 93 | 92 | 94 | 90 |
| 1_3_6_10 | 93 | 94 | 93 | 93 | 94 | 91 |
| 1_3_6_11 | 94 | 96 | 93 | 92 | 94 | 90 |
| 1_3_7_8 | 94 | 94 | 94 | 94 | 96 | 93 |
| 1_3_7_9 | 95 | 95 | 95 | 93 | 96 | 91 |
| 1_3_7_10 | 95 | 95 | 95 | 95 | 96 | 95 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 1_3_7_11 | 95 | 96 | 95 | 95 | 96 | 95 |
| 1_3_8_9 | 91 | 91 | 90 | 92 | 94 | 90 |
| 1_3_8_10 | 92 | 91 | 92 | 94 | 94 | 95 |
| 1_3_8_11 | 92 | 94 | 90 | 91 | 94 | 88 |
| 1_3_9_10 | 92 | 91 | 93 | 93 | 94 | 93 |
| 1_3_9_11 | 92 | 91 | 92 | 93 | 94 | 91 |
| 1_3_10_11 | 92 | 91 | 92 | 93 | 94 | 93 |
| 1_4_5_6 | 93 | 92 | 94 | 92 | 94 | 90 |
| 1_4_5_7 | 93 | 92 | 94 | 93 | 92 | 93 |
| 1_4_5_8 | 93 | 94 | 91 | 93 | 94 | 91 |
| 1_4_5_9 | 93 | 91 | 94 | 91 | 92 | 90 |
| 1_4_5_10 | 93 | 91 | 94 | 93 | 92 | 93 |
| 1_4_5_11 | 93 | 93 | 94 | 92 | 94 | 90 |
| 1_4_6_7 | 93 | 93 | 92 | 92 | 94 | 90 |
| 1_4_6_8 | 92 | 91 | 92 | 93 | 94 | 91 |
| 1_4_6_9 | 93 | 91 | 95 | 92 | 92 | 91 |
| 1_4_6_10 | 92 | 91 | 92 | 91 | 92 | 90 |
| 1_4_6_11 | 93 | 91 | 95 | 90 | 92 | 88 |
| 1_4_7_8 | 92 | 90 | 93 | 93 | 92 | 93 |
| 1_4_7_9 | 90 | 88 | 92 | 93 | 90 | 95 |
| 1_4_7_10 | 90 | 88 | 92 | 94 | 94 | 95 |
| 1_4_7_11 | 91 | 89 | 92 | 94 | 94 | 95 |
| 1_4_8_9 | 92 | 93 | 91 | 93 | 92 | 93 |
| 1_4_8_10 | 91 | 89 | 93 | 93 | 92 | 93 |
| 1_4_8_11 | 90 | 90 | 90 | 93 | 92 | 95 |
| 1_4_9_10 | 90 | 88 | 92 | 93 | 94 | 93 |
| 1_4_9_11 | 91 | 91 | 90 | 93 | 94 | 93 |
| 1_4_10_11 | 91 | 90 | 92 | 94 | 94 | 95 |
| 1_5_6_7 | 92 | 92 | 92 | 90 | 88 | 91 |
| 1_5_6_8 | 93 | 92 | 93 | 90 | 86 | 93 |
| 1_5_6_9 | 91 | 86 | 95 | 88 | 84 | 91 |
| 1_5_6_10 | 90 | 88 | 92 | 90 | 90 | 90 |
| 1_5_6_11 | 92 | 90 | 94 | 93 | 92 | 93 |
| 1_5_7_8 | 92 | 95 | 89 | 90 | 90 | 90 |
| 1_5_7_9 | 92 | 92 | 91 | 90 | 88 | 91 |
| 1_5_7_10 | 92 | 92 | 91 | 90 | 88 | 91 |
| 1_5_7_11 | 92 | 92 | 91 | 91 | 90 | 91 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 1_5_8_9 | 93 | 93 | 92 | 91 | 88 | 93 |
| 1_5_8_10 | 91 | 95 | 88 | 90 | 88 | 91 |
| 1_5_8_11 | 92 | 93 | 91 | 90 | 88 | 91 |
| 1_5_9_10 | 90 | 91 | 90 | 90 | 90 | 90 |
| 1_5_9_11 | 91 | 89 | 93 | 91 | 88 | 93 |
| 1_5_10_11 | 89 | 90 | 89 | 91 | 90 | 91 |
| 1_6_7_8 | 91 | 93 | 90 | 90 | 90 | 90 |
| 1_6_7_9 | 94 | 94 | 94 | 93 | 92 | 95 |
| 1_6_7_10 | 93 | 94 | 93 | 92 | 92 | 91 |
| 1_6_7_11 | 94 | 94 | 94 | 93 | 92 | 93 |
| 1_6_8_9 | 92 | 92 | 91 | 90 | 90 | 90 |
| 1_6_8_10 | 91 | 92 | 90 | 91 | 90 | 91 |
| 1_6_8_11 | 91 | 90 | 91 | 90 | 90 | 90 |
| 1_6_9_10 | 92 | 91 | 92 | 90 | 90 | 90 |
| 1_6_9_11 | 93 | 90 | 96 | 91 | 88 | 93 |
| 1_6_10_11 | 91 | 91 | 91 | 90 | 90 | 90 |
| 1_7_8_9 | 90 | 90 | 90 | 92 | 90 | 93 |
| 1_7_8_10 | 89 | 92 | 86 | 92 | 90 | 93 |
| 1_7_8_11 | 90 | 90 | 90 | 90 | 88 | 91 |
| 1_7_9_10 | 91 | 91 | 91 | 90 | 90 | 90 |
| 1_7_9_11 | 90 | 88 | 91 | 91 | 90 | 91 |
| 1_7_10_11 | 91 | 90 | 91 | 90 | 90 | 90 |
| 1_8_9_10 | 89 | 92 | 86 | 92 | 90 | 93 |
| 1_8_9_11 | 89 | 89 | 90 | 89 | 88 | 90 |
| 1_8_10_11 | 89 | 92 | 86 | 91 | 90 | 91 |
| 1_9_10_11 | 90 | 92 | 89 | 90 | 88 | 91 |
| 2_3_4_5 | 93 | 93 | 93 | 92 | 96 | 88 |
| 2_3_4_6 | 91 | 89 | 92 | 88 | 90 | 86 |
| 2_3_4_7 | 90 | 89 | 91 | 89 | 86 | 91 |
| 2_3_4_8 | 85 | 87 | 84 | 86 | 88 | 84 |
| 2_3_4_9 | 87 | 88 | 86 | 88 | 92 | 84 |
| 2_3_4_10 | 89 | 88 | 90 | 88 | 88 | 88 |
| 2_3_4_11 | 88 | 88 | 88 | 86 | 88 | 84 |
| 2_3_5_6 | 93 | 93 | 94 | 92 | 96 | 88 |
| 2_3_5_7 | 94 | 94 | 95 | 93 | 96 | 90 |
| 2_3_5_8 | 94 | 95 | 94 | 91 | 96 | 86 |
| 2_3_5_9 | 93 | 93 | 94 | 91 | 96 | 86 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 2_3_5_10 | 94 | 93 | 95 | 92 | 96 | 88 |
| 2_3_5_11 | 93 | 94 | 93 | 92 | 96 | 88 |
| 2_3_6_7 | 93 | 93 | 94 | 91 | 90 | 91 |
| 2_3_6_8 | 89 | 90 | 89 | 89 | 92 | 86 |
| 2_3_6_9 | 89 | 89 | 90 | 87 | 90 | 84 |
| 2_3_6_10 | 91 | 90 | 92 | 89 | 90 | 88 |
| 2_3_6_11 | 89 | 89 | 90 | 88 | 90 | 86 |
| 2_3_7_8 | 89 | 88 | 90 | 86 | 86 | 86 |
| 2_3_7_9 | 91 | 91 | 90 | 90 | 90 | 90 |
| 2_3_7_10 | 90 | 89 | 90 | 90 | 88 | 91 |
| 2_3_7_11 | 91 | 90 | 91 | 90 | 86 | 93 |
| 2_3_8_9 | 86 | 88 | 84 | 87 | 92 | 83 |
| 2_3_8_10 | 86 | 87 | 86 | 88 | 86 | 90 |
| 2_3_8_11 | 86 | 87 | 85 | 86 | 90 | 83 |
| 2_3_9_10 | 90 | 88 | 91 | 89 | 92 | 86 |
| 2_3_9_11 | 87 | 88 | 87 | 88 | 90 | 86 |
| 2_3_10_11 | 88 | 87 | 89 | 89 | 88 | 90 |
| 2_4_5_6 | 94 | 94 | 94 | 91 | 94 | 88 |
| 2_4_5_7 | 93 | 91 | 94 | 90 | 94 | 86 |
| 2_4_5_8 | 92 | 92 | 92 | 91 | 94 | 88 |
| 2_4_5_9 | 92 | 91 | 92 | 91 | 94 | 88 |
| 2_4_5_10 | 93 | 91 | 94 | 90 | 94 | 86 |
| 2_4_5_11 | 93 | 94 | 91 | 93 | 96 | 90 |
| 2_4_6_7 | 92 | 90 | 93 | 89 | 86 | 91 |
| 2_4_6_8 | 88 | 88 | 89 | 89 | 92 | 86 |
| 2_4_6_9 | 88 | 87 | 90 | 88 | 88 | 88 |
| 2_4_6_10 | 90 | 86 | 93 | 89 | 88 | 90 |
| 2_4_6_11 | 88 | 86 | 90 | 90 | 90 | 90 |
| 2_4_7_8 | 84 | 84 | 85 | 86 | 86 | 86 |
| 2_4_7_9 | 84 | 83 | 85 | 84 | 82 | 86 |
| 2_4_7_10 | 86 | 84 | 89 | 88 | 86 | 90 |
| 2_4_7_11 | 87 | 85 | 90 | 91 | 88 | 93 |
| 2_4_8_9 | 84 | 84 | 84 | 84 | 86 | 83 |
| 2_4_8_10 | 84 | 83 | 85 | 85 | 84 | 86 |
| 2_4_8_11 | 83 | 83 | 83 | 87 | 86 | 88 |
| 2_4_9_10 | 86 | 82 | 90 | 85 | 84 | 86 |
| 2_4_9_11 | 84 | 82 | 85 | 86 | 86 | 86 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 2_4_10_11 | 84 | 83 | 86 | 90 | 86 | 93 |
| 2_5_6_7 | 92 | 89 | 95 | 93 | 96 | 90 |
| 2_5_6_8 | 94 | 92 | 96 | 93 | 94 | 91 |
| 2_5_6_9 | 92 | 89 | 95 | 93 | 94 | 93 |
| 2_5_6_10 | 93 | 92 | 94 | 94 | 98 | 91 |
| 2_5_6_11 | 92 | 90 | 93 | 95 | 96 | 95 |
| 2_5_7_8 | 90 | 88 | 91 | 89 | 90 | 88 |
| 2_5_7_9 | 91 | 89 | 92 | 92 | 94 | 90 |
| 2_5_7_10 | 91 | 90 | 92 | 93 | 96 | 90 |
| 2_5_7_11 | 92 | 91 | 93 | 95 | 96 | 95 |
| 2_5_8_9 | 92 | 89 | 95 | 92 | 90 | 93 |
| 2_5_8_10 | 90 | 89 | 90 | 90 | 92 | 88 |
| 2_5_8_11 | 91 | 90 | 92 | 93 | 94 | 91 |
| 2_5_9_10 | 91 | 90 | 91 | 93 | 96 | 90 |
| 2_5_9_11 | 92 | 91 | 93 | 94 | 96 | 93 |
| 2_5_10_11 | 92 | 91 | 93 | 94 | 96 | 93 |
| 2_6_7_8 | 89 | 88 | 90 | 88 | 86 | 90 |
| 2_6_7_9 | 88 | 84 | 91 | 91 | 86 | 95 |
| 2_6_7_10 | 87 | 85 | 90 | 90 | 88 | 91 |
| 2_6_7_11 | 87 | 83 | 91 | 93 | 90 | 95 |
| 2_6_8_9 | 89 | 89 | 89 | 87 | 86 | 88 |
| 2_6_8_10 | 89 | 89 | 89 | 87 | 86 | 88 |
| 2_6_8_11 | 89 | 89 | 89 | 88 | 88 | 88 |
| 2_6_9_10 | 87 | 84 | 90 | 89 | 86 | 91 |
| 2_6_9_11 | 87 | 84 | 90 | 91 | 88 | 93 |
| 2_6_10_11 | 87 | 84 | 90 | 92 | 88 | 95 |
| 2_7_8_9 | 85 | 86 | 84 | 88 | 86 | 90 |
| 2_7_8_10 | 83 | 86 | 81 | 86 | 86 | 86 |
| 2_7_8_11 | 84 | 85 | 83 | 86 | 82 | 90 |
| 2_7_9_10 | 83 | 83 | 83 | 87 | 88 | 86 |
| 2_7_9_11 | 84 | 83 | 85 | 89 | 84 | 93 |
| 2_7_10_11 | 84 | 84 | 84 | 88 | 84 | 91 |
| 2_8_9_10 | 83 | 83 | 83 | 84 | 84 | 84 |
| 2_8_9_11 | 84 | 84 | 84 | 87 | 84 | 90 |
| 2_8_10_11 | 83 | 83 | 83 | 86 | 82 | 90 |
| 2_9_10_11 | 83 | 81 | 85 | 87 | 84 | 90 |
| 3_4_5_6 | 95 | 96 | 94 | 92 | 98 | 86 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 3_4_5_7 | 94 | 95 | 94 | 93 | 98 | 88 |
| 3_4_5_8 | 93 | 96 | 90 | 92 | 98 | 86 |
| 3_4_5_9 | 94 | 96 | 92 | 91 | 96 | 86 |
| 3_4_5_10 | 95 | 96 | 94 | 92 | 96 | 88 |
| 3_4_5_11 | 93 | 96 | 91 | 91 | 96 | 86 |
| 3_4_6_7 | 92 | 92 | 91 | 91 | 94 | 88 |
| 3_4_6_8 | 89 | 90 | 88 | 89 | 92 | 86 |
| 3_4_6_9 | 90 | 90 | 90 | 88 | 92 | 84 |
| 3_4_6_10 | 91 | 89 | 92 | 88 | 90 | 86 |
| 3_4_6_11 | 89 | 89 | 90 | 89 | 92 | 86 |
| 3_4_7_8 | 85 | 84 | 87 | 87 | 90 | 84 |
| 3_4_7_9 | 86 | 88 | 85 | 86 | 90 | 83 |
| 3_4_7_10 | 85 | 81 | 89 | 86 | 88 | 84 |
| 3_4_7_11 | 85 | 83 | 87 | 88 | 90 | 86 |
| 3_4_8_9 | 86 | 89 | 84 | 86 | 88 | 84 |
| 3_4_8_10 | 84 | 82 | 85 | 84 | 88 | 81 |
| 3_4_8_11 | 81 | 84 | 79 | 83 | 86 | 81 |
| 3_4_9_10 | 90 | 90 | 90 | 87 | 88 | 86 |
| 3_4_9_11 | 86 | 88 | 85 | 86 | 88 | 84 |
| 3_4_10_11 | 85 | 80 | 90 | 87 | 86 | 88 |
| 3_5_6_7 | 95 | 96 | 95 | 93 | 98 | 88 |
| 3_5_6_8 | 94 | 95 | 94 | 91 | 98 | 84 |
| 3_5_6_9 | 95 | 96 | 94 | 92 | 98 | 86 |
| 3_5_6_10 | 95 | 96 | 95 | 93 | 98 | 88 |
| 3_5_6_11 | 95 | 96 | 94 | 92 | 98 | 86 |
| 3_5_7_8 | 95 | 95 | 95 | 91 | 98 | 84 |
| 3_5_7_9 | 95 | 97 | 93 | 91 | 98 | 84 |
| 3_5_7_10 | 95 | 96 | 94 | 92 | 98 | 86 |
| 3_5_7_11 | 96 | 97 | 95 | 93 | 98 | 90 |
| 3_5_8_9 | 92 | 95 | 90 | 90 | 98 | 83 |
| 3_5_8_10 | 95 | 95 | 96 | 91 | 94 | 88 |
| 3_5_8_11 | 95 | 96 | 94 | 92 | 98 | 86 |
| 3_5_9_10 | 94 | 94 | 94 | 92 | 96 | 88 |
| 3_5_9_11 | 93 | 95 | 91 | 90 | 96 | 84 |
| 3_5_10_11 | 94 | 95 | 94 | 93 | 98 | 88 |
| 3_6_7_8 | 90 | 89 | 91 | 88 | 92 | 84 |
| 3_6_7_9 | 91 | 92 | 90 | 87 | 94 | 81 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 3_6_7_10 | 92 | 90 | 93 | 87 | 90 | 84 |
| 3_6_7_11 | 91 | 90 | 92 | 88 | 92 | 84 |
| 3_6_8_9 | 89 | 90 | 89 | 87 | 92 | 83 |
| 3_6_8_10 | 90 | 91 | 90 | 88 | 90 | 86 |
| 3_6_8_11 | 89 | 91 | 88 | 85 | 92 | 79 |
| 3_6_9_10 | 92 | 91 | 93 | 89 | 90 | 88 |
| 3_6_9_11 | 90 | 91 | 89 | 88 | 92 | 84 |
| 3_6_10_11 | 90 | 89 | 91 | 89 | 92 | 86 |
| 3_7_8_9 | 87 | 92 | 83 | 87 | 92 | 83 |
| 3_7_8_10 | 83 | 83 | 83 | 82 | 88 | 77 |
| 3_7_8_11 | 84 | 84 | 83 | 81 | 86 | 77 |
| 3_7_9_10 | 89 | 92 | 87 | 86 | 90 | 83 |
| 3_7_9_11 | 89 | 92 | 86 | 88 | 92 | 84 |
| 3_7_10_11 | 84 | 84 | 85 | 84 | 86 | 83 |
| 3_8_9_10 | 87 | 88 | 87 | 85 | 88 | 83 |
| 3_8_9_11 | 87 | 90 | 84 | 86 | 88 | 84 |
| 3_8_10_11 | 82 | 82 | 83 | 80 | 82 | 79 |
| 3_9_10_11 | 89 | 89 | 90 | 86 | 90 | 83 |
| 4_5_6_7 | 94 | 94 | 94 | 91 | 94 | 88 |
| 4_5_6_8 | 93 | 93 | 92 | 92 | 96 | 88 |
| 4_5_6_9 | 93 | 93 | 94 | 91 | 96 | 86 |
| 4_5_6_10 | 94 | 94 | 94 | 90 | 90 | 90 |
| 4_5_6_11 | 93 | 94 | 92 | 93 | 98 | 88 |
| 4_5_7_8 | 91 | 91 | 91 | 91 | 94 | 88 |
| 4_5_7_9 | 92 | 91 | 92 | 89 | 92 | 86 |
| 4_5_7_10 | 93 | 92 | 94 | 90 | 92 | 88 |
| 4_5_7_11 | 94 | 95 | 93 | 92 | 94 | 90 |
| 4_5_8_9 | 90 | 91 | 89 | 89 | 94 | 84 |
| 4_5_8_10 | 93 | 92 | 94 | 91 | 94 | 88 |
| 4_5_8_11 | 91 | 92 | 90 | 90 | 94 | 86 |
| 4_5_9_10 | 93 | 92 | 94 | 90 | 92 | 88 |
| 4_5_9_11 | 92 | 94 | 90 | 90 | 96 | 84 |
| 4_5_10_11 | 93 | 92 | 95 | 93 | 94 | 93 |
| 4_6_7_8 | 91 | 93 | 90 | 89 | 92 | 86 |
| 4_6_7_9 | 91 | 91 | 90 | 88 | 88 | 88 |
| 4_6_7_10 | 91 | 90 | 91 | 89 | 90 | 88 |
| 4_6_7_11 | 89 | 89 | 90 | 88 | 88 | 88 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 4_6_8_9 | 89 | 89 | 89 | 87 | 92 | 83 |
| 4_6_8_10 | 89 | 89 | 90 | 89 | 92 | 86 |
| 4_6_8_11 | 88 | 89 | 88 | 89 | 94 | 84 |
| 4_6_9_10 | 89 | 86 | 91 | 87 | 88 | 86 |
| 4_6_9_11 | 89 | 89 | 90 | 89 | 90 | 88 |
| 4_6_10_11 | 89 | 88 | 90 | 90 | 92 | 88 |
| 4_7_8_9 | 85 | 88 | 83 | 81 | 84 | 79 |
| 4_7_8_10 | 81 | 81 | 82 | 86 | 88 | 84 |
| 4_7_8_11 | 84 | 84 | 83 | 87 | 90 | 84 |
| 4_7_9_10 | 86 | 87 | 86 | 82 | 78 | 86 |
| 4_7_9_11 | 86 | 88 | 84 | 83 | 84 | 83 |
| 4_7_10_11 | 85 | 83 | 88 | 89 | 86 | 91 |
| 4_8_9_10 | 84 | 84 | 83 | 83 | 84 | 83 |
| 4_8_9_11 | 85 | 87 | 83 | 84 | 88 | 81 |
| 4_8_10_11 | 82 | 82 | 83 | 85 | 86 | 84 |
| 4_9_10_11 | 85 | 85 | 86 | 85 | 84 | 86 |
| 5_6_7_8 | 91 | 96 | 86 | 91 | 98 | 84 |
| 5_6_7_9 | 93 | 94 | 92 | 91 | 94 | 88 |
| 5_6_7_10 | 93 | 94 | 91 | 90 | 94 | 86 |
| 5_6_7_11 | 93 | 96 | 90 | 91 | 96 | 86 |
| 5_6_8_9 | 88 | 89 | 87 | 86 | 84 | 88 |
| 5_6_8_10 | 87 | 90 | 84 | 90 | 94 | 86 |
| 5_6_8_11 | 90 | 91 | 89 | 91 | 94 | 88 |
| 5_6_9_10 | 86 | 88 | 85 | 90 | 94 | 86 |
| 5_6_9_11 | 89 | 89 | 89 | 89 | 92 | 86 |
| 5_6_10_11 | 89 | 91 | 87 | 91 | 96 | 86 |
| 5_7_8_9 | 91 | 95 | 87 | 87 | 90 | 84 |
| 5_7_8_10 | 91 | 95 | 87 | 88 | 90 | 86 |
| 5_7_8_11 | 91 | 95 | 87 | 88 | 90 | 86 |
| 5_7_9_10 | 93 | 95 | 90 | 89 | 92 | 86 |
| 5_7_9_11 | 90 | 93 | 87 | 90 | 94 | 86 |
| 5_7_10_11 | 93 | 94 | 91 | 90 | 94 | 86 |
| 5_8_9_10 | 86 | 90 | 83 | 86 | 90 | 83 |
| 5_8_9_11 | 87 | 90 | 84 | 87 | 86 | 88 |
| 5_8_10_11 | 87 | 92 | 83 | 89 | 92 | 86 |
| 5_9_10_11 | 87 | 92 | 83 | 89 | 92 | 86 |
| 6_7_8_9 | 87 | 88 | 87 | 81 | 82 | 81 |

(continued)

| SEQ ID NO: | Training cohort | | | Validation cohort | | |
|---|---|---|---|---|---|---|
| | Accuracy (%) | Sensitivity (%) | Specificity (%) | Accuracy (%) | Sensitivity (%) | Specificity (%) |
| 6_7_8_10 | 87 | 89 | 86 | 79 | 82 | 77 |
| 6_7_8_11 | 87 | 87 | 87 | 80 | 80 | 81 |
| 6_7_9_10 | 86 | 86 | 87 | 83 | 84 | 83 |
| 6_7_9_11 | 86 | 87 | 86 | 87 | 88 | 86 |
| 6_7_10_11 | 85 | 88 | 83 | 87 | 88 | 86 |
| 6_8_9_10 | 87 | 91 | 83 | 81 | 84 | 79 |
| 6_8_9_11 | 86 | 86 | 87 | 83 | 84 | 83 |
| 6_8_10_11 | 88 | 90 | 86 | 80 | 84 | 77 |
| 6_9_10_11 | 86 | 87 | 85 | 83 | 84 | 83 |
| 7_8_9_10 | 83 | 85 | 82 | 85 | 88 | 83 |
| 7_8_9_11 | 83 | 85 | 82 | 85 | 88 | 83 |
| 7_8_10_11 | 81 | 80 | 83 | 80 | 82 | 79 |
| 7_9_10_11 | 82 | 81 | 83 | 84 | 86 | 83 |
| 8_9_10_11 | 84 | 85 | 83 | 86 | 90 | 83 |

[Example 3]

<Method for evaluating discriminant performance in other malignant brain tumor samples by using a combination of multiple gene markers using the validation cohort>

In this Example, a threshold or a discriminant was constructed in the training cohort using combinations of expression level measurement values of one to four of the polynucleotides consisting of the nucleotide sequences represented by SEQ ID NOs: 1 to 11 in the same way as in Examples 1 and 2, and was used to evaluate the discriminant performance in the validation cohort described in Reference Example 2 in the same way as the methods described in Examples 1 and 2.

Specifically, first, the miRNA expression levels of the training cohort obtained in Reference Example 1 and the validation cohort obtained in Reference Example 2 above were normalized. The normalization was carried out in a manner that the ratio of the average of expression level measurement values of three internal miRNA controls (hsa-miR-2861, hsa-miR-149-3p, and hsa-miR-4463) on a DNA chip relative to the pre-set value is determined for each sample, and this ratio is applied to all detection values of miRNAs in each sample. This approach is described in A. Shimomura et al., 2016, Cancer Sci., DOI: 10.1111.

Next, Fisher's linear discriminant analysis was conducted using the training cohort obtained in Reference Example 1 as to combinations of the expression level measurement values of one to four of the polynucleotides consisting of the nucleotide sequences represented by SEQ ID NOs: 1 to 11 corresponding to the 11 genes selected in Example 1, to construct a discriminant for determining the presence or absence of malignant brain tumor. Next, sensitivity was calculated in the validation cohort involving 51 persons in total of 37 primary central nervous system lymphoma patients, 6 ependymoma patients, 5 ganglioglioma patients, and 3 pilocytic astrocytoma patients (Reference Example 2) (Table 7), and the discriminant performance of the selected polynucleotides was validated on the basis of the sensitivity, using the independent samples.

The presence or absence of malignant brain tumor in the validation cohort of Reference Example 2 was determined using the combinations of the expression level measurement values of one to four of the polynucleotides consisting of the nucleotide sequences represented by SEQ ID NOs: 1 to 11. For example, as for the expression level measurement values of the polynucleotides consisting of the nucleotide sequences represented by SEQ ID NO: 1 and SEQ ID NO: 2, a discriminant score was calculated using the discriminant coefficients (SEQ ID NO: 1: -1.952, SEQ ID NO: 2: -1.071) and the constant term (-30.884) indicated in Table 5 on the basis of the discriminant prepared for determining the presence or absence of malignant brain tumor in the training cohort. Discriminant results were obtained from the discriminant scores by determining a sample having a discriminant score larger than 0 as being derived from malignant brain tumor and a sample having a discriminant score smaller than 0 as being derived from a benign brain tumor patient or a healthy subject and were compared between the training cohort and the validation cohort. As a result, a scatter diagram similar to that for glioma (astrocytoma, oligodendroglioma, and oligoastrocytoma) in the training cohort was obtained for other malignant brain tumors (primary central nervous system lymphoma, ependymoma, ganglioglioma, and pilocytic astrocytoma) in the validation cohort (see the right diagram of Figure 4). As for these nucleotide sequences represented by SEQ ID NO: 1 and SEQ ID NO: 2, the number of correctly identified samples in the detection of malignant brain tumor was calculated using the discriminant constructed in the training cohort. As a result, 43 true positives and 8 false negatives were obtained. From these values, 84% sensitivity was obtained as the discriminant performance. The sensitivity in the training cohort was 87%.

In this way, the discriminant performance was calculated for all combinations of the expression level measurement values of one to four of the polynucleotides consisting of the nucleotide sequences represented by SEQ ID NOs: 1 to 11. Among the 561 combinations in total, 514 combinations (Table 7) exhibited sensitivity beyond 71% sensitivity of the polynucleotide of SEQ ID NO: 11 alone, which is the smallest discriminant performance in Examples 1 and 2 and were found to be able to detect (discriminate) not only malignant brain tumor (astrocytoma, oligodendroglioma, and oligoastrocytoma) but primary central nervous system lymphoma, ependymoma, ganglioglioma, and pilocytic astrocytoma.

Examples of the number of the polynucleotides in any combination of the polynucleotides consisting of the nucleotide sequences represented by SEQ ID NOs: 1 to 11, which can also discriminate the malignant brain tumor mentioned above other than glioma (astrocytoma, oligodendroglioma, and oligoastrocytoma) include, but are not limited to, 1,2, 3, 4, 5 or more. The combinations of 2 or more polynucleotides described above were able to exhibit discrimination accuracy of 90% or higher, over the sensitivity of 86% of the polynucleotide of SEQ ID NO: 5 alone, which exhibited the highest discriminant performance.

Thus, use of these polynucleotides was also able to correctly discriminate malignant brain tumor for any of the malignant brain tumors described above other than glioma (astrocytoma, oligodendroglioma, and oligoastrocytoma) in the validation cohort.

Table 7

| SEQ ID NO: | Training cohort | | | Validation cohort |
|---|---|---|---|---|
| | Accuracy (%) | Sensitivity (%) | Specificity (%) | Sensitivity (%) |
| 1 | 88.7 | 83.7 | 93 | 82.4 |
| 2 | 84.9 | 80.6 | 88.6 | 72.5 |
| 5 | 85.4 | 74.5 | 94.7 | 86.3 |
| 6 | 79.2 | 76.5 | 81.6 | 78.4 |
| 9 | 76.4 | 73.5 | 78.9 | 74.5 |
| 10 | 78.3 | 74.5 | 81.6 | 72.5 |
| 1_2 | 90.1 | 86.7 | 93 | 84.3 |
| 1_3 | 91.5 | 91.8 | 91.2 | 90.2 |
| 1_4 | 91 | 91.8 | 90.4 | 86.3 |
| 1_5 | 90.1 | 85.7 | 93.9 | 90.2 |
| 1_6 | 91 | 85.7 | 95.6 | 88.2 |
| 1_7 | 90.1 | 88.8 | 91.2 | 86.3 |
| 1_8 | 89.2 | 87.8 | 90.4 | 88.2 |
| 1_9 | 88.7 | 83.7 | 93 | 82.4 |
| 1_10 | 90.6 | 91.8 | 89.5 | 84.3 |
| 1_11 | 90.6 | 87.8 | 93 | 84.3 |
| 2_3 | 87.7 | 87.8 | 87.7 | 74.5 |
| 2_4 | 85.4 | 81.6 | 88.6 | 74.5 |
| 2_5 | 91.5 | 86.7 | 95.6 | 86.3 |

(continued)

| SEQ ID NO: | Training cohort | | | Validation cohort |
|---|---|---|---|---|
| | Accuracy (%) | Sensitivity (%) | Specificity (%) | Sensitivity (%) |
| 2_6 | 88.7 | 84.7 | 92.1 | 84.3 |
| 2_7 | 84.9 | 80.6 | 88.6 | 72.5 |
| 2_8 | 83.5 | 81.6 | 85.1 | 74.5 |
| 2_9 | 84 | 80.6 | 86.8 | 76.5 |
| 2_10 | 84 | 81.6 | 86 | 74.5 |
| 2_11 | 84.9 | 82.7 | 86.8 | 78.4 |
| 3_5 | 94.3 | 95.9 | 93 | 92.2 |
| 3_6 | 88.7 | 88.8 | 88.6 | 88.2 |
| 3_8 | 80.2 | 79.6 | 80.7 | 72.5 |
| 3_9 | 86.3 | 88.8 | 84.2 | 72.5 |
| 4_5 | 92 | 91.8 | 92.1 | 84.3 |
| 4_6 | 87.7 | 86.7 | 88.6 | 86.3 |
| 4_8 | 82.1 | 82.7 | 81.6 | 72.5 |
| 4_9 | 84.4 | 83.7 | 85.1 | 72.5 |
| 5_6 | 88.7 | 83.7 | 93 | 82.4 |
| 5_7 | 92 | 93.9 | 90.4 | 84.3 |
| 5_8 | 88.7 | 88.8 | 88.6 | 88.2 |
| 5_9 | 84.4 | 78.6 | 89.5 | 88.2 |
| 5_10 | 86.3 | 89.8 | 83.3 | 90.2 |
| 5_11 | 88.2 | 87.8 | 88.6 | 88.2 |
| 6_7 | 86.3 | 87.8 | 85.1 | 80.4 |
| 6_8 | 85.4 | 86.7 | 84.2 | 80.4 |
| 6_9 | 83.5 | 80.6 | 86 | 76.5 |
| 6_10 | 84 | 85.7 | 82.5 | 78.4 |
| 6_11 | 84.4 | 85.7 | 83.3 | 74.5 |
| 8_9 | 80.7 | 81.6 | 79.8 | 80.4 |
| 9_10 | 82.5 | 82.7 | 82.5 | 80.4 |
| 9_11 | 81.1 | 81.6 | 80.7 | 74.5 |
| 1_2_3 | 91.5 | 91.8 | 91.2 | 84.3 |
| 1_2_4 | 90.6 | 89.8 | 91.2 | 86.3 |
| 1_2_5 | 92.9 | 89.8 | 95.6 | 88.2 |
| 1_2_6 | 91.5 | 87.8 | 94.7 | 86.3 |
| 1_2_7 | 89.6 | 85.7 | 93 | 84.3 |
| 1_2_8 | 90.6 | 89.8 | 91.2 | 86.3 |
| 1_2_9 | 89.6 | 86.7 | 92.1 | 84.3 |
| 1_2_10 | 89.6 | 86.7 | 92.1 | 84.3 |
| 1_2_11 | 89.6 | 86.7 | 92.1 | 84.3 |

(continued)

| SEQ ID NO: | Training cohort | | | Validation cohort |
|---|---|---|---|---|
| | Accuracy (%) | Sensitivity (%) | Specificity (%) | Sensitivity (%) |
| 1_3_4 | 91.5 | 91.8 | 91.2 | 88.2 |
| 1_3_5 | 94.3 | 94.9 | 93.9 | 90.2 |
| 1_3_6 | 92.9 | 93.9 | 92.1 | 88.2 |
| 1_3_7 | 94.8 | 94.9 | 94.7 | 86.3 |
| 1_3_8 | 90.6 | 90.8 | 90.4 | 90.2 |
| 1_3_9 | 91 | 90.8 | 91.2 | 90.2 |
| 1_3_10 | 92.9 | 91.8 | 93.9 | 86.3 |
| 1_3_11 | 92.5 | 90.8 | 93.9 | 90.2 |
| 1_4_5 | 92.9 | 91.8 | 93.9 | 84.3 |
| 1_4_6 | 92.9 | 90.8 | 94.7 | 90.2 |
| 1_4_7 | 90.1 | 87.8 | 92.1 | 90.2 |
| 1_4_8 | 92 | 92.9 | 91.2 | 88.2 |
| 1_4_9 | 91.5 | 91.8 | 91.2 | 86.3 |
| 1_4_10 | 90.6 | 88.8 | 92.1 | 84.3 |
| 1_4_11 | 90.1 | 90.8 | 89.5 | 86.3 |
| 1_5_6 | 90.6 | 85.7 | 94.7 | 86.3 |
| 1_5_7 | 91.5 | 91.8 | 91.2 | 88.2 |
| 1_5_8 | 92.5 | 92.9 | 92.1 | 90.2 |
| 1_5_9 | 90.1 | 85.7 | 93.9 | 88.2 |
| 1_5_10 | 90.1 | 90.8 | 89.5 | 90.2 |
| 1_5_11 | 91 | 88.8 | 93 | 92.2 |
| 1_6_7 | 93.9 | 93.9 | 93.9 | 88.2 |
| 1_6_8 | 91.5 | 91.8 | 91.2 | 88.2 |
| 1_6_9 | 90.6 | 85.7 | 94.7 | 88.2 |
| 1_6_10 | 91 | 90.8 | 91.2 | 90.2 |
| 1_6_11 | 92.9 | 89.8 | 95.6 | 88.2 |
| 1_7_8 | 90.1 | 89.8 | 90.4 | 82.4 |
| 1_7_9 | 89.2 | 87.8 | 90.4 | 86.3 |
| 1_7_10 | 90.6 | 90.8 | 90.4 | 84.3 |
| 1_7_11 | 90.1 | 88.8 | 91.2 | 86.3 |
| 1_8_9 | 89.2 | 87.8 | 90.4 | 86.3 |
| 1_8_10 | 88.7 | 91.8 | 86 | 86.3 |
| 1_8_11 | 91 | 88.8 | 93 | 88.2 |
| 1_9_10 | 89.6 | 91.8 | 87.7 | 84.3 |
| 1_9_11 | 89.6 | 87.8 | 91.2 | 84.3 |
| 1_10_11 | 90.6 | 91.8 | 89.5 | 84.3 |
| 2_3_5 | 92.9 | 92.9 | 93 | 92.2 |

(continued)

| SEQ ID NO: | Training cohort | | | Validation cohort |
|---|---|---|---|---|
| | Accuracy (%) | Sensitivity (%) | Specificity (%) | Sensitivity (%) |
| 2_3_6 | 90.6 | 88.8 | 92.1 | 88.2 |
| 2_3_7 | 89.6 | 87.8 | 91.2 | 78.4 |
| 2_3_8 | 85.8 | 86.7 | 85.1 | 76.5 |
| 2_3_9 | 86.8 | 87.8 | 86 | 76.5 |
| 2_3_10 | 88.2 | 86.7 | 89.5 | 74.5 |
| 2_3_11 | 87.3 | 87.8 | 86.8 | 72.5 |
| 2_4_5 | 92.5 | 90.8 | 93.9 | 84.3 |
| 2_4_6 | 88.7 | 85.7 | 91.2 | 90.2 |
| 2_4_7 | 87.7 | 82.7 | 92.1 | 76.5 |
| 2_4_8 | 83.5 | 82.7 | 84.2 | 82.4 |
| 2_4_9 | 83.5 | 81.6 | 85.1 | 80.4 |
| 2_4_10 | 84.4 | 81.6 | 86.8 | 74.5 |
| 2_4_11 | 84 | 82.7 | 85.1 | 76.5 |
| 2_5_6 | 92 | 88.8 | 94.7 | 92.2 |
| 2_5_7 | 90.6 | 88.8 | 92.1 | 86.3 |
| 2_5_8 | 91.5 | 88.8 | 93.9 | 88.2 |
| 2_5_9 | 91 | 86.7 | 94.7 | 88.2 |
| 2_5_10 | 91 | 89.8 | 92.1 | 88.2 |
| 2_5_11 | 92 | 90.8 | 93 | 88.2 |
| 2_6_7 | 88.2 | 84.7 | 91.2 | 86.3 |
| 2_6_8 | 88.7 | 88.8 | 88.6 | 88.2 |
| 2_6_9 | 88.2 | 84.7 | 91.2 | 84.3 |
| 2_6_10 | 88.7 | 85.7 | 91.2 | 86.3 |
| 2_6_11 | 87.3 | 83.7 | 90.4 | 84.3 |
| 2_7_8 | 84.4 | 84.7 | 84.2 | 78.4 |
| 2_7_9 | 84 | 80.6 | 86.8 | 76.5 |
| 2_7_10 | 83 | 81.6 | 84.2 | 72.5 |
| 2_7_11 | 84 | 81.6 | 86 | 78.4 |
| 2_8_9 | 83.5 | 82.7 | 84.2 | 78.4 |
| 2_8_10 | 82.5 | 80.6 | 84.2 | 76.5 |
| 2_8_11 | 83.5 | 82.7 | 84.2 | 80.4 |
| 2_9_10 | 83.5 | 81.6 | 85.1 | 76.5 |
| 2_9_11 | 83 | 80.6 | 85.1 | 78.4 |
| 2_10_11 | 84.9 | 82.7 | 86.8 | 78.4 |
| 3_4_5 | 93.9 | 95.9 | 92.1 | 92.2 |
| 3_4_6 | 89.2 | 88.8 | 89.5 | 88.2 |
| 3_4_7 | 84.4 | 80.6 | 87.7 | 76.5 |

(continued)

| SEQ ID NO: | Training cohort | | | Validation cohort |
|---|---|---|---|---|
| | Accuracy (%) | Sensitivity (%) | Specificity (%) | Sensitivity (%) |
| 3_5_6 | 94.8 | 95.9 | 93.9 | 94.1 |
| 3_5_7 | 95.3 | 95.9 | 94.7 | 94.1 |
| 3_5_8 | 93.4 | 94.9 | 92.1 | 92.2 |
| 3_5_9 | 93.9 | 94.9 | 93 | 92.2 |
| 3_5_10 | 94.8 | 95.9 | 93.9 | 92.2 |
| 3_5_11 | 93.4 | 94.9 | 92.1 | 92.2 |
| 3_6_7 | 91.5 | 89.8 | 93 | 88.2 |
| 3_6_8 | 89.6 | 90.8 | 88.6 | 88.2 |
| 3_6_9 | 89.6 | 90.8 | 88.6 | 88.2 |
| 3_6_10 | 89.6 | 87.8 | 91.2 | 88.2 |
| 3_6_11 | 88.7 | 88.8 | 88.6 | 88.2 |
| 3_7_8 | 82.5 | 82.7 | 82.5 | 78.4 |
| 3_7_9 | 88.2 | 91.8 | 85.1 | 78.4 |
| 3_8_9 | 85.8 | 87.8 | 84.2 | 76.5 |
| 3_9_11 | 86.3 | 88.8 | 84.2 | 76.5 |
| 4_5_6 | 93.9 | 93.9 | 93.9 | 92.2 |
| 4_5_7 | 92 | 91.8 | 92.1 | 84.3 |
| 4_5_8 | 91.5 | 91.8 | 91.2 | 86.3 |
| 4_5_9 | 91 | 90.8 | 91.2 | 84.3 |
| 4_5_10 | 92.9 | 91.8 | 93.9 | 84.3 |
| 4_5_11 | 93.4 | 94.9 | 92.1 | 84.3 |
| 4_6_7 | 90.6 | 89.8 | 91.2 | 90.2 |
| 4_6_8 | 88.2 | 88.8 | 87.7 | 84.3 |
| 4_6_9 | 88.7 | 87.8 | 89.5 | 88.2 |
| 4_6_10 | 90.1 | 87.8 | 92.1 | 86.3 |
| 4_6_11 | 88.2 | 87.8 | 88.6 | 88.2 |
| 4_7_8 | 82.1 | 81.6 | 82.5 | 74.5 |
| 4_7_9 | 85.4 | 86.7 | 84.2 | 76.5 |
| 4_7_11 | 83 | 83.7 | 82.5 | 74.5 |
| 4_8_9 | 84.9 | 86.7 | 83.3 | 76.5 |
| 4_8_10 | 81.6 | 81.6 | 81.6 | 72.5 |
| 4_8_11 | 82.1 | 82.7 | 81.6 | 72.5 |
| 4_9_10 | 84.4 | 82.7 | 86 | 76.5 |
| 4_9_11 | 84.9 | 84.7 | 85.1 | 72.5 |
| 5_6_7 | 92.5 | 93.9 | 91.2 | 88.2 |
| 5_6_8 | 89.2 | 88.8 | 89.5 | 86.3 |
| 5_6_9 | 89.6 | 83.7 | 94.7 | 86.3 |

(continued)

| SEQ ID NO: | Training cohort | | | Validation cohort |
|---|---|---|---|---|
| | Accuracy (%) | Sensitivity (%) | Specificity (%) | Sensitivity (%) |
| 5_6_10 | 85.8 | 86.7 | 85.1 | 90.2 |
| 5_6_11 | 89.2 | 88.8 | 89.5 | 84.3 |
| 5_7_8 | 90.6 | 94.9 | 86.8 | 86.3 |
| 5_7_9 | 92.5 | 94.9 | 90.4 | 86.3 |
| 5_7_10 | 92 | 93.9 | 90.4 | 84.3 |
| 5_7_11 | 91 | 93.9 | 88.6 | 86.3 |
| 5_8_9 | 86.3 | 87.8 | 85.1 | 90.2 |
| 5_8_10 | 86.8 | 90.8 | 83.3 | 88.2 |
| 5_8_11 | 88.7 | 89.8 | 87.7 | 90.2 |
| 5_9_10 | 85.4 | 88.8 | 82.5 | 92.2 |
| 5_9_11 | 88.2 | 88.8 | 87.7 | 86.3 |
| 5_10_11 | 85.8 | 88.8 | 83.3 | 90.2 |
| 6_7_8 | 86.8 | 86.7 | 86.8 | 84.3 |
| 6_7_9 | 86.8 | 85.7 | 87.7 | 78.4 |
| 6_7_10 | 86.3 | 86.7 | 86 | 84.3 |
| 6_7_11 | 85.8 | 87.8 | 84.2 | 80.4 |
| 6_8_9 | 85.8 | 85.7 | 86 | 80.4 |
| 6_8_10 | 86.8 | 88.8 | 85.1 | 78.4 |
| 6_8_11 | 87.3 | 88.8 | 86 | 80.4 |
| 6_9_10 | 85.8 | 85.7 | 86 | 78.4 |
| 6_9_11 | 85.4 | 82.7 | 87.7 | 76.5 |
| 6_10_11 | 84.4 | 86.7 | 82.5 | 80.4 |
| 7_8_9 | 83 | 84.7 | 81.6 | 76.5 |
| 8_9_10 | 83.5 | 84.7 | 82.5 | 80.4 |
| 8_9_11 | 80.7 | 81.6 | 79.8 | 80.4 |
| 8_10_11 | 81.1 | 78.6 | 83.3 | 72.5 |
| 9_10_11 | 84 | 84.7 | 83.3 | 78.4 |
| 1_2_3_4 | 91.5 | 91.8 | 91.2 | 84.3 |
| 1_2_3_5 | 95.3 | 95.9 | 94.7 | 90.2 |
| 1_2_3_6 | 93.4 | 92.9 | 93.9 | 88.2 |
| 1_2_3_7 | 92.9 | 93.9 | 92.1 | 82.4 |
| 1_2_3_8 | 91.5 | 91.8 | 91.2 | 86.3 |
| 1_2_3_9 | 91.5 | 91.8 | 91.2 | 84.3 |
| 1_2_3_10 | 91.5 | 91.8 | 91.2 | 82.4 |
| 1_2_3_11 | 91 | 91.8 | 90.4 | 84.3 |
| 1_2_4_5 | 92.5 | 90.8 | 93.9 | 84.3 |
| 1_2_4_6 | 92 | 89.8 | 93.9 | 90.2 |

(continued)

| SEQ ID NO: | Training cohort | | | Validation cohort |
|---|---|---|---|---|
| | Accuracy (%) | Sensitivity (%) | Specificity (%) | Sensitivity (%) |
| 1_2_4_7 | 91.5 | 88.8 | 93.9 | 88.2 |
| 1_2_4_8 | 90.6 | 89.8 | 91.2 | 88.2 |
| 1_2_4_9 | 90.6 | 89.8 | 91.2 | 86.3 |
| 1_2_4_10 | 91 | 88.8 | 93 | 86.3 |
| 1_2_4_11 | 89.6 | 88.8 | 90.4 | 86.3 |
| 1_2_5_6 | 93.9 | 91.8 | 95.6 | 90.2 |
| 1_2_5_7 | 92.9 | 90.8 | 94.7 | 86.3 |
| 1_2_5_8 | 93.4 | 93.9 | 93 | 88.2 |
| 1_2_5_9 | 92.5 | 89.8 | 94.7 | 88.2 |
| 1_2_5_10 | 91 | 89.8 | 92.1 | 88.2 |
| 1_2_5_11 | 92 | 90.8 | 93 | 88.2 |
| 1_2_6_7 | 91.5 | 87.8 | 94.7 | 84.3 |
| 1_2_6_8 | 90.6 | 89.8 | 91.2 | 90.2 |
| 1_2_6_9 | 92 | 87.8 | 95.6 | 86.3 |
| 1_2_6_10 | 91.5 | 87.8 | 94.7 | 84.3 |
| 1_2_6_11 | 91 | 87.8 | 93.9 | 86.3 |
| 1_2_7_8 | 90.6 | 87.8 | 93 | 84.3 |
| 1_2_7_9 | 89.2 | 85.7 | 92.1 | 84.3 |
| 1_2_7_10 | 89.6 | 85.7 | 93 | 84.3 |
| 1_2_7_11 | 90.1 | 86.7 | 93 | 84.3 |
| 1_2_8_9 | 90.6 | 88.8 | 92.1 | 86.3 |
| 1_2_8_10 | 90.6 | 89.8 | 91.2 | 86.3 |
| 1_2_8_11 | 89.6 | 88.8 | 90.4 | 86.3 |
| 1_2_9_10 | 89.2 | 86.7 | 91.2 | 84.3 |
| 1_2_9_11 | 89.6 | 86.7 | 92.1 | 84.3 |
| 1_2_10_11 | 89.6 | 86.7 | 92.1 | 84.3 |
| 1_3_4_5 | 94.3 | 95.9 | 93 | 90.2 |
| 1_3_4_6 | 93.4 | 93.9 | 93 | 90.2 |
| 1_3_4_7 | 94.3 | 94.9 | 93.9 | 88.2 |
| 1_3_4_8 | 91 | 92.9 | 89.5 | 88.2 |
| 1_3_4_9 | 91 | 91.8 | 90.4 | 88.2 |
| 1_3_4_10 | 92 | 90.8 | 93 | 88.2 |
| 1_3_4_11 | 92 | 91.8 | 92.1 | 88.2 |
| 1_3_5_6 | 94.8 | 95.9 | 93.9 | 92.2 |
| 1_3_5_7 | 95.3 | 95.9 | 94.7 | 90.2 |
| 1_3_5_8 | 92.9 | 94.9 | 91.2 | 92.2 |
| 1_3_5_9 | 93.4 | 94.9 | 92.1 | 90.2 |

(continued)

| SEQ ID NO: | Training cohort | | | Validation cohort |
|---|---|---|---|---|
| | Accuracy (%) | Sensitivity (%) | Specificity (%) | Sensitivity (%) |
| 1_3_5_10 | 95.8 | 96.9 | 94.7 | 90.2 |
| 1_3_5_11 | 94.3 | 94.9 | 93.9 | 90.2 |
| 1_3_6_7 | 94.3 | 94.9 | 93.9 | 90.2 |
| 1_3_6_8 | 92.9 | 93.9 | 92.1 | 90.2 |
| 1_3_6_9 | 93.4 | 93.9 | 93 | 88.2 |
| 1_3_6_10 | 93.4 | 93.9 | 93 | 88.2 |
| 1_3_6_11 | 94.3 | 95.9 | 93 | 88.2 |
| 1_3_7_8 | 93.9 | 93.9 | 93.9 | 86.3 |
| 1_3_7_9 | 94.8 | 94.9 | 94.7 | 86.3 |
| 1_3_7_10 | 94.8 | 94.9 | 94.7 | 86.3 |
| 1_3_7_11 | 95.3 | 95.9 | 94.7 | 86.3 |
| 1_3_8_9 | 90.6 | 90.8 | 90.4 | 90.2 |
| 1_3_8_10 | 91.5 | 90.8 | 92.1 | 88.2 |
| 1_3_8_11 | 91.5 | 93.9 | 89.5 | 90.2 |
| 1_3_9_10 | 92 | 90.8 | 93 | 84.3 |
| 1_3_9_11 | 91.5 | 90.8 | 92.1 | 88.2 |
| 1_3_10_11 | 91.5 | 90.8 | 92.1 | 86.3 |
| 1_4_5_6 | 92.9 | 91.8 | 93.9 | 90.2 |
| 1_4_5_7 | 92.9 | 91.8 | 93.9 | 88.2 |
| 1_4_5_8 | 92.5 | 93.9 | 91.2 | 88.2 |
| 1_4_5_9 | 92.5 | 90.8 | 93.9 | 84.3 |
| 1_4_5_10 | 92.5 | 90.8 | 93.9 | 84.3 |
| 1_4_5_11 | 93.4 | 92.9 | 93.9 | 86.3 |
| 1_4_6_7 | 92.5 | 92.9 | 92.1 | 88.2 |
| 1_4_6_8 | 91.5 | 90.8 | 92.1 | 92.2 |
| 1_4_6_9 | 92.9 | 90.8 | 94.7 | 90.2 |
| 1_4_6_10 | 91.5 | 90.8 | 92.1 | 90.2 |
| 1_4_6_11 | 92.9 | 90.8 | 94.7 | 90.2 |
| 1_4_7_8 | 91.5 | 89.8 | 93 | 90.2 |
| 1_4_7_9 | 90.1 | 87.8 | 92.1 | 90.2 |
| 1_4_7_10 | 90.1 | 87.8 | 92.1 | 90.2 |
| 1_4_7_11 | 90.6 | 88.8 | 92.1 | 90.2 |
| 1_4_8_9 | 92 | 92.9 | 91.2 | 88.2 |
| 1_4_8_10 | 91 | 88.8 | 93 | 86.3 |
| 1_4_8_11 | 89.6 | 89.8 | 89.5 | 86.3 |
| 1_4_9_10 | 90.1 | 87.8 | 92.1 | 84.3 |
| 1_4_9_11 | 90.6 | 90.8 | 90.4 | 86.3 |

(continued)

| SEQ ID NO: | Training cohort | | | Validation cohort |
|---|---|---|---|---|
| | Accuracy (%) | Sensitivity (%) | Specificity (%) | Sensitivity (%) |
| 1_4_10_11 | 91 | 89.8 | 92.1 | 84.3 |
| 1_5_6_7 | 92 | 91.8 | 92.1 | 92.2 |
| 1_5_6_8 | 92.5 | 91.8 | 93 | 88.2 |
| 1_5_6_9 | 90.6 | 85.7 | 94.7 | 86.3 |
| 1_5_6_10 | 90.1 | 87.8 | 92.1 | 88.2 |
| 1_5_6_11 | 92 | 89.8 | 93.9 | 86.3 |
| 1_5_7_8 | 91.5 | 94.9 | 88.6 | 94.1 |
| 1_5_7_9 | 91.5 | 91.8 | 91.2 | 88.2 |
| 1_5_7_10 | 91.5 | 91.8 | 91.2 | 92.2 |
| 1_5_7_11 | 91.5 | 91.8 | 91.2 | 92.2 |
| 1_5_8_9 | 92.5 | 92.9 | 92.1 | 90.2 |
| 1_5_8_10 | 91 | 94.9 | 87.7 | 94.1 |
| 1_5_8_11 | 92 | 92.9 | 91.2 | 92.2 |
| 1_5_9_10 | 90.1 | 90.8 | 89.5 | 90.2 |
| 1_5_9_11 | 91 | 88.8 | 93 | 92.2 |
| 1_5_10_11 | 89.2 | 89.8 | 88.6 | 90.2 |
| 1_6_7_8 | 91 | 92.9 | 89.5 | 90.2 |
| 1_6_7_9 | 93.9 | 93.9 | 93.9 | 88.2 |
| 1_6_7_10 | 93.4 | 93.9 | 93 | 88.2 |
| 1_6_7_11 | 93.9 | 93.9 | 93.9 | 88.2 |
| 1_6_8_9 | 91.5 | 91.8 | 91.2 | 88.2 |
| 1_6_8_10 | 90.6 | 91.8 | 89.5 | 90.2 |
| 1_6_8_11 | 90.6 | 89.8 | 91.2 | 88.2 |
| 1_6_9_10 | 91.5 | 90.8 | 92.1 | 90.2 |
| 1_6_9_11 | 92.9 | 89.8 | 95.6 | 88.2 |
| 1_6_10_11 | 91 | 90.8 | 91.2 | 88.2 |
| 1_7_8_9 | 90.1 | 89.8 | 90.4 | 82.4 |
| 1_7_8_10 | 88.7 | 91.8 | 86 | 84.3 |
| 1_7_8_11 | 89.6 | 89.8 | 89.5 | 88.2 |
| 1_7_9_10 | 91 | 90.8 | 91.2 | 84.3 |
| 1_7_9_11 | 89.6 | 87.8 | 91.2 | 86.3 |
| 1_7_10_11 | 90.6 | 89.8 | 91.2 | 84.3 |
| 1_8_9_10 | 88.7 | 91.8 | 86 | 86.3 |
| 1_8_9_11 | 89.2 | 88.8 | 89.5 | 88.2 |
| 1_8_10_11 | 88.7 | 91.8 | 86 | 88.2 |
| 1_9_10_11 | 90.1 | 91.8 | 88.6 | 84.3 |
| 2_3_4_5 | 92.9 | 92.9 | 93 | 92.2 |

(continued)

| SEQ ID NO: | Training cohort | | | Validation cohort |
|---|---|---|---|---|
| | Accuracy (%) | Sensitivity (%) | Specificity (%) | Sensitivity (%) |
| 2_3_4_6 | 90.6 | 88.8 | 92.1 | 88.2 |
| 2_3_4_7 | 90.1 | 88.8 | 91.2 | 76.5 |
| 2_3_4_8 | 85.4 | 86.7 | 84.2 | 76.5 |
| 2_3_4_9 | 86.8 | 87.8 | 86 | 76.5 |
| 2_3_4_10 | 88.7 | 87.8 | 89.5 | 74.5 |
| 2_3_4_11 | 87.7 | 87.8 | 87.7 | 72.5 |
| 2_3_5_6 | 93.4 | 92.9 | 93.9 | 94.1 |
| 2_3_5_7 | 94.3 | 93.9 | 94.7 | 92.2 |
| 2_3_5_8 | 94.3 | 94.9 | 93.9 | 92.2 |
| 2_3_5_9 | 93.4 | 92.9 | 93.9 | 90.2 |
| 2_3_5_10 | 93.9 | 92.9 | 94.7 | 92.2 |
| 2_3_5_11 | 93.4 | 93.9 | 93 | 94.1 |
| 2_3_6_7 | 93.4 | 92.9 | 93.9 | 84.3 |
| 2_3_6_8 | 89.2 | 89.8 | 88.6 | 90.2 |
| 2_3_6_9 | 89.2 | 88.8 | 89.5 | 90.2 |
| 2_3_6_10 | 91 | 89.8 | 92.1 | 88.2 |
| 2_3_6_11 | 89.2 | 88.8 | 89.5 | 88.2 |
| 2_3_7_8 | 88.7 | 87.8 | 89.5 | 80.4 |
| 2_3_7_9 | 90.6 | 90.8 | 90.4 | 80.4 |
| 2_3_7_10 | 89.6 | 88.8 | 90.4 | 78.4 |
| 2_3_7_11 | 90.6 | 89.8 | 91.2 | 78.4 |
| 2_3_8_9 | 85.8 | 87.8 | 84.2 | 78.4 |
| 2_3_8_10 | 86.3 | 86.7 | 86 | 76.5 |
| 2_3_8_11 | 85.8 | 86.7 | 85.1 | 76.5 |
| 2_3_9_10 | 89.6 | 87.8 | 91.2 | 76.5 |
| 2_3_9_11 | 87.3 | 87.8 | 86.8 | 76.5 |
| 2_3_10_11 | 87.7 | 86.7 | 88.6 | 74.5 |
| 2_4_5_6 | 93.9 | 93.9 | 93.9 | 90.2 |
| 2_4_5_7 | 92.5 | 90.8 | 93.9 | 84.3 |
| 2_4_5_8 | 92 | 91.8 | 92.1 | 88.2 |
| 2_4_5_9 | 91.5 | 90.8 | 92.1 | 84.3 |
| 2_4_5_10 | 92.5 | 90.8 | 93.9 | 84.3 |
| 2_4_5_11 | 92.5 | 93.9 | 91.2 | 84.3 |
| 2_4_6_7 | 91.5 | 89.8 | 93 | 92.2 |
| 2_4_6_8 | 88.2 | 87.8 | 88.6 | 90.2 |
| 2_4_6_9 | 88.2 | 86.7 | 89.5 | 90.2 |
| 2_4_6_10 | 89.6 | 85.7 | 93 | 90.2 |

(continued)

| SEQ ID NO: | Training cohort | | | Validation cohort |
|---|---|---|---|---|
| | Accuracy (%) | Sensitivity (%) | Specificity (%) | Sensitivity (%) |
| 2_4_6_11 | 87.7 | 85.7 | 89.5 | 90.2 |
| 2_4_7_8 | 84.4 | 83.7 | 85.1 | 82.4 |
| 2_4_7_9 | 84 | 82.7 | 85.1 | 82.4 |
| 2_4_7_10 | 86.3 | 83.7 | 88.6 | 74.5 |
| 2_4_7_11 | 87.3 | 84.7 | 89.5 | 84.3 |
| 2_4_8_9 | 84 | 83.7 | 84.2 | 80.4 |
| 2_4_8_10 | 84 | 82.7 | 85.1 | 80.4 |
| 2_4_8_11 | 83 | 82.7 | 83.3 | 82.4 |
| 2_4_9_10 | 85.8 | 81.6 | 89.5 | 82.4 |
| 2_4_9_11 | 83.5 | 81.6 | 85.1 | 80.4 |
| 2_4_10_11 | 84.4 | 82.7 | 86 | 76.5 |
| 2_5_6_7 | 92 | 88.8 | 94.7 | 90.2 |
| 2_5_6_8 | 93.9 | 91.8 | 95.6 | 92.2 |
| 2_5_6_9 | 92 | 88.8 | 94.7 | 92.2 |
| 2_5_6_10 | 92.9 | 91.8 | 93.9 | 92.2 |
| 2_5_6_11 | 91.5 | 89.8 | 93 | 92.2 |
| 2_5_7_8 | 89.6 | 87.8 | 91.2 | 86.3 |
| 2_5_7_9 | 90.6 | 88.8 | 92.1 | 84.3 |
| 2_5_7_10 | 91 | 89.8 | 92.1 | 84.3 |
| 2_5_7_11 | 92 | 90.8 | 93 | 88.2 |
| 2_5_8_9 | 92 | 88.8 | 94.7 | 88.2 |
| 2_5_8_10 | 89.6 | 88.8 | 90.4 | 88.2 |
| 2_5_8_11 | 91 | 89.8 | 92.1 | 88.2 |
| 2_5_9_10 | 90.6 | 89.8 | 91.2 | 88.2 |
| 2_5_9_11 | 92 | 90.8 | 93 | 88.2 |
| 2_5_10_11 | 92 | 90.8 | 93 | 88.2 |
| 2_6_7_8 | 88.7 | 87.8 | 89.5 | 88.2 |
| 2_6_7_9 | 87.7 | 83.7 | 91.2 | 86.3 |
| 2_6_7_10 | 87.3 | 84.7 | 89.5 | 86.3 |
| 2_6_7_11 | 87.3 | 82.7 | 91.2 | 82.4 |
| 2_6_8_9 | 88.7 | 88.8 | 88.6 | 88.2 |
| 2_6_8_10 | 88.7 | 88.8 | 88.6 | 88.2 |
| 2_6_8_11 | 88.7 | 88.8 | 88.6 | 88.2 |
| 2_6_9_10 | 87.3 | 83.7 | 90.4 | 86.3 |
| 2_6_9_11 | 86.8 | 83.7 | 89.5 | 84.3 |
| 2_6_10_11 | 87.3 | 83.7 | 90.4 | 82.4 |
| 2_7_8_9 | 84.9 | 85.7 | 84.2 | 78.4 |

(continued)

| SEQ ID NO: | Training cohort | | | Validation cohort |
|---|---|---|---|---|
| | Accuracy (%) | Sensitivity (%) | Specificity (%) | Sensitivity (%) |
| 2_7_8_10 | 83 | 85.7 | 80.7 | 82.4 |
| 2_7_8_11 | 84 | 84.7 | 83.3 | 78.4 |
| 2_7_9_10 | 83 | 82.7 | 83.3 | 80.4 |
| 2_7_9_11 | 84 | 82.7 | 85.1 | 80.4 |
| 2_7_10_11 | 84 | 83.7 | 84.2 | 78.4 |
| 2_8_9_10 | 82.5 | 82.7 | 82.5 | 78.4 |
| 2_8_9_11 | 84 | 83.7 | 84.2 | 80.4 |
| 2_8_10_11 | 83 | 82.7 | 83.3 | 80.4 |
| 2_9_10_11 | 83 | 80.6 | 85.1 | 78.4 |
| 3_4_5_6 | 94.8 | 95.9 | 93.9 | 94.1 |
| 3_4_5_7 | 94.3 | 94.9 | 93.9 | 90.2 |
| 3_4_5_8 | 92.9 | 95.9 | 90.4 | 92.2 |
| 3_4_5_9 | 93.9 | 95.9 | 92.1 | 90.2 |
| 3_4_5_10 | 94.8 | 95.9 | 93.9 | 92.2 |
| 3_4_5_11 | 93.4 | 95.9 | 91.2 | 92.2 |
| 3_4_6_7 | 91.5 | 91.8 | 91.2 | 92.2 |
| 3_4_6_8 | 88.7 | 89.8 | 87.7 | 88.2 |
| 3_4_6_9 | 89.6 | 89.8 | 89.5 | 86.3 |
| 3_4_6_10 | 90.6 | 88.8 | 92.1 | 86.3 |
| 3_4_6_11 | 89.2 | 88.8 | 89.5 | 88.2 |
| 3_4_7_8 | 85.4 | 83.7 | 86.8 | 74.5 |
| 3_4_7_9 | 86.3 | 87.8 | 85.1 | 86.3 |
| 3_4_7_10 | 84.9 | 80.6 | 88.6 | 76.5 |
| 3_4_7_11 | 84.9 | 82.7 | 86.8 | 78.4 |
| 3_4_8_9 | 86.3 | 88.8 | 84.2 | 76.5 |
| 3_5_6_7 | 95.3 | 95.9 | 94.7 | 94.1 |
| 3_5_6_8 | 94.3 | 94.9 | 93.9 | 94.1 |
| 3_5_6_9 | 94.8 | 95.9 | 93.9 | 94.1 |
| 3_5_6_10 | 95.3 | 95.9 | 94.7 | 94.1 |
| 3_5_6_11 | 94.8 | 95.9 | 93.9 | 94.1 |
| 3_5_7_8 | 94.8 | 94.9 | 94.7 | 94.1 |
| 3_5_7_9 | 94.8 | 96.9 | 93 | 94.1 |
| 3_5_7_10 | 94.8 | 95.9 | 93.9 | 94.1 |
| 3_5_7_11 | 95.8 | 96.9 | 94.7 | 94.1 |
| 3_5_8_9 | 92 | 94.9 | 89.5 | 92.2 |
| 3_5_8_10 | 95.3 | 94.9 | 95.6 | 92.2 |
| 3_5_8_11 | 94.8 | 95.9 | 93.9 | 92.2 |

(continued)

| SEQ ID NO: | Training cohort | | | Validation cohort |
|---|---|---|---|---|
| | Accuracy (%) | Sensitivity (%) | Specificity (%) | Sensitivity (%) |
| 3_5_9_10 | 93.9 | 93.9 | 93.9 | 94.1 |
| 3_5_9_11 | 92.9 | 94.9 | 91.2 | 92.2 |
| 3_5_10_11 | 94.3 | 94.9 | 93.9 | 94.1 |
| 3_6_7_8 | 90.1 | 88.8 | 91.2 | 86.3 |
| 3_6_7_9 | 91 | 91.8 | 90.4 | 88.2 |
| 3_6_7_10 | 91.5 | 89.8 | 93 | 88.2 |
| 3_6_7_11 | 91 | 89.8 | 92.1 | 86.3 |
| 3_6_8_9 | 89.2 | 89.8 | 88.6 | 88.2 |
| 3_6_8_10 | 90.1 | 90.8 | 89.5 | 88.2 |
| 3_6_8_11 | 89.2 | 90.8 | 87.7 | 88.2 |
| 3_6_9_10 | 92 | 90.8 | 93 | 88.2 |
| 3_6_9_11 | 89.6 | 90.8 | 88.6 | 88.2 |
| 3_6_10_11 | 90.1 | 88.8 | 91.2 | 88.2 |
| 3_7_8_9 | 87.3 | 91.8 | 83.3 | 82.4 |
| 3_7_8_10 | 83 | 82.7 | 83.3 | 76.5 |
| 3_7_8_11 | 83.5 | 83.7 | 83.3 | 78.4 |
| 3_7_9_10 | 89.2 | 91.8 | 86.8 | 78.4 |
| 3_7_9_11 | 88.7 | 91.8 | 86 | 78.4 |
| 3_8_9_10 | 87.3 | 87.8 | 86.8 | 72.5 |
| 3_8_9_11 | 86.8 | 89.8 | 84.2 | 76.5 |
| 4_5_6_7 | 93.9 | 93.9 | 93.9 | 92.2 |
| 4_5_6_8 | 92.5 | 92.9 | 92.1 | 94.1 |
| 4_5_6_9 | 93.4 | 92.9 | 93.9 | 92.2 |
| 4_5_6_10 | 93.9 | 93.9 | 93.9 | 92.2 |
| 4_5_6_11 | 92.9 | 93.9 | 92.1 | 92.2 |
| 4_5_7_8 | 91 | 90.8 | 91.2 | 88.2 |
| 4_5_7_9 | 91.5 | 90.8 | 92.1 | 84.3 |
| 4_5_7_10 | 92.9 | 91.8 | 93.9 | 84.3 |
| 4_5_7_11 | 93.9 | 94.9 | 93 | 84.3 |
| 4_5_8_9 | 89.6 | 90.8 | 88.6 | 86.3 |
| 4_5_8_10 | 92.9 | 91.8 | 93.9 | 86.3 |
| 4_5_8_11 | 91 | 91.8 | 90.4 | 86.3 |
| 4_5_9_10 | 92.9 | 91.8 | 93.9 | 84.3 |
| 4_5_9_11 | 92 | 93.9 | 90.4 | 84.3 |
| 4_5_10_11 | 93.4 | 91.8 | 94.7 | 84.3 |
| 4_6_7_8 | 91 | 92.9 | 89.5 | 90.2 |
| 4_6_7_9 | 90.6 | 90.8 | 90.4 | 90.2 |

(continued)

| SEQ ID NO: | Training cohort | | | Validation cohort |
|---|---|---|---|---|
| | Accuracy (%) | Sensitivity (%) | Specificity (%) | Sensitivity (%) |
| 4_6_7_10 | 90.6 | 89.8 | 91.2 | 90.2 |
| 4_6_7_11 | 89.2 | 88.8 | 89.5 | 92.2 |
| 4_6_8_9 | 88.7 | 88.8 | 88.6 | 88.2 |
| 4_6_8_10 | 89.2 | 88.8 | 89.5 | 84.3 |
| 4_6_8_11 | 88.2 | 88.8 | 87.7 | 84.3 |
| 4_6_9_10 | 88.7 | 85.7 | 91.2 | 88.2 |
| 4_6_9_11 | 89.2 | 88.8 | 89.5 | 88.2 |
| 4_6_10_11 | 89.2 | 87.8 | 90.4 | 88.2 |
| 4_7_8_9 | 85.4 | 87.8 | 83.3 | 82.4 |
| 4_7_8_10 | 81.1 | 80.6 | 81.6 | 74.5 |
| 4_7_8_11 | 83.5 | 83.7 | 83.3 | 76.5 |
| 4_7_9_10 | 86.3 | 86.7 | 86 | 78.4 |
| 4_7_9_11 | 85.8 | 87.8 | 84.2 | 80.4 |
| 4_7_10_11 | 85.4 | 82.7 | 87.7 | 72.5 |
| 4_8_9_10 | 83.5 | 83.7 | 83.3 | 78.4 |
| 4_8_9_11 | 84.9 | 86.7 | 83.3 | 76.5 |
| 4_8_10_11 | 82.1 | 81.6 | 82.5 | 72.5 |
| 4_9_10_11 | 85.4 | 84.7 | 86 | 74.5 |
| 5_6_7_8 | 90.6 | 95.9 | 86 | 88.2 |
| 5_6_7_9 | 92.9 | 93.9 | 92.1 | 88.2 |
| 5_6_7_10 | 92.5 | 93.9 | 91.2 | 88.2 |
| 5_6_7_11 | 92.5 | 95.9 | 89.5 | 92.2 |
| 5_6_8_9 | 87.7 | 88.8 | 86.8 | 86.3 |
| 5_6_8_10 | 86.8 | 89.8 | 84.2 | 90.2 |
| 5_6_8_11 | 89.6 | 90.8 | 88.6 | 90.2 |
| 5_6_9_10 | 86.3 | 87.8 | 85.1 | 90.2 |
| 5_6_9_11 | 88.7 | 88.8 | 88.6 | 90.2 |
| 5_6_10_11 | 88.7 | 90.8 | 86.8 | 92.2 |
| 5_7_8_9 | 90.6 | 94.9 | 86.8 | 88.2 |
| 5_7_8_10 | 90.6 | 94.9 | 86.8 | 86.3 |
| 5_7_8_11 | 90.6 | 94.9 | 86.8 | 88.2 |
| 5_7_9_10 | 92.5 | 94.9 | 90.4 | 86.3 |
| 5_7_9_11 | 89.6 | 92.9 | 86.8 | 86.3 |
| 5_7_10_11 | 92.5 | 93.9 | 91.2 | 86.3 |
| 5_8_9_10 | 86.3 | 89.8 | 83.3 | 88.2 |
| 5_8_9_11 | 86.8 | 89.8 | 84.2 | 90.2 |
| 5_8_10_11 | 87.3 | 91.8 | 83.3 | 90.2 |

(continued)

| SEQ ID NO: | Training cohort | | | Validation cohort |
|---|---|---|---|---|
| | Accuracy (%) | Sensitivity (%) | Specificity (%) | Sensitivity (%) |
| 5_9_10_11 | 86.8 | 91.8 | 82.5 | 90.2 |
| 6_7_8_9 | 87.3 | 87.8 | 86.8 | 84.3 |
| 6_7_8_10 | 87.3 | 88.8 | 86 | 84.3 |
| 6_7_8_11 | 86.8 | 86.7 | 86.8 | 84.3 |
| 6_7_9_10 | 86.3 | 85.7 | 86.8 | 80.4 |
| 6_7_9_11 | 86.3 | 86.7 | 86 | 76.5 |
| 6_7_10_11 | 85.4 | 87.8 | 83.3 | 82.4 |
| 6_8_9_10 | 86.8 | 90.8 | 83.3 | 80.4 |
| 6_8_9_11 | 86.3 | 85.7 | 86.8 | 80.4 |
| 6_8_10_11 | 87.7 | 89.8 | 86 | 78.4 |
| 6_9_10_11 | 85.8 | 86.7 | 85.1 | 80.4 |
| 7_8_9_10 | 83 | 84.7 | 81.6 | 76.5 |
| 7_8_9_11 | 83 | 84.7 | 81.6 | 76.5 |
| 8_9_10_11 | 83.5 | 84.7 | 82.5 | 78.4 |

As shown in these Examples, use of the polynucleotide, the kit, etc. and the method of the present invention enable a malignant brain tumor patient to be sensitively discriminated not only from a healthy subject but from a benign brain tumor patient. This permits early clinical decision to carry out the surgical resection of a cancer site. As a result, improvement in 5-year survival rate and reduction in the rate of recurrence can be achieved.

Industrial Applicability

According to the present invention, malignant brain tumor can be effectively detected by a simple and inexpensive method. This permits early detection, diagnosis and treatment of malignant brain tumor. The method of the present invention can detect malignant brain tumor low invasively using the blood of a patient and therefore allows malignant brain tumor to be detected conveniently and rapidly.

All publications, patents, and patent applications cited herein are incorporated herein by reference in their entirety.

## Claims

1. A kit for the detection of malignant brain tumor, comprising nucleic acid(s) capable of specifically binding to at least one polynucleotide selected from the group consisting of malignant brain tumor markers: miR-1909-3p, miR-6869-5p, miR-3178, miR-4787-5p, miR-6510-5p, miR-4695-5p, miR-4634, miR-4449, miR-3195, and miR-6836-3p.

2. The kit according to claim 1, wherein miR-1909-3p is hsa-miR-1909-3p, miR-6869-5p is hsa-miR-6869-5p, miR-3178 is hsa-miR-3178, miR-4787-5p is hsa-miR-4787-5p, miR-6510-5p is hsa-miR-6510-5p, miR-4695-5p is hsa-miR-4695-5p, miR-4634 is hsa-miR-4634, miR-4449 is hsa-miR-4449, miR-3195 is hsa-miR-3195, and miR-6836-3p is hsa-miR-6836-3p.

3. The kit according to claim 1 or 2, wherein the nucleic acid is a polynucleotide selected from the group consisting of the following polynucleotides (a) to (e):

(a) a polynucleotide consisting of a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,
(b) a polynucleotide comprising a nucleotide sequence represented by any of SEQ ID NOs: 1 to 10,
(c) a polynucleotide consisting of a nucleotide sequence complementary to a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,
(d) a polynucleotide comprising a nucleotide sequence complementary to a nucleotide sequence represented by any of SEQ ID NOs: 1 to 10 or a nucleotide sequence derived from the nucleotide sequence by the replacement of u with t, and
(e) a polynucleotide hybridizing under stringent conditions to any of the polynucleotides (a) to (d).

4. The kit according to any one of claims 1 to 3, wherein the kit further comprises a nucleic acid capable of specifically binding to a polynucleotide of another malignant brain tumor marker miR-187-5p.

5. The kit according to claim 4, wherein miR-187-5p is hsa-miR-187-5p.

6. The kit according to claim 4 or 5, wherein the nucleic acid capable of specifically binding to the polynucleotide of miR-187-5p is a polynucleotide selected from the group consisting of the following polynucleotides (f) to (j):

(f) a polynucleotide consisting of a nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(g) a polynucleotide comprising a nucleotide sequence represented by SEQ ID NO: 11,
(h) a polynucleotide consisting of a nucleotide sequence complementary to a nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(i) a polynucleotide comprising a nucleotide sequence complementary to a nucleotide sequence represented by SEQ ID NO: 11 or a nucleotide sequence derived from the nucleotide sequence by the replacement of u with t, and
(j) a polynucleotide hybridizing under stringent conditions to any of the polynucleotides (f) to (i).

7. A device for the detection of malignant brain tumor, comprising nucleic acid(s) capable of specifically binding to at least one polynucleotide selected from the group consisting of malignant brain tumor markers: miR-1909-3p, miR-6869-5p, miR-3178, miR-4787-5p, miR-6510-5p, miR-4695-5p, miR-4634, miR-4449, miR-3195, and miR-6836-3p.

**8.** The device according to claim 7, wherein miR-1909-3p is hsa-miR-1909-3p, miR-6869-5p is hsa-miR-6869-5p, miR-3178 is hsa-miR-3178, miR-4787-5p is hsa-miR-4787-5p, miR-6510-5p is hsa-miR-6510-5p, miR-4695-5p is hsa-miR-4695-5p, miR-4634 is hsa-miR-4634, miR-4449 is hsa-miR-4449, miR-3195 is hsa-miR-3195, and miR-6836-3p is hsa-miR-6836-3p.

**9.** The device according to claim 7 or 8, wherein the nucleic acid is a polynucleotide selected from the group consisting of the following polynucleotides (a) to (e):

(a) a polynucleotide consisting of a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,
(b) a polynucleotide comprising a nucleotide sequence represented by any of SEQ ID NOs: 1 to 10,
(c) a polynucleotide consisting of a nucleotide sequence complementary to a nucleotide sequence represented by any of SEQ ID NOs: 1 to 10 or a nucleotide sequence derived from the nucleotide sequence by the replacement of u with t, or a variant thereof, a derivative thereof, or a fragment thereof comprising 15 or more consecutive nucleotides,
(d) a polynucleotide comprising a nucleotide sequence complementary to a nucleotide sequence represented by any of SEQ ID NO: 1 to 10 or a nucleotide sequence derived from the nucleotide sequence by the replacement of u with t, and
(e) a polynucleotide hybridizing under stringent conditions to any of the polynucleotides (a) to (d).

**10.** The device according to any one of claims 7 to 9, wherein the device further comprises a nucleic acid capable of specifically binding to a polynucleotide of another malignant brain tumor marker miR-187-5p.

**11.** The device according to claim 10, wherein miR-187-5p is hsa-miR-187-5p.

**12.** The device according to claim 10 or 11, wherein the nucleic acid capable of specifically binding to the polynucleotide of miR-187-5p is a polynucleotide selected from the group consisting of the following polynucleotides (f) to (j):

(f) a polynucleotide consisting of a nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(g) a polynucleotide comprising a nucleotide sequence represented by SEQ ID NO: 11,
(h) a polynucleotide consisting of a nucleotide sequence complementary to a nucleotide sequence represented by SEQ ID NO: 11 or a nucleotide sequence derived from the nucleotide sequence by the replacement of u with t, or a variant thereof, a derivative thereof, or a fragment thereof comprising 15 or more consecutive nucleotides,
(i) a polynucleotide comprising a nucleotide sequence complementary to a nucleotide sequence represented by SEQ ID NO: 11 or a nucleotide sequence derived from the nucleotide sequence by the replacement of u with t, and
(j) a polynucleotide hybridizing under stringent conditions to any of the polynucleotides (f) to (i).

**13.** The device according to any one of claims 7 to 12, wherein the device is a device for measurement by a hybridization technique.

**14.** The device according to claim 13, wherein the hybridization technique is a nucleic acid array technique.

**15.** A method for detecting malignant brain tumor, comprising measuring an expression level of a target nucleic acid in a sample of a subject using the kit according to any one of claims 1 to 6 or the device according to any one of claims 7 to 14; and evaluating whether or not the subject has malignant brain tumor using the measured expression level and a control expression level for a healthy subject or a benign brain tumor patient measured in the same way, to detect the presence or absence of malignant brain tumor in the subject.

**16.** A method for detecting malignant brain tumor in a subject, comprising measuring an expression level of a target gene in a sample of the subject using the kit according to any one of claims 1 to 6 or the device according to any one of claims 7 to 14; and substituting the expression level of the target gene in the sample derived from the subject into a discriminant that is prepared with a gene expression level in a sample derived from a subject known to have malignant brain tumor and a gene expression level in a sample derived from a healthy subject or a benign brain tumor patient as supervising samples and is capable of differentially discriminating a malignant brain tumor patient from a healthy subject or a benign brain tumor patient, thereby evaluating the presence or absence of malignant brain tumor.

**17.** The method according to claim 15 or 16, wherein the subject is a human.

**18.** The method according to any one of claims 15 to 17, wherein the sample is blood, serum, or plasma.

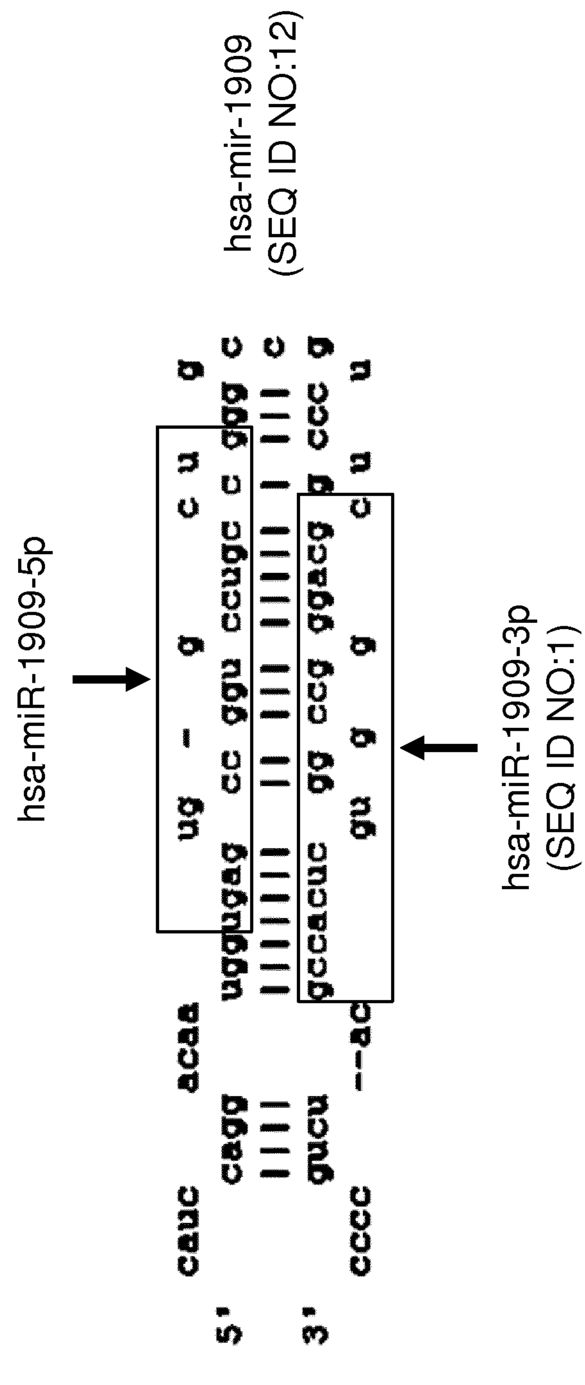
hsa-mir-1909
(SEQ ID NO:12)
hsa-miR-1909-5p
hsa-miR-1909-3p
(SEQ ID NO:1)
5'
3'

Fig. 1

Fig. 2

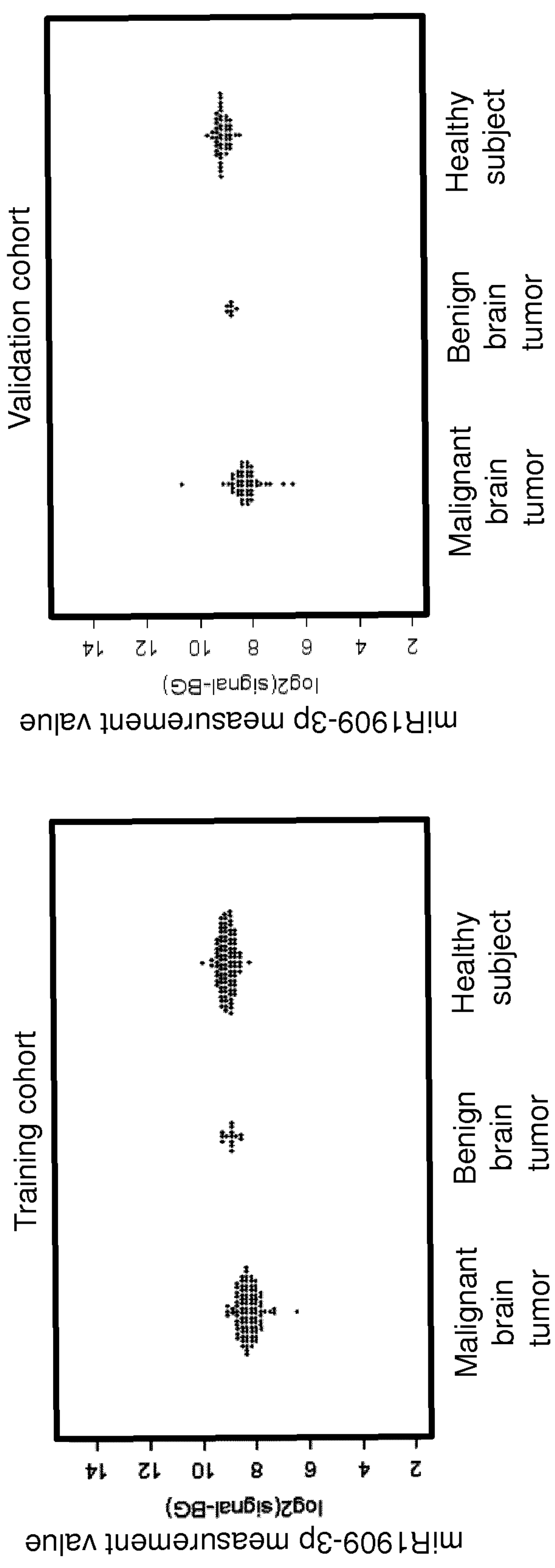
Training cohort
Validation cohort
miR1909-3p measurement value
log2(signal-BG)
2
4
6
8
10
12
14
Malignant brain tumor
Benign brain tumor
Healthy subject

Fig. 3

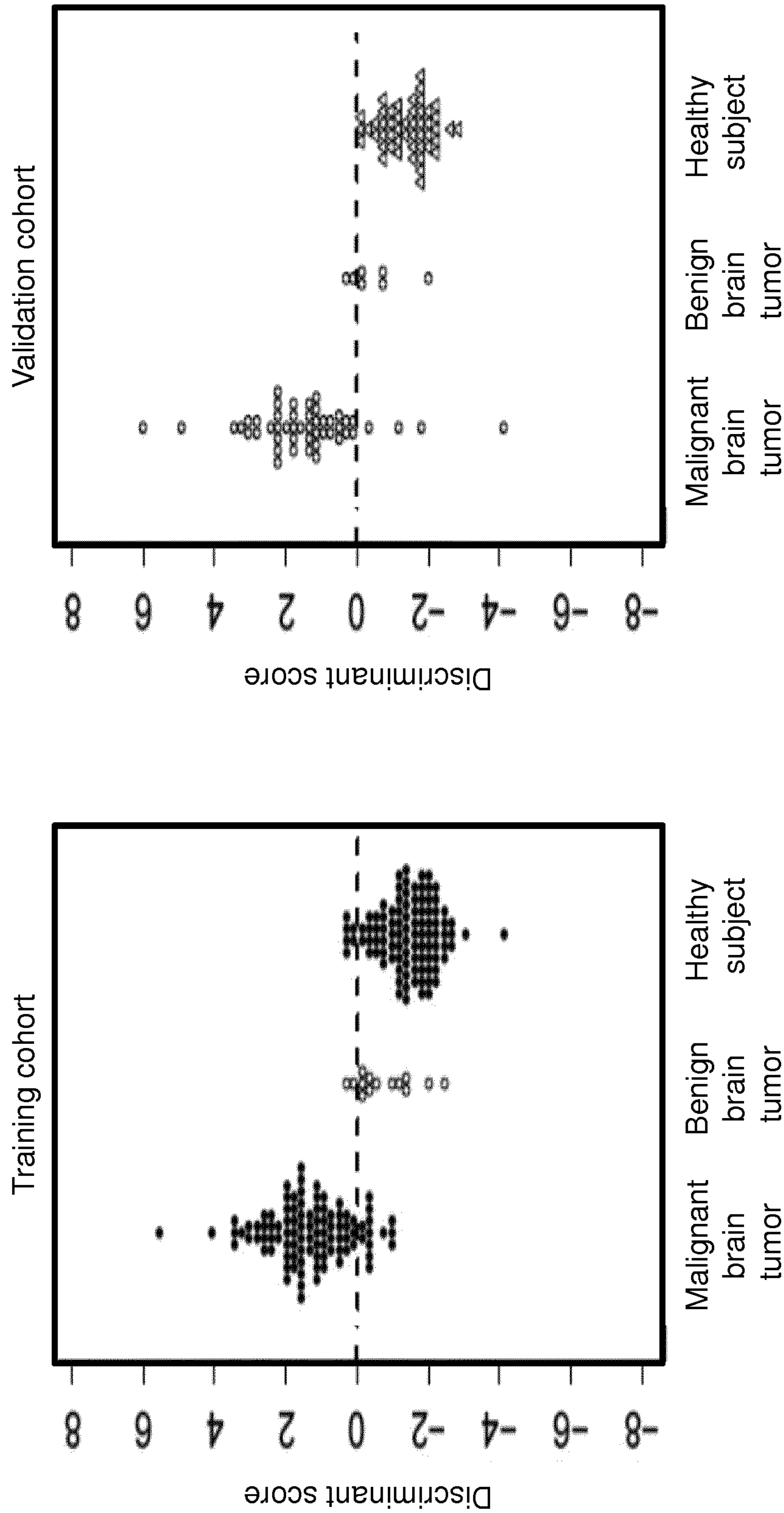
Training cohort
Validation cohort
Discriminant score
8
6
4
2
0
-2
-4
-6
-8
Malignant brain tumor
Benign brain tumor
Healthy subject

Fig. 4

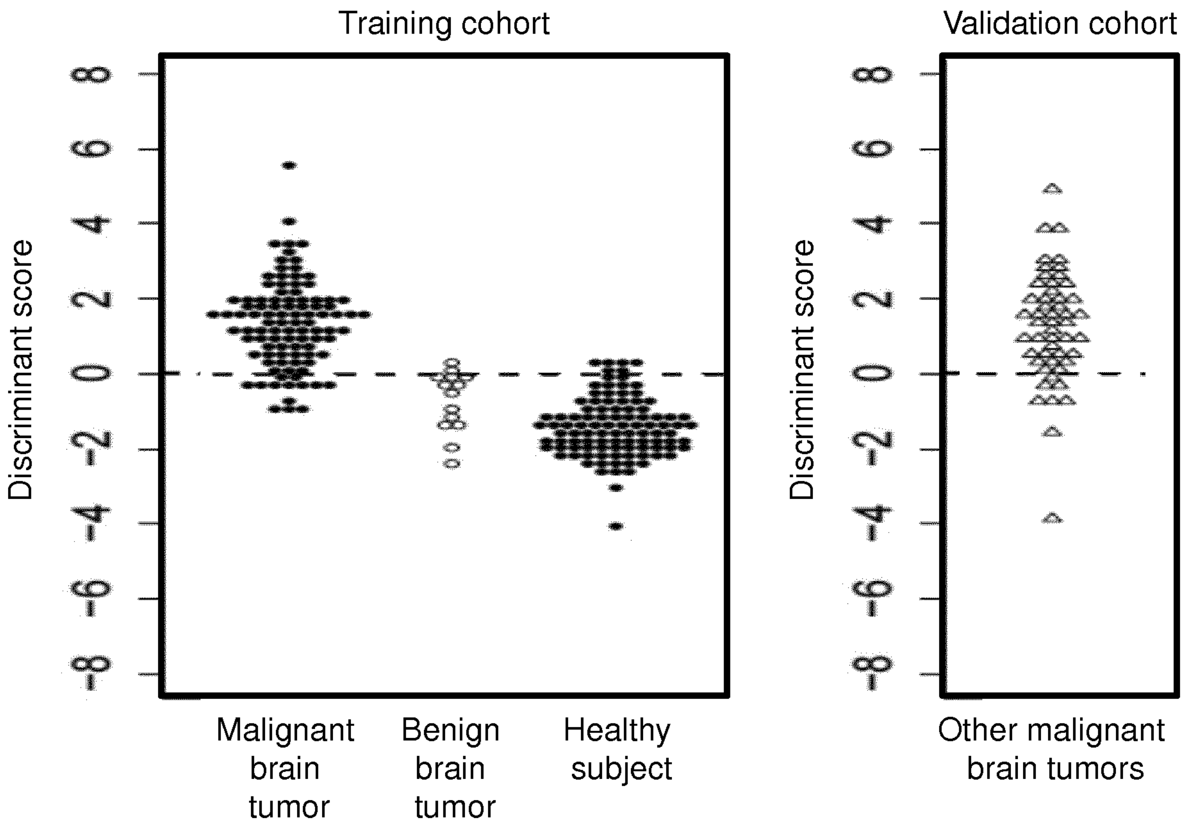
Training cohort
Validation cohort
Discriminant score
8
6
4
2
0
-2
-4
-6
-8
Malignant brain tumor
Benign brain tumor
Healthy subject
Discriminant score
8
6
4
2
0
-2
-4
-6
-8
Other malignant brain tumors

**INTERNATIONAL SEARCH REPORT**

International application No.
PCT/JP2017/013708

A. CLASSIFICATION OF SUBJECT MATTER
*C12N15/113*(2010.01)i, *C12M1/00*(2006.01)i, *C12N15/09*(2006.01)i, *C12Q1/68*(2006.01)i, *G01N33/50*(2006.01)i

According to International Patent Classification (IPC) or to both national classification and IPC

B. FIELDS SEARCHED

Minimum documentation searched (classification system followed by classification symbols)
C12N15/113, C12M1/00, C12N15/09, C12Q1/68, G01N33/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/BIOSIS(STN), PubMed, CiNii

C. DOCUMENTS CONSIDERED TO BE RELEVANT

| Category* | Citation of document, with indication, where appropriate, of the relevant passages | Relevant to claim No. |
|---|---|---|
| Y | US 2014/0088170 A1 (CITY OF HOPE), 27 March 2014 (27.03.2014), claims; abstract; example 1 & US 2016/0362689 A1 | 1-18 |
| Y | Masahiro MIZOGUCHI et al., "Shinkei Koshu ni Okeru microRNA Hatsugen Kaiseki no Igi", the 72nd Annual Meeting of the Japan Neurosurgical Society Abstract, 2013, 2J-0066-03, particularly, column of 'Object', column of 'Subject and Method' | 1-18 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 June 2017 (21.06.17) | 04 July 2017 (04.07.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No. PCT/JP2017/013708

C (Continuation). DOCUMENTS CONSIDERED TO BE RELEVANT

| Category* | Citation of document, with indication, where appropriate, of the relevant passages | Relevant to claim No. |
|---|---|---|
| Y | OCHIYA, T., Secretory microRNA as a novel diagnostic marker, Drug Delivery System, 2011, Vol.26-1, pages 10-14, ONLINE ISSN: 1881-2732, PRINT ISSN: 0913-5006, page 10, right column, line 1 to page 11, left column, line 7, page 12, right column, 15th line from the bottom to page 13, left column, 7th line from the bottom, fig. 2 | 1-18 |
| Y | KONDO, S. High-performance DNA chip and analysis of a variety sample, Bunseki, 2012, pages 709-715, ISSN: 0386-2178, page 709, right column, 2nd line from the bottom to page 713, left column, 16th line from the bottom | 1-18 |
| Y | KOJIMA, M. et al., MicroRNA markers for the diagnosis of pancreatic and biliary-tract cancers, PLOS ONE, 2015, Vol.10, No.2, e0118220, pages 1-22, eISSN: 1932-6203, page 1, summary, page 3, line 12 to page 7, line 21 | 1-18 |
| A | BRAOUDAKI, M. et al., MicroRNA expression profiles in pediatric dysembryoplastic neuroepithelial tumors, Med. Oncol., 2016, Vol.33, No.5, pages 1-7, ISSN: 1357-0560 (Print), particularly, page 1, summary | 1-18 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20140088170 A
- JP 2016074717 A


### Non-patent literature cited in the description


- **ZHENG ZHANG et al.** *J. Comput. Biol.,* 2000, vol. 7, 203-214
- **ALTSCHUL, S.F. et al.** *Journal of Molecular Biology,* vol. 215, 403-410
- **PEARSON, W.R. et al.** *Proc. Natl. Acad. Sci. U. S. A.,* vol. 85, 2444-2448
- **NIELSEN, P.E. et al.** *Science,* 1991, vol. 254, 1497-500
- **OBIKA, S. et al.** *Tetrahedron Lett.,* 1998, vol. 39, 5401-5404
- **BAR M et al.** *Stem Cells,* 2008, vol. 26, 2496-2505
- **LADEWIG E et al.** *Genome Res,* 2012, vol. 22, 1634-1645
- **STARK MS et al.** *PLoS One,* 2010, vol. 5, e9685
- **PERSSON H et al.** *Cancer Res,* 2011, vol. 71, 78-86
- **JOYCE CE et al.** *Hum Mol Genet,* 2011, vol. 20, 4025-4040
- **JIMA DD et al.** *Blood,* 2010, vol. 116, e118-127
- **LIM LP et al.** *Science,* vol. 299, 1540
- **MORIN RD. et al.** *Genome Research,* 2008, vol. 18, 610-621
- **AUSUBEL et al.** Current Protocols in Molecular Biology. John Willey & Sons, 1993
- **SAMBROOK et al.** Molecular Cloning - A Laboratory Manual. Cold Spring Harbor Laboratory Press, 1989
- **SAMBROOK, J. ; RUSSEL, D.** Molecular Cloning, A LABORATORY MANUAL. Cold Spring Harbor Laboratory Press, 15 January 2001, vol. 1, 2, 7.42-7.45, 8.9-8.17
- **BOLSTAD, B. M. et al.** *Bioinformatics,* 2003, vol. 19, 185-193
- **A. SHIMOMURA et al.** *Cancer Sci,* 2016
- **VENABLES, W.N. et al.** Modern Applied Statistics with S. Springer, 2002
- **TAKAFUMI KANAMORI et al.** Pattern Recognition. Kyoritsu Shuppan Co., Ltd, 2009
- **RICHARD O. et al.** Pattern Classification. Wiley-Interscience, 2000
- **V. VAPNIK.** The Nature of Statistical Leaning Theory. Springer, 1995
- **HIDEKI ASO et al.** Frontier of Statistical Science 6 "Statistics of pattern recognition and learning - New concepts and approaches. Iwanami Shoten, 2004
- **NELLO CRISTIANINI et al.** Introduction to SVM. Kyoritsu Shuppan Co., Ltd, 2008
- **C. CORTES et al.** *Machine Learning,* 1995, vol. 20, 273-297
- **YASUSHI NAGATA et al.** Basics of statistical multiple comparison methods. Scientist Press Co., Ltd, 2007
- **FUREY TS. et al.** *Bioinformatics.,* 2000, vol. 16, 906-14
- **VENABLES, W. N. ; RIPLEY, B. D.** Modern Applied Statistics with S. Springer, 2002
- **A. SHIMOMURA et al.** *Cancer Sci.,* 2016

SEQUENCE LISTING

<110>  Toray Industries, Inc.
National Cancer Center

<120>  Kit or device and method for detecting malignant brain cancer

<130>  PH-6838-PCT

<150>  JP 2016-074717
<151>  2016-04-01

<160>  39

<170>  PatentIn version 3.5

<210>  1
<211>  22
<212>  RNA
<213>  Homo sapiens

<400>  1
cgcaggggcc gggugcucac cg    22

<210>  2
<211>  22
<212>  RNA
<213>  Homo sapiens

<400>  2
gugaguagug gcgcgcggcg gc    22

<210>  3
<211>  17
<212>  RNA
<213>  Homo sapiens

<400>  3
ggggcgcggc cggaucg    17

<210>  4
<211>  22
<212>  RNA
<213>  Homo sapiens

<400>  4
gcgggggugg cggcggcauc cc    22

<210>  5
<211>  22
<212>  RNA
<213>  Homo sapiens

<400>  5
cagcagggga gagagaggag uc    22

<210>  6
<211>  22

<212>  RNA
<213>  Homo sapiens

<400>  6
caggaggcag ugggcgagca gg  22

<210>  7
<211>  19
<212>  RNA
<213>  Homo sapiens

<400>  7
cggcgcgacc ggcccgggg  19

<210>  8
<211>  22
<212>  RNA
<213>  Homo sapiens

<400>  8
cgucccgggg cugcgcgagg ca  22

<210>  9
<211>  17
<212>  RNA
<213>  Homo sapiens

<400>  9
cgcgccgggc ccggguu  17

<210>  10
<211>  21
<212>  RNA
<213>  Homo sapiens

<400>  10
augccucccc cggccccgca g  21

<210>  11
<211>  22
<212>  RNA
<213>  Homo sapiens

<400>  11
ggcuacaaca caggacccgg gc  22

<210>  12
<211>  80
<212>  RNA
<213>  Homo sapiens

<400>  12
cauccaggac aauggugagu gccggugccu gcccuggggc cgucccugcg caggggccgg  60

gugcucaccg caucugcccc  80

<210> 13
<211> 62
<212> RNA
<213> Homo sapiens

<400> 13
gugaguagug gcgcgcggcg gcucggagua ccucugccgc cgcgcgcauc ggcucagcau 60

gc 62

<210> 14
<211> 84
<212> RNA
<213> Homo sapiens

<400> 14
gaggcugggc ggggcgcggc cggaucgguc gagagcgucc uggcugauga cggucucccg 60

ugcccacgcc ccaaacgcag ucuc 84

<210> 15
<211> 84
<212> RNA
<213> Homo sapiens

<400> 15
cgguccagac guggcggggg uggcggcggc aucccggacg gccugugagg gaugcgccgc 60

ccacugcccc gcgccgccug accg 84

<210> 16
<211> 54
<212> RNA
<213> Homo sapiens

<400> 16
agcagcaggg gagagagagg aguccucuag acaccgacuc ugucuccugc agau 54

<210> 17
<211> 74
<212> RNA
<213> Homo sapiens

<400> 17
ccugcaggag gcagugggcg agcaggcggg gcagcccaau gccaugggcc ugaucucacc 60

gcugccuccu uccc 74

<210> 18
<211> 54
<212> RNA
<213> Homo sapiens

<400> 18
ggacaagggc ggcgcgaccg gcccggggcu cuugggcggc cgcguuuccc cucc 54

<210> 19

<211> 66
<212> RNA
<213> Homo sapiens

<400> 19
agcagcccuc ggcggcccgg ggggcgggcg gcggugcccg ucccggggcu gcgcgaggca 60

caggcg 66

<210> 20
<211> 84
<212> RNA
<213> Homo sapiens

<400> 20
ccgcagccgc cgcgccgggc ccggguuggc cgcugacccc cgcggggccc ccggcggccg 60

gggcgggggc gggggcugcc ccgg 84

<210> 21
<211> 63
<212> RNA
<213> Homo sapiens

<400> 21
ggcuccgcag ggcccuggcg caggcaucca gacagcgggc gaaugccucc cccggccccg 60

cag 63

<210> 22
<211> 77
<212> RNA
<213> Homo sapiens

<400> 22
ggucgggcuc accaugacac agugugagac cucgggcuac aacacaggac ccgggcgcug 60

cucugacccc ucguguc 77

<210> 23
<211> 23
<212> RNA
<213> Homo sapiens

<400> 23
ugcgcagggg ccgggugcuc acc 23

<210> 24
<211> 15
<212> RNA
<213> Homo sapiens

<400> 24
ggcgcgcggc ggcuc 15

<210> 25
<211> 24

<212> RNA
<213> Homo sapiens

<400> 25
gaucggucga gagcguccug gcug 24

<210> 26
<211> 21
<212> RNA
<213> Homo sapiens

<400> 26
ggcggggggug gcggcggcau c 21

<210> 27
<211> 21
<212> RNA
<213> Homo sapiens

<400> 27
cagcagggga gagagaggag u 21

<210> 28
<211> 21
<212> RNA
<213> Homo sapiens

<400> 28
aggaggcagu gggcgagcag g 21

<210> 29
<211> 19
<212> RNA
<213> Homo sapiens

<400> 29
cggcgcgacc ggcccgggg 19

<210> 30
<211> 26
<212> RNA
<213> Homo sapiens

<400> 30
gucccggggc ugcgcgaggc acaggc 26

<210> 31
<211> 16
<212> RNA
<213> Homo sapiens

<400> 31
cgcgccgggc ccgggu 16

<210> 32
<211> 22

<212> RNA
<213> Homo sapiens

<400> 32
gggcuacaac acaggacccg gg 22

<210> 33
<211> 19
<212> RNA
<213> Homo sapiens

<400> 33
cgcaggggcc gggugcuca 19

<210> 34
<211> 15
<212> RNA
<213> Homo sapiens

<400> 34
gggcgcggcc ggauc 15

<210> 35
<211> 15
<212> RNA
<213> Homo sapiens

<400> 35
gguggcggcg gcauc 15

<210> 36
<211> 20
<212> RNA
<213> Homo sapiens

<400> 36
cagcagggga gagagaggag 20

<210> 37
<211> 17
<212> RNA
<213> Homo sapiens

<400> 37
ccggggcugc gcgaggc 17

<210> 38
<211> 15
<212> RNA
<213> Homo sapiens

<400> 38
gcgccgggcc cgggu 15

<210> 39
<211> 21

```
<212>  RNA
<213>  Homo sapiens

<400>  39
ggcuacaaca caggacccgg g                                                21
```